(19) Europäisches Patentamt
European Patent Office
Office européen des brevets

(11) EP 4 520 756 A1

(12) EUROPEAN PATENT APPLICATION
published in accordance with Art. 153(4) EPC

(43) Date of publication:
**12.03.2025 Bulletin 2025/11**

(21) Application number: **23799708.5**

(22) Date of filing: **04.05.2023**

(51) International Patent Classification (IPC):
***C07D 403/14*** *(2006.01)* ***H10K 85/60*** *(2023.01)*
***H10K 50/11*** *(2023.01)* ***C09K 11/06*** *(2006.01)*

(52) Cooperative Patent Classification (CPC):
**C07D 403/14; C09K 11/06; H10K 50/00; H10K 50/11; H10K 85/60; H10K 99/00**

(86) International application number:
**PCT/KR2023/006129**

(87) International publication number:
**WO 2023/214827 (09.11.2023 Gazette 2023/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.05.2022 KR 20220056283**

(71) Applicant: **Solus Advanced Materials Co., Ltd.**
**Iksan-si, Jeollabuk-do 54584 (KR)**

(72) Inventors:
- **KIM, Youngbae**
  **Yongin-si, Gyeonggi-do 16858 (KR)**
- **PARK, Hocheol**
  **Yongin-si, Gyeonggi-do 16858 (KR)**
- **JO, Hyunjong**
  **Yongin-si, Gyeonggi-do 16858 (KR)**
- **JUNG, Hwasoon**
  **Yongin-si, Gyeonggi-do 16858 (KR)**
- **SONG, Hyobum**
  **Yongin-si, Gyeonggi-do 16858 (KR)**
- **KIM, Geunhyeong**
  **Yongin-si, Gyeonggi-do 16858 (KR)**

(74) Representative: **Office Freylinger**
**P.O. Box 48**
**8001 Strassen (LU)**

# (54) ORGANIC LIGHT-EMITTING COMPOUND, AND ORGANIC ELECTROLUMINESCENT ELEMENT COMPRISING SAME

(57) The present invention relates to: a novel compound with excellent carrier transport capacity, light emission, and heat stability; and an organic electroluminescent element that includes same in at least one organic material layer to have improved properties in terms of light-emitting efficiency, driving voltage, and lifespan.

EP 4 520 756 A1

## Description

### Technical Field

The present invention relates to a novel organic compound and an organic electroluminescent element using the same and, more specifically, to a novel compound with excellent carrier transporting ability, light emitting ability, and heat resistance, and an organic electroluminescent element exhibiting improved characteristics, such as luminous efficiency, driving voltages, and lifespan, by containing the novel compound in one or more organic layers.

### Background Art

In studies on organic electroluminescent elements, which have continued from Bernanose's observation on organic thin film light emission in the 1950s to blue electroluminescence using an anthracene single crystal in 1965, Tang proposed an organic electroluminescent element with a lamination structure including functional layers of a hole layer and an emissive layer. Thereafter, in order to manufacture organic electroluminescent elements having high efficiency and long lifespan, the introduction of each specific organic layer into an element has been performed, and specialized materials used therein have been developed.

In an organic electroluminescent element, upon the application of voltage between two electrodes, holes from an anode and electrons from a cathode are injected into organic layers. The injected holes and electrons combine with each other to form excitons, and the excitons fall down to the ground state to emit light. Particularly, materials used for the organic layers may be classified into light emission materials, hole injection materials, hole transport materials, electron transport materials, electron injection materials, and the like according to the function thereof.

Materials for forming an emissive layer of the organic electroluminescent element may be classified into blue, green and red light emission materials according to the color of light emission. Additionally, yellow and orange light emission materials may be used as light emission materials for displaying better natural colors. Additionally, host/dopant-based light emission materials may be used as light emission materials to increase color purity and improve luminous efficiency through energy transfer. Dopant materials may be classified into fluorescent dopants using organic materials and phosphorescent dopants using metal complex compounds containing heavy atoms, such as Ir and Pt. These phosphorescent materials can theoretically improve the luminous efficiency up to four times compared to fluorescent materials, so attention is focused on phosphorescent host materials as well as phosphorescent dopants. Until today, NPB, BCP, $Alq_3$, and the like have been widely known as materials for use in a hole injection layer, a hole transport layer, an electron transport layer, and an electron injection layer, and as for light emission materials, anthracene derivatives have been reported as fluorescent dopant/host materials. Particularly, as for phosphorescent materials having a large advantage in terms of efficiency improvement among light emission materials, metal complex compounds containing Ir, such as Firpic, $Ir(ppy)_3$, and $(acac)Ir(btp)_2$, are used as blue, green, and red dopant materials. Until today, CBP shows excellent properties as a phosphorescent host material.

However, conventional light emission materials have advantages in terms of light emission characteristics, but are not satisfactory in terms of lifespan of organic electroluminescent elements due to low glass transition temperatures and very poor thermal stability. Accordingly, there is a need to develop light emission materials with excellent performance.

Meanwhile, deuterium has a natural abundance of approximately 0.015%. Deuterated compounds rich in concentrations of deuterium are well known. Deuterated aromatic compounds have been used to study chemical reactions and metabolic pathways and have also been used as raw materials for pharmaceuticals, agricultural chemicals, functional materials, and analytical tracers. Some deuterated electroluminescent materials have been reported to exhibit improved performance (efficiency and lifespan) compared with non-deuterated isotopomers (see, e.g., Tong, et al. J. Phys. Chem. C 2007, 111, 3490-4). Current methods of synthesizing deuterated compounds may require self-weighting to achieve high levels of deuteration. Since these methods are expensive or time-consuming, they are not suitable in terms of cost and efficiency. Therefore, there is a continuous need for an improved manufacturing method for synthesizing deuterated aromatic compounds.

### Disclosure of Invention

### Technical Problem

The present invention has been made to solve the above-mentioned problems, and an aspect of the present invention is to provide a novel compound, which can be used as a material for an organic layer of an organic electroluminescent element, specifically, a host material for an emissive layer, due to excellent carrier transporting ability, light emitting ability, and heat resistance.

Another aspect of the present invention is to provide an organic electroluminescent element exhibiting a low

driving voltage, high luminous efficiency, and improved lifespan by containing the above-described novel compound.

Other purposes and advantages of the present disclosure will be clarified by following detailed description and claims.

Solution to Problem

In accordance with an aspect of the present invention, there is provided a compound represented by Chemical Formula 1 below, specifically, a deuterated triscarbazole-based compound.

[Chemical Formula 1]

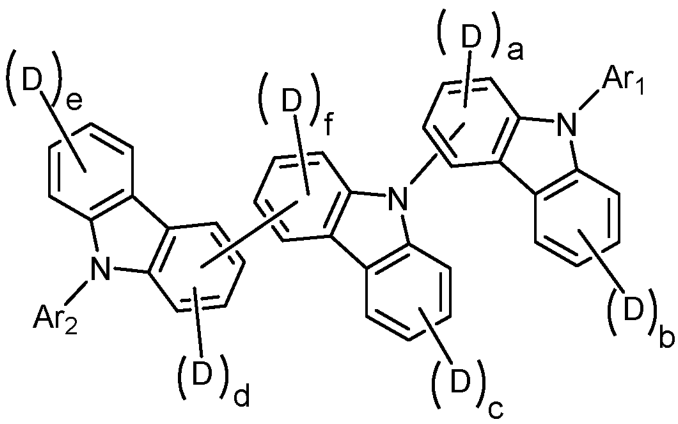

wherein Chemical Formula 1,

$Ar_1$ and $Ar_2$ are the same or different from each other and are each independently selected from the group consisting of a $C_1$ to $C_{40}$ alkyl group, a $C_2$ to $C_{40}$ alkenyl group, a $C_2$ to $C_{40}$ alkynyl group, a $C_6$ to $C_{40}$ aryl group, a heteroaryl group having 5 to 40 nuclear atoms, a $C_6$ to $C_{40}$ aryloxy group, a $C_1$ to $C_{40}$ alkyloxy group, a $C_6$ to $C_{40}$ arylamine group, a $C_3$ to $C_{40}$ cycloalkyl group, a heterocycloalkyl group having 3 to 40 nuclear atoms, a $C_1$ to $C_{40}$ alkylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylsilyl group;

a, d, and f are each independently 1 to 3;

b, c, and e are each independently 1 to 4, provided that

$$a + b + c + d + e + f \geq 15;$$

hydrogen of a benzene ring within a carbazole unsubstituted with deuterium (D) may be substituted with a substituent selected from the group consisting of a $C_1$ to $C_{40}$ alkyl group, a $C_6$ to $C_{40}$ aryl group, and a heteroaryl group having 5 to 40 nuclear atoms; and

the alkyl group, alkenyl group, alkynyl group, aryl group, heteroaryl group, aryloxy group, alkyloxy group, arylamine group, cycloalkyl group, heterocycloalkyl group, alkylsilyl group, alkylboron group, arylboron group, arylphosphine group, arylphosphine oxide group and arylsilyl group of $Ar_1$ to $Ar_2$ are each independently substituted or unsubstituted with at least one substituent selected from the group consisting of deuterium (D), halogen, a cyano group, a nitro group, a $C_1$ to $C_{40}$ alkyl group, a $C_2$ to $C_{40}$ alkenyl group, a $C_2$ to $C_{40}$ alkynyl group, a $C_3$ to $C_{40}$ cycloalkyl group, a heterocycloalkyl group having 3 to 40 nuclear atoms, a $C_6$ to $C_{40}$ aryl group, a heteroaryl group having 5 to 40 nuclear atoms, a $C_1$ to $C_{40}$ alkyloxy group, a $C_6$ to $C_{60}$ aryloxy group, a $C_1$ to $C_{40}$ alkylsilyl group, a $C_6$ to $C_{40}$ arylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylamine group, and when the substituents are plural in number, the substituents may be the same or different from each other.

In accordance with an aspect of the present invention, there is provided an organic electroluminescent element, including: an anode; a cathode; and one or more organic layers interposed between the anode and the cathode, wherein at least one of the one or more organic layers contains the compound represented by Chemical Formula 1.

In an embodiment, the one or more organic layers may include at least one selected from the group consisting of an emissive layer, an auxiliary emissive layer, a hole injection layer, a hole transport layer, an electron injection layer, an

electron transport layer, and an auxiliary electron transport layer, and the emissive layer may contain the compound of Chemical Formula 1 as a host.

In an embodiment, the electron transport layer may contain an electron transporting compound containing at least two electron withdrawing groups (EWGs).

Advantageous Effects of Invention

According to an embodiment of the present invention, the compound represented by Chemical Formula 1 has excellent carrier transporting ability, light emitting ability, and heat resistance, and thus can be used as a material for an organic layer of an organic electroluminescent element.

Particularly, the use of the compound represented by Chemical Formula 1 as a host for an emissive layer can achieve high thermal stability, low driving voltage, fast mobility, high current efficiency, and lifespan characteristics compared with conventionally known host materials.

Accordingly, an organic electroluminescent element including the compound represented by Chemical Formula 1 can achieve significantly improved performance, such as excellent light emission performance, low driving voltages, long lifespan, and high efficiency, and thus can be effectively applied to full-color display panels and the like.

The advantageous effects according to the present invention are not limited by the contents exemplified above, and more various advantageous effects are included herein.

Best Mode for Carrying out the Invention

Hereinafter, the present invention will be described in detail.

<Organic compound>

The present invention provides a novel electroluminescent material, which contains at least three carbazole (Cz) groups, exhibits enhanced carrier transporting ability, light emitting ability, and chemical structure stability through deuterium (D) substitution on the carbazole groups, thereby achieving all the characteristics of an element, for example, low voltage, high efficiency, and long lifespan characteristics of the electroluminescent element.

Specifically, a compound represented by Chemical Formula 1 according to the present invention has a basic skeletal structure necessarily containing at least three carbazole (Cz) groups, where at least 15 or more deuteriums (D) are substituted. Such a compound is a novel P-type host composed of a triscarbazole, and has a stronger hole character than existing host materials and thus can maximize the performance of an N-type host. Additionally, the compound has excellent hole stability compared with existing P-type host materials, and thus can continuously maintain stable lifespan characteristics even under the initial characteristics of an element. Therefore, the inclusion of a P-type host composed of at least three or more carbazole groups can improve the hole stability of an element itself, thus enabling the production of high-performance OLED elements.

In view of the potential energy, deuterium (D) has a higher molecular mass and a lower zero-point energy than hydrogen (H), thus making the dissociation of deuterium relatively more difficult in a reaction. This low zero-point energy increases the bond dissociation energy, resulting in low reactivity, and consequently, increased stability of molecules containing deuterium (Molecules 2014, 19 Chem. Commun., 2014, 50, 14870-14872 J. Org. Chem. 2004, 69, 7212-7219*)*. Therefore, the compound of Chemical Formula 1 of the present invention, which contains at least three carbazole groups and has at least 15 deuteriums (D) substituted in the carbazole groups, can maximize the green color purity compared with compounds of the same structure that does not contain deuterium, and can further enhance the weakened carbon-hydrogen intramolecular bonding strength and improve the material stability, thereby significantly improving the lifespan characteristics of an element.

In addition, the present invention enables the optimization of the performance of an organic electroluminescent element through the improvement in hole stability of an emissive layer (EML) and the maximization of electron transporting ability of an electron transport layer (ETL), by configuring the emissive layer containing a compound of Chemical Formula 1 and using the emissive layer in combination with an electron transport layer containing a dual EWG-type material having high electron transporting ability through the containing of at least two EWGs.

Specifically, the compound represented by Chemical Formula 1 according to the present invention has a basic skeletal structure necessarily containing at least three carbazole (Cz) groups, where at least 15 or more deuteriums (D) are substituted on the carbazole groups. For example, the number of deuteriums (D) included in the basic skeletal structure is 15 or more, and namely, in Chemical Formula 1, a, d, and f are each independently 1 to 3, and b, c, and e are each independently 1 to 4, provided that $a + b + c + d + e + f \geq 15$. Preferably, in Chemical Formula 1, $a = d = f = 3$, and $b = c = e = 4$, so the number of deuteriums (D) is 21 or more.

Particularly, hydrogen of a benzene ring within a carbazole unsubstituted with deuterium (D) may be substituted

with a substituent selected from the group consisting of a $C_1$ to $C_{40}$ alkyl group, a $C_6$ to $C_{40}$ aryl group, and a heteroaryl group having 5 to 40 nuclear atoms.

In Chemical Formula 1, $Ar_1$ and $Ar_2$ as various substituents may be introduced at the N positions of two carbazole groups located at both terminals of the at least three carbazole (Cz) groups. $Ar_1$ and $Ar_2$ may be the same or different from each other and may each be independently selected from the group consisting of a $C_1$ to $C_{40}$ alkyl group, a $C_2$ to $C_{40}$ alkenyl group, a $C_2$ to $C_{40}$ alkynyl group, a $C_6$ to $C_{40}$ aryl group, a heteroaryl group having 5 to 40 nuclear atoms, a $C_6$ to $C_{40}$ aryloxy group, a $C_1$ to $C_{40}$ alkyloxy group, a $C_6$ to $C_{40}$ arylamine group, a $C_3$ to $C_{40}$ cycloalkyl group, a heterocycloalkyl group having 3 to 40 nuclear atoms, a $C_1$ to $C_{40}$ alkylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylsilyl group. Specifically, $Ar_1$ and $Ar_2$ may be the same or different from each other and may be each independently selected from the group consisting of a $C_6$ to $C_{40}$ aryl group and a heteroaryl group having 5 to 40 nuclear atoms. More specifically, $Ar_1$ and $Ar_2$ are each independently a $C_6$ to $C_{40}$ aryl group, and the aryl group of $Ar_1$ and $Ar_2$ is substituted or unsubstituted with at least one substituent selected from the group consisting of deuterium (D), a $C_6$ to $C_{40}$ aryl group, and a heteroaryl group having 5 to 40 nuclear atoms, and when the substituents are plural in number, the substituents may be the same or different from each other. Particularly, the aryl group of $Ar_1$ and $Ar_2$ may be unsubstituted with deuterium (D) or partially substituted with at least one deuterium (D), provided that the substitution of the aryl group of each $Ar_1$ and $Ar_2$ with deuterium (D) may be excluded.

Particularly, the compound according to Chemical Formula 1 preferably secures a deuterium substitution rate of at least 67% in order to ensure the deuteration substitution effect, wherein the upper limit is not particularly limited.

In an embodiment, $Ar_1$ and $Ar_2$ may be the same or different from each other and may each be independently represented by any one selected from the group consisting of substituents S1 to S9. However, the substituents are not limited thereto.

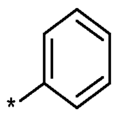
S1

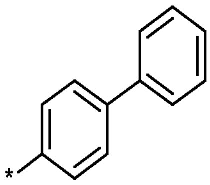
S2

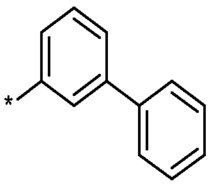
S3

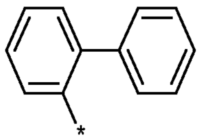
S4

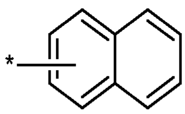
S5

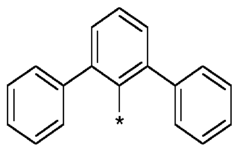
S6

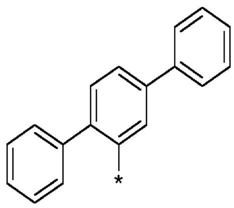
S7

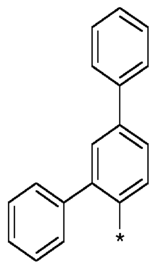
S8

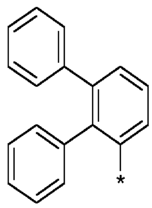
S9

In the chemical formulas,
the mark * is a site linked to Chemical Formula 1. Although not shown in the above-described structural formula, at least one deuterium (D) may be substituted. Also, at least one substituent (e.g., the same as the definition of $R_1$ in Chemical Formula 18 to be described later) known in the art may be substituted.

In the above-described Chemical Formula 1, the alkyl group, alkenyl group, alkynyl group, aryl group, heteroaryl group, aryloxy group, alkyloxy group, arylamine group, cycloalkyl group, heterocycloalkyl group, alkylsilyl group, alkylboron group, arylboron group, arylphosphine group, arylphosphine oxide group and arylsilyl group of $Ar_1$ to $Ar_2$ may each be independently substituted or unsubstituted with at least one substituent selected from the group consisting of deuterium (D), halogen, a cyano group, a nitro group, a $C_1$ to $C_{40}$ alkyl group, a $C_2$ to $C_{40}$ alkenyl group, a $C_2$ to $C_{40}$ alkynyl group, a $C_3$ to $C_{40}$ cycloalkyl group, a heterocycloalkyl group having 3 to 40 nuclear atoms, a $C_6$ to $C_{40}$ aryl group, a heteroaryl group having 5 to 40 nuclear atoms, a $C_1$ to $C_{40}$ alkyloxy group, a $C_6$ to $C_{60}$ aryloxy group, a $C_1$ to $C_{40}$ alkylsilyl group, a $C_6$ to $C_{40}$ arylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylamine group, and when the substituents are plural in number, the substituents may be the same or different from each other.

In an embodiment of the present invention, the compound of Chemical Formula 1 may be further embodied as any one of Chemical formulas 2 to 17 according to the linkage position between at least three carbazole groups.

[Chemical Formula 2]

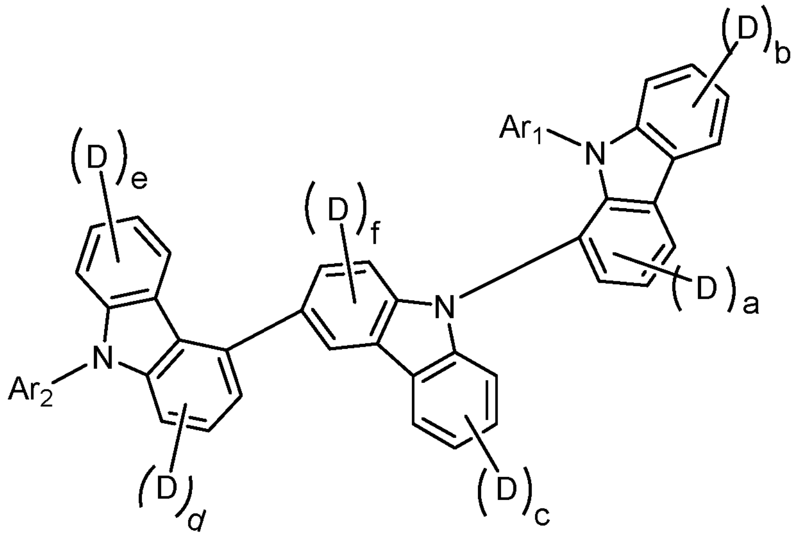

[Chemical Formula 3]

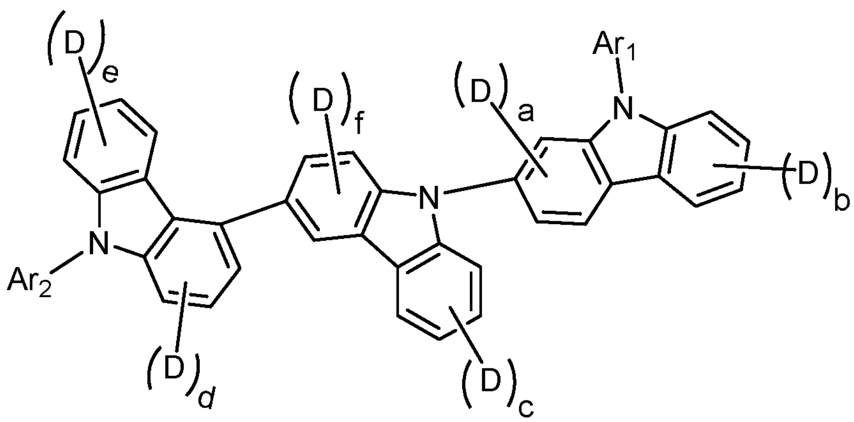

[Chemical Formula 4]

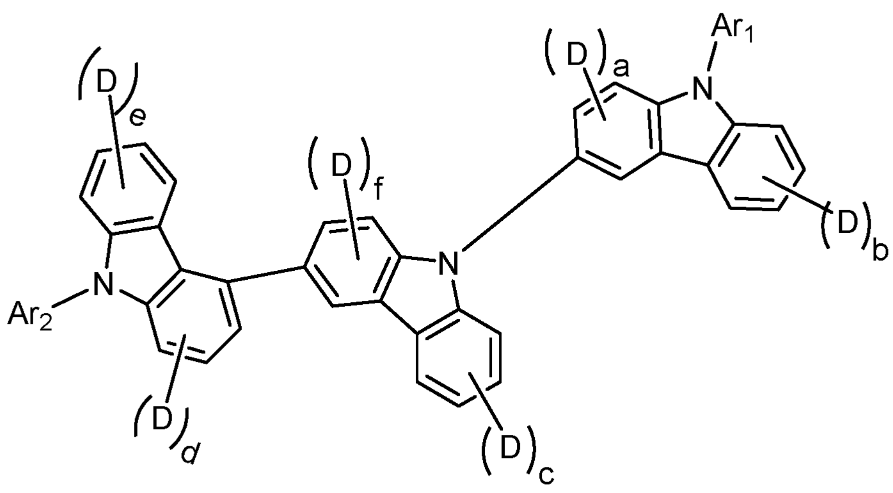

[Chemical Formula 5]

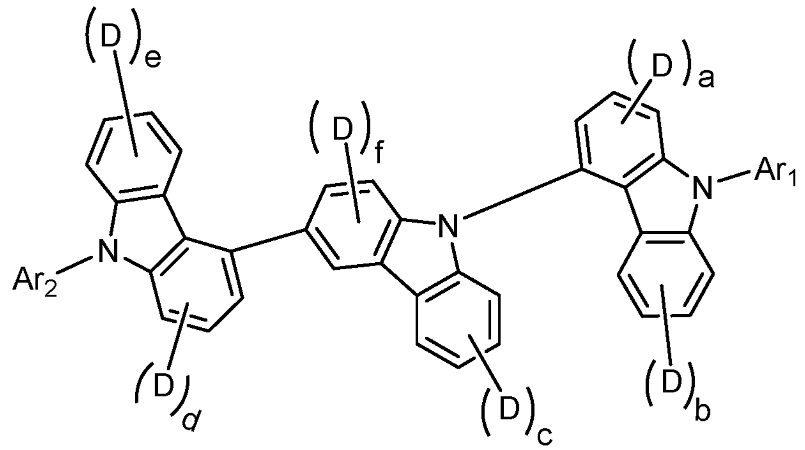

[Chemical Formula 6]

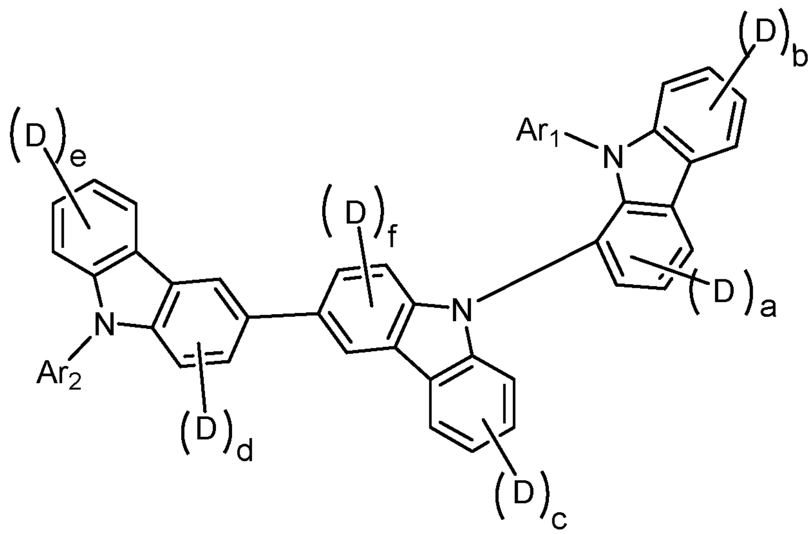

[Chemical Formula 7]

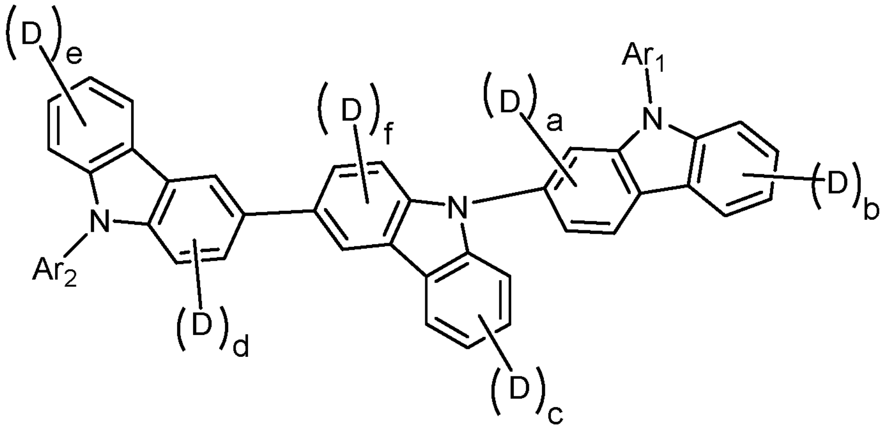

[Chemical Formula 8]

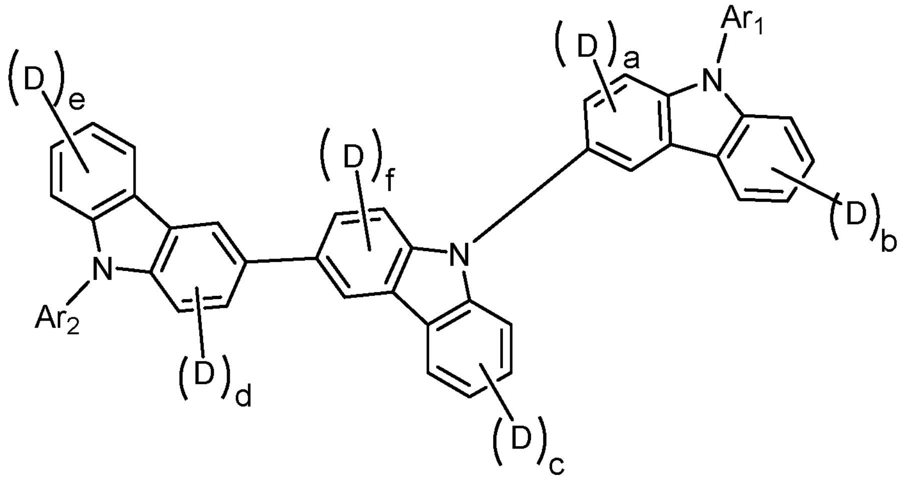

[Chemical Formula 9]

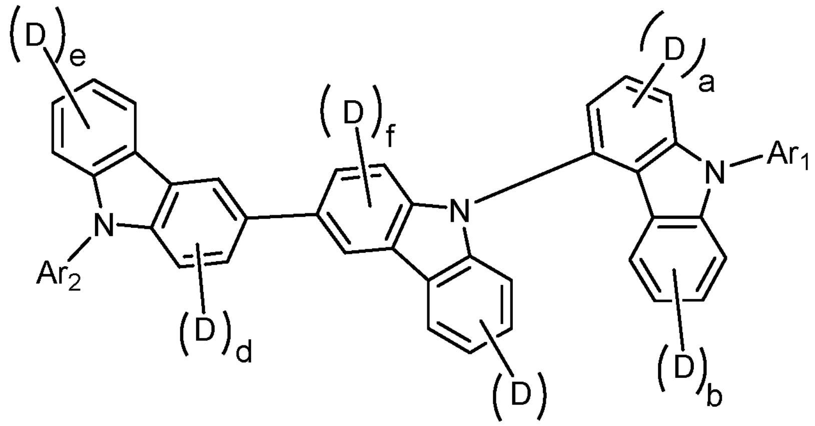

[Chemical Formula 10]

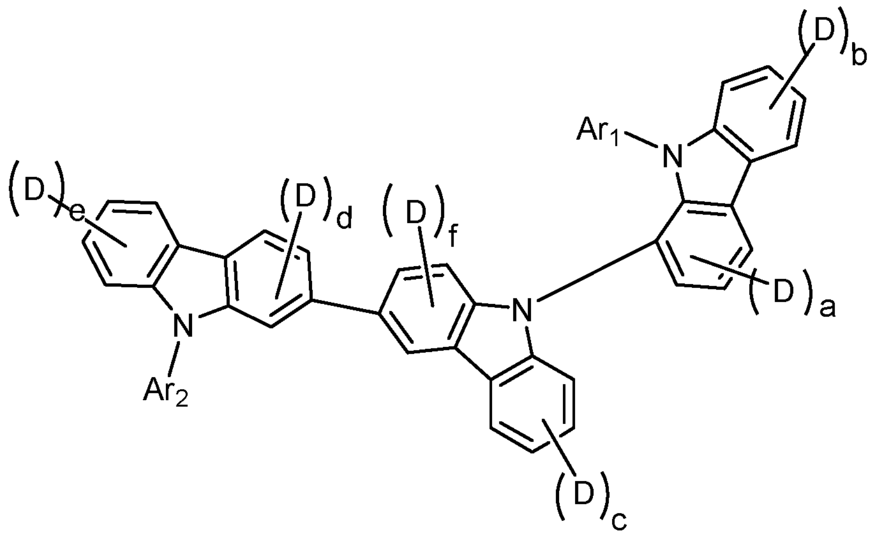

[Chemical Formula 11]

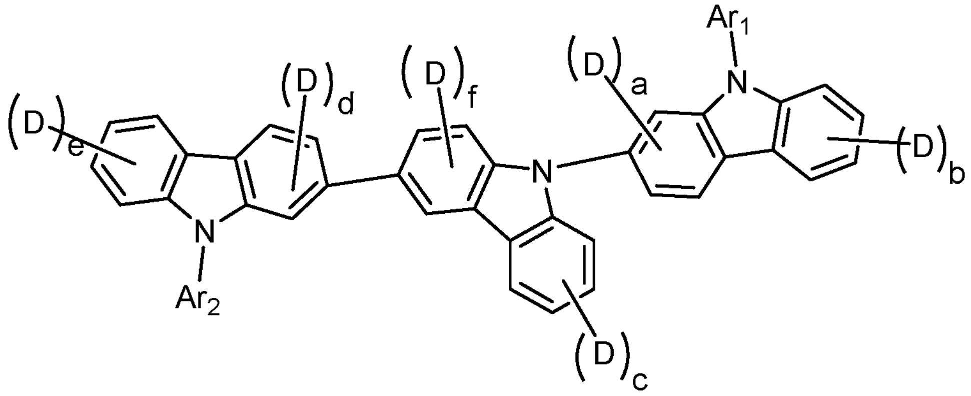

[Chemical Formula 12]

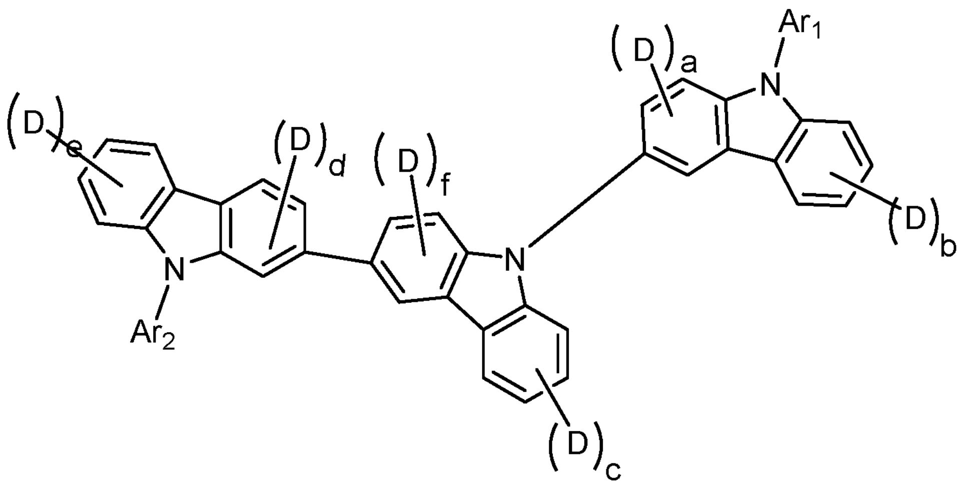

[Chemical Formula 13]

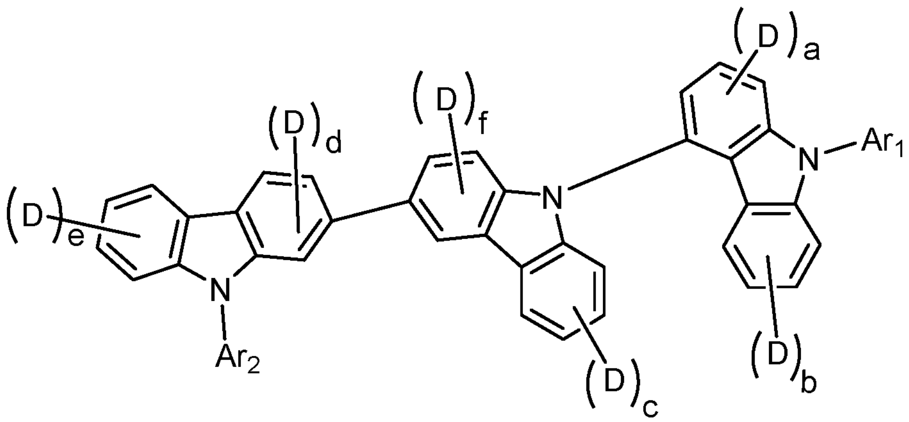

[Chemical Formula 14]

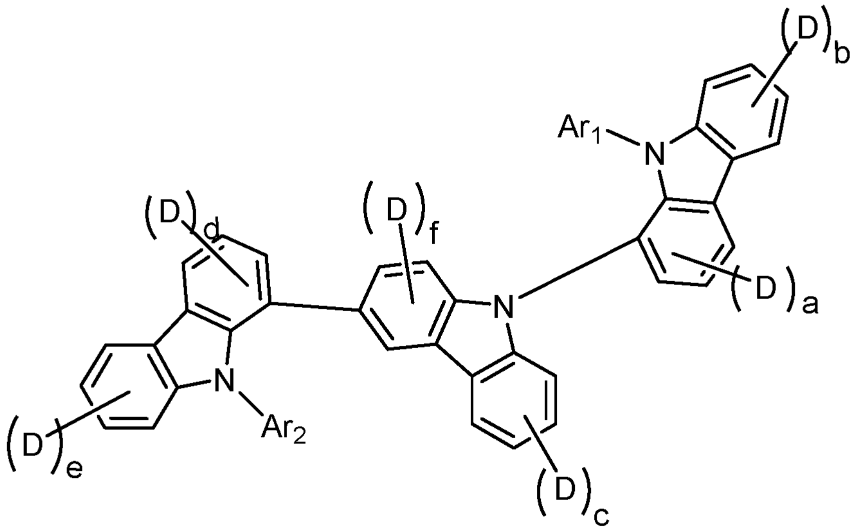

[Chemical Formula 15]

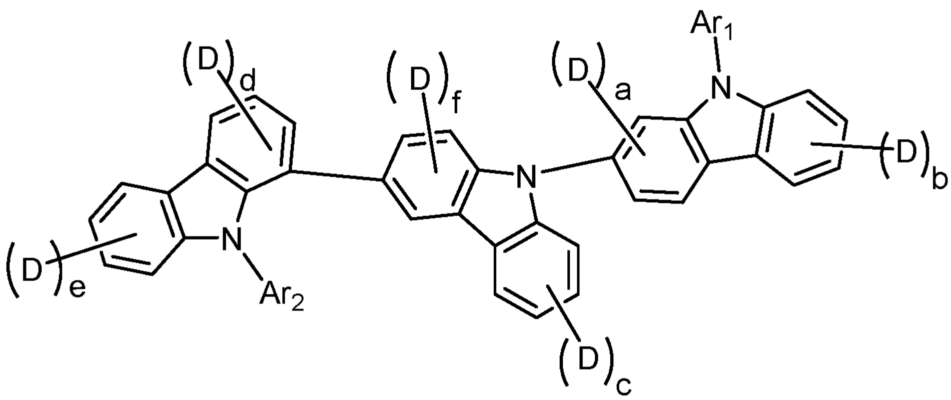

[Chemical Formula 16]

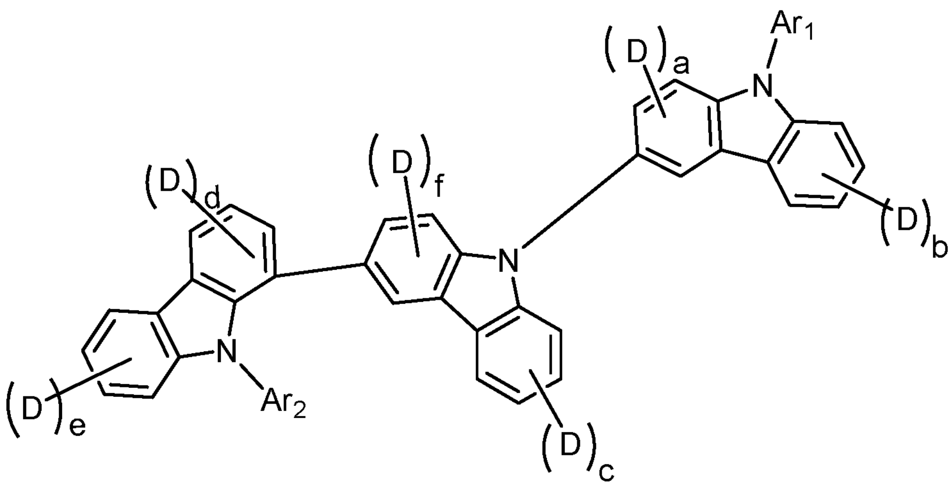

[Chemical Formula 17]

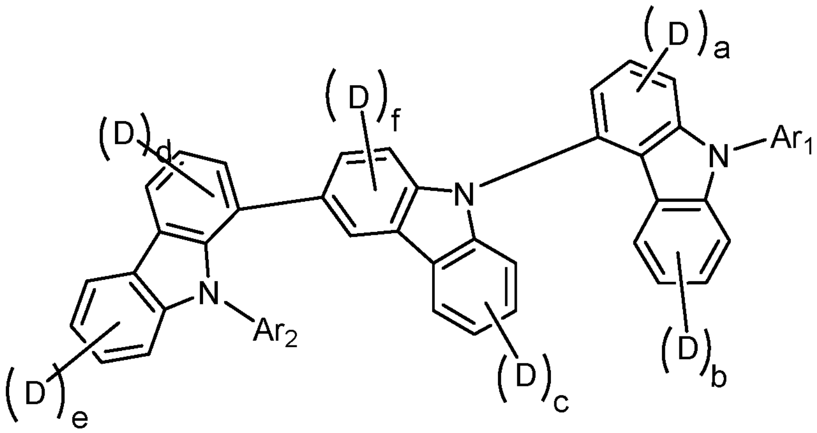

wherein the chemical formulas,

$Ar_1$, $Ar_2$, a, b, c, d, e, and f are each as defined in Chemical Formula 1.

The compounds represented by Chemical Formulas 2 to 17 according to the present invention are triscarbazole type materials, in which one carbazole is added to existing a bis-type carbazole, and can further maximize the hole stability of the existing bis-type materials due to the additionally linked carbazole. This contributes to the performance optimization of elements. Furthermore, the thermal stability of the material itself can be maximized by deuteration of the main skeletal structure of the material. All of the compounds represented by Chemical Formulas 2 to 17 can achieve the hole stability improving effect as described above, and preferably, the compounds represented by Chemical Formulas 6 to 9 can ensure better hole stability in view of structure. More specifically, such a compound may be represented by Chemical Formula 8, in which at least three carbazole groups are linked at position 3, an active site, of each carbazole group.

The compound represented by Chemical Formula 1 according to present invention described above may be further embodied as a compound represented by any one of Compounds A-1 to U-1 exemplified below. However, the compound represented by Chemical Formula 1 of the present invention is not limited to those exemplified below.

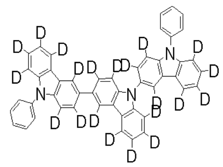

A-1

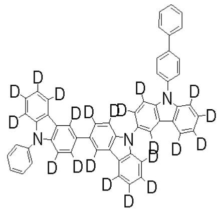

A-2

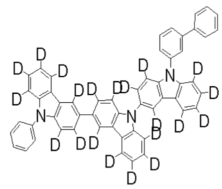

A-3

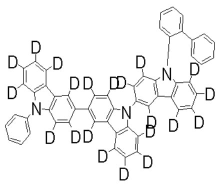

A-4

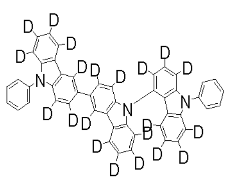

A-5

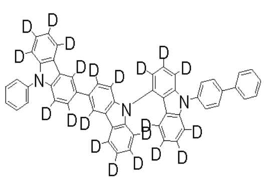

A-6

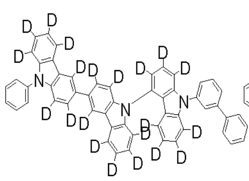

A-7

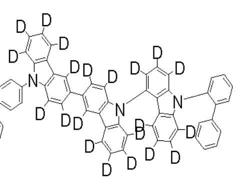

A-8

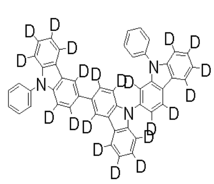

A-9

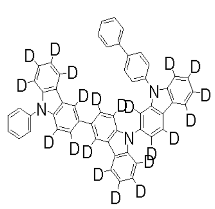

A-10

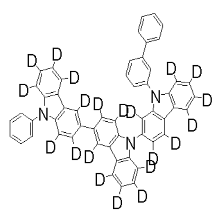

A-11

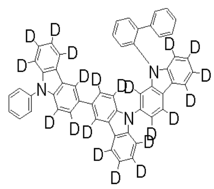

A-12

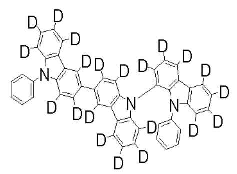
A-13

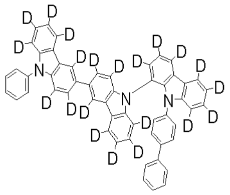
A-14

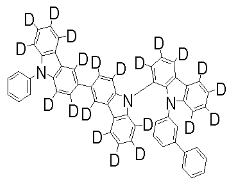
A-15

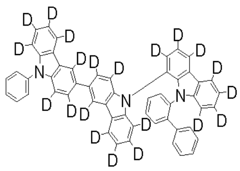
A-16

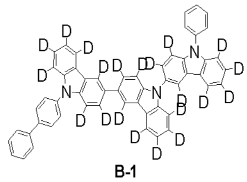
B-1

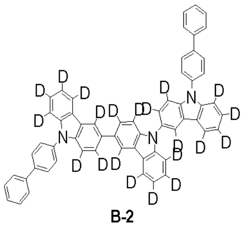
B-2

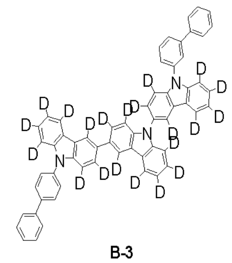
B-3

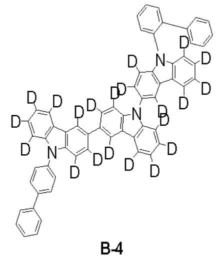
B-4

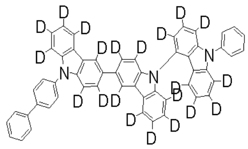
B-5

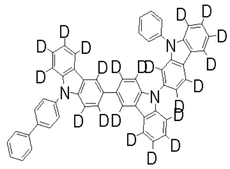
B-6

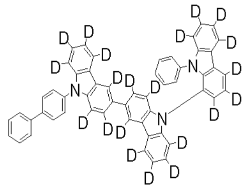
B-7

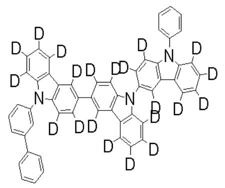
C-1

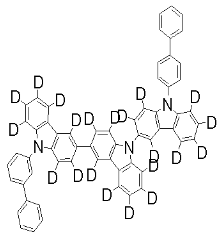
C-2

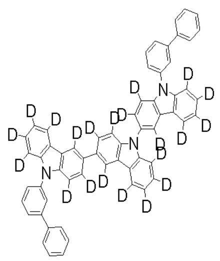
C-3

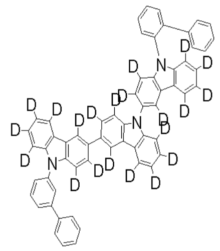
C-4

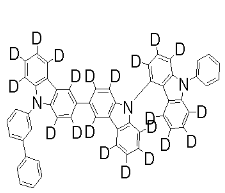
C-5

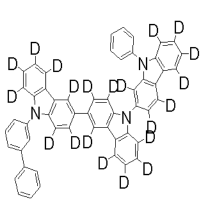
C-6

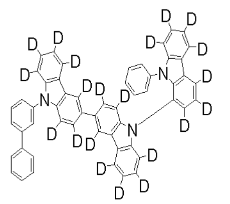
C-7

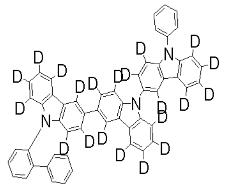
D-1

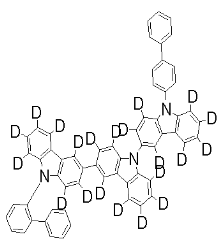
D-2

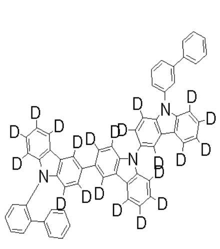
D-3

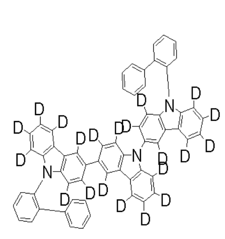
D-4

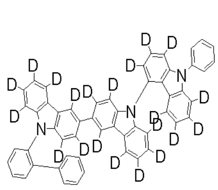
D-5

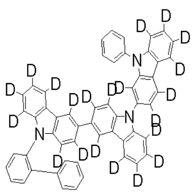
D-6

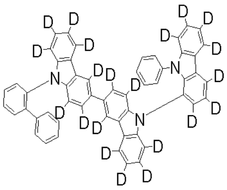
D-7

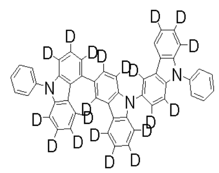
E-1

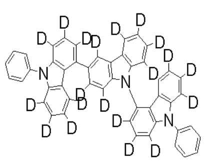
E-2

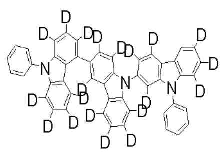
E-3

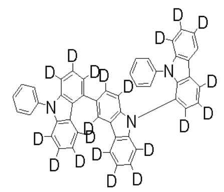
E-4

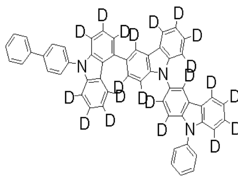
F-1

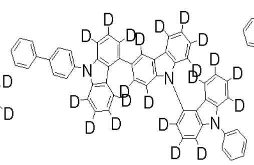
F-2

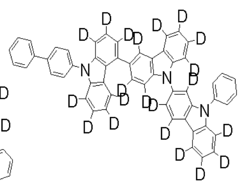
F-3

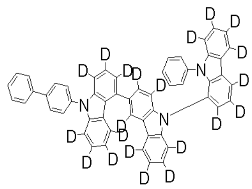
F-4

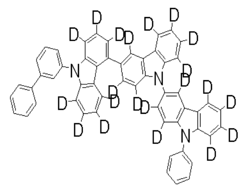
G-1

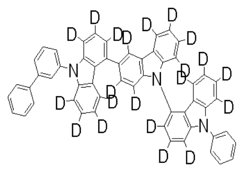
G-2

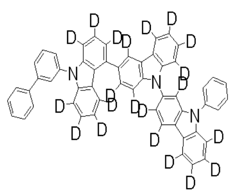
G-3

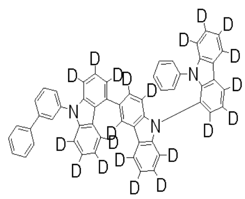
G-4

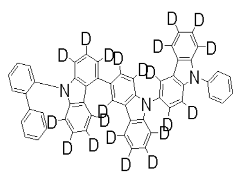
H-1

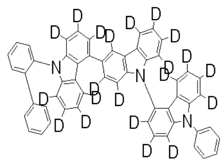
H-2

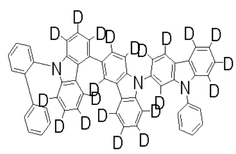
H-3

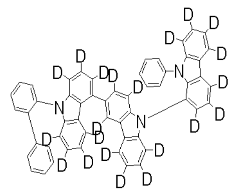
H-4

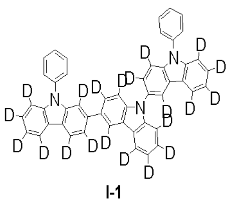
I-1

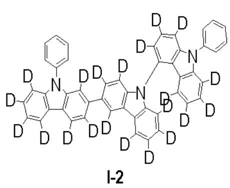
I-2

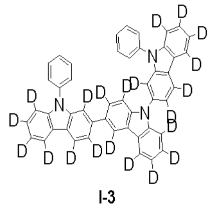
I-3

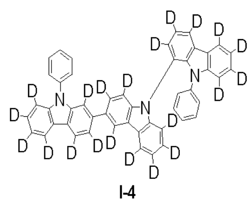
I-4

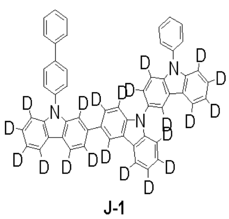
J-1

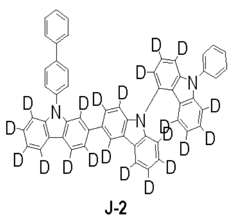
J-2

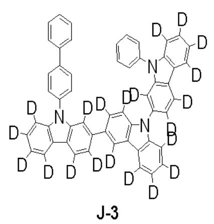
J-3

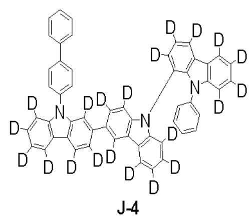
J-4

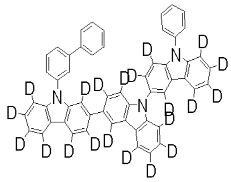
K-1

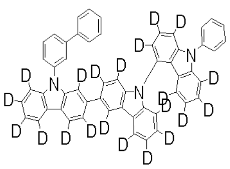
K-2

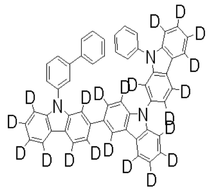
K-3

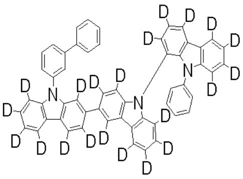
K-4

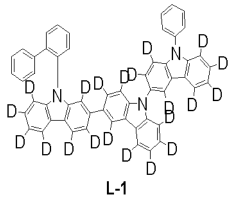
L-1

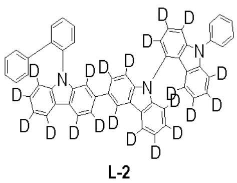
L-2

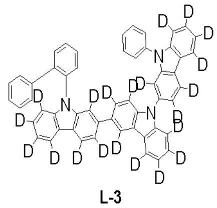
L-3

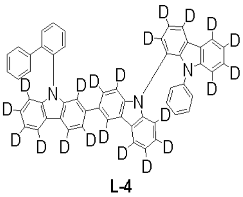
L-4

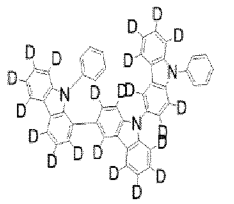
M-1

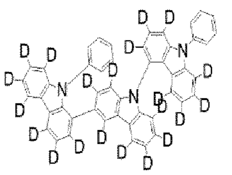
M-2

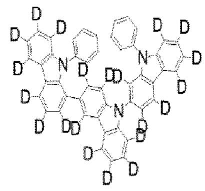
M-3

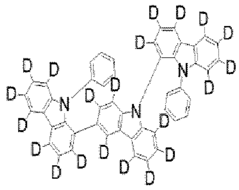
M-4

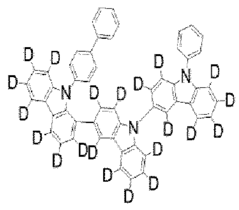
N-1

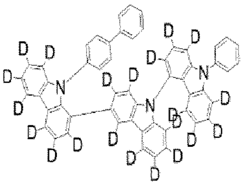
N-2

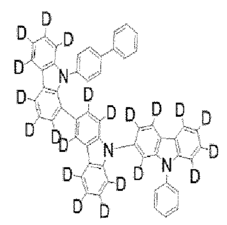
N-3

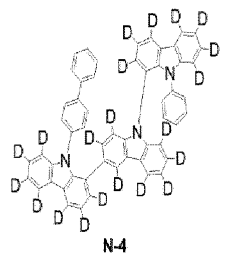
N-4

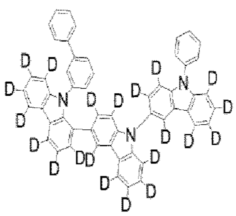
O-1

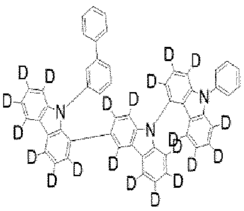
O-2

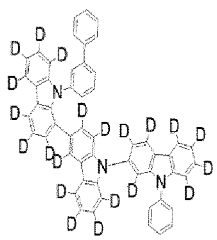
O-3

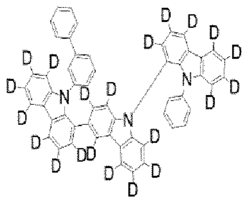
O-4

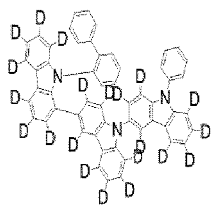
P-1

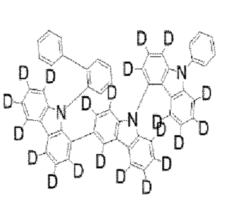
P-2

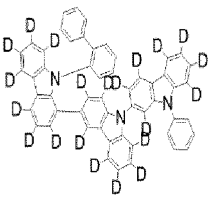
P-3

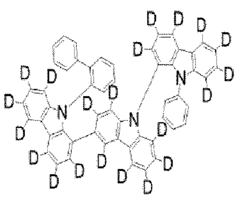
P-4

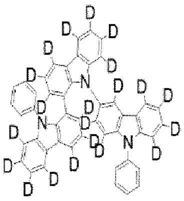

Q-1

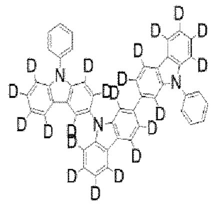

R-1

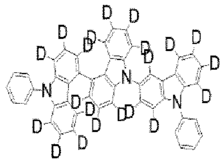

S-1

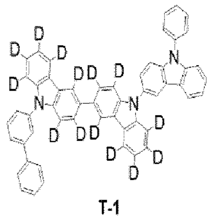

T-1

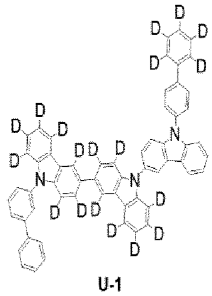

U-1

As used herein, the term "alkyl" refers to a monovalent substituent derived from a linear or branched, saturated hydrocarbon having 1 to 40 carbon atoms. Examples thereof may include methyl, ethyl, propyl, isobutyl, sec-butyl, pentyl, iso-amyl, hexyl, and the like, but are not limited thereto.

As used herein, the term "alkenyl" refers to a monovalent substituent derived from a linear or branched, unsaturated hydrocarbon having 2 to 40 carbon atoms having one or more carbon-carbon double bonds. Examples thereof may include vinyl, allyl, isopropenyl, 2-butenyl, and the like, but are not limited thereto.

As used herein, the term "alkynyl" refers to a monovalent substituent derived from a linear or branched, unsaturated hydrocarbon having 2 to 40 carbon atoms with one or more carbon-carbon triple bonds. Examples thereof may include ethynyl, 2-propynyl, and the like, but are not limited thereto.

As used herein, the term "cycloalkyl" refers to a monovalent substituent derived from a monocyclic or polycyclic non-aromatic hydrocarbon having 3 to 40 carbon atoms. Examples of the cycloalkyl may include cyclopropyl, cyclopentyl, cyclohexyl, norbornyl, adamantine, and the like, but are not limited thereto.

As used herein, the term "heterocycloalkyl" refers to a monovalent substituent derived from a non-aromatic hydrocarbon having 3 to 40 nuclear atoms, of which one or more carbon atoms on the ring, preferably 1 to 3 carbon atoms, are substituted with a heteroatom, such as N, O, S, or Se. Examples of the heterocycloalkyl may include morpholine, piperazine, and the like, but are not limited thereto.

As used herein, the term "aryl" refers to a monovalent substituent derived from an aromatic hydrocarbon having 6 to 60 carbon atoms, in which a single ring or two or more rings are combined. Additionally, the aryl may also include a form in which two or more rings are simply pendant to each other or condensed to each other. Examples of the aryl may include phenyl, naphthyl, phenanthryl, anthryl, and the like, but are not limited thereto.

As used herein, the term "heteroaryl" refers to a monovalent substituent derived from a monoheterocyclic or polyheterocyclic aromatic hydrocarbon having 5 to 60 nuclear atoms. Particularly, at least one carbon atom, preferably one to three carbon atoms on the ring are substituted with a heteroatom, such as N, O, S, or Se. In addition, the heteroaryl may include a form in which two or more rings may be simply pendant to each other or condensed to each other, and may furthermore include a form of being condensed with an aryl group. Examples of the heteroaryl may include: 6-membered monocyclic rings, such as pyridyl, pyrazinyl, pyrimidinyl, pyridazinyl, and triazinyl; polycyclic rings, such as phenoxathienyl, indolizinyl, indolyl, purinyl, quinolyl, benzothiazole, carbazolyl, dibenzofuranyl, and dibenzothiophenyl; and 2-furanyl, N-imidazolyl, 2-isoxazolyl, 2-pyridinyl, 2-pyrimidinyl, and the like, but are not limited thereto.

As used herein, the "alkyloxy" refers to a monovalent substituent represented by R'O-, wherein R' means alkyl having 1 to 40 carbon atoms. This alkyloxy may include a linear, branched, or cyclic structure. Examples of the alkyloxy may include methoxy, ethoxy, n-propoxy, 1-propoxy, t-butoxy, n-butoxy, pentoxy, and the like, but are not limited thereto.

As used herein, the term "aryloxy" refers to a monovalent substituent represented by RO-, wherein R is aryl having 6 to 60 carbon atoms. Examples of the aryloxy may include phenyloxy, naphthyloxy, diphenyloxy, and the like, but are not limited thereto.

As used herein, the term "alkylsilyl" refers to a silyl substituted with alkyl having 1 to 40 carbon atoms, and the term "arylsilyl" refers to a silyl substituted with aryl having 6 to 60 carbon atoms

As used herein, the term "alkylboron group" refers to a boron substituted with alkyl having 1 to 40 carbon atoms, and the term "arylboron group" refers to a boron group substituted with aryl having 6 to 60 carbon atoms.

As used herein, the term "arylphosphine" refers to a phosphine substituted with aryl having 6 to 60 carbon atoms, and the term "arylphosphine oxide group" refers to a group where a phosphine substituted with aryl having 6 to 60 carbon

atoms contains O.

As used herein, the term "condensed ring" refers to a condensed aliphatic ring, a condensed aromatic, a condensed heteroaliphatic ring, a condensed heteroaromatic ring, or a combination thereof.

As used herein, the term "arylamine" refers to an amine substituted with an aryl having 6 to 60 carbon atoms.

The compound represented by Chemical Formula 1 of the present invention can be manufactured by a method known in the art, without limitation. For instance, the compounds may be variously synthesized with reference to synthetic schemes of the following examples.

<Organic electroluminescent element>

The present invention provides an organic electroluminescent element containing the compound represented by Chemical Formula 1.

Specifically, the organic electroluminescent element according to the present invention includes an anode, a cathode, and one or more organic layers interposed between the anode and the cathode, wherein at least one of the one or more organic layers contains the compound represented by Chemical Formula 1.

The one or more organic layers include at least one selected from the group consisting of an emissive layer, an auxiliary emissive layer, a hole injection layer, a hole transport layer, an electron injection layer, an electron transport layer, and an auxiliary electron transport layer, of which at least one organic layer contains the compound represented by Chemical Formula 1. More specifically, the organic layer containing the compound of the Chemical Formula 1 is the emissive layer, wherein the compound of Chemical Formula 1 is contained as a host material.

The emissive layer may contain a host material and/or a dopant material, wherein the compound of Chemical Formula 1 may be used as a P-type host material. The emissive layer may further contain at least one of common P-type hosts and N-type hosts known in the art, in addition to the above-described host material of Chemical Formula 1. Alternatively, a dopant known in the art may be further contained without limitation. The content ratio (mixing ratio) of these is not particularly limited, and may be appropriately adjusted within a content range known in the art. For instance, the emissive layer may contain 70 to 99.9 parts by weight of a host and 0.1 to 30 parts by weight of a dopant relative to the total weight thereof.

The dopant contained in the emissive layer is not particularly limited as long as it is known in the art, and examples thereof may include at least one of a fluorescent emissive dopant and a phosphorescent emissive dopant. Non-limiting examples of usable dopants may include anthracene derivatives, pyrene derivatives, arylamine derivatives, metal complex compounds containing iridium (Ir) or platinum (Pt), and the like. The dopants may be classified into red, green, and blue dopants, and red, green, and red dopants, green dopants, and blue dopants commonly known in the art may be used without particular limitation.

The organic electroluminescent element according to the present invention may include both of an emissive layer (EML), which contains the compound of Chemical Formula 1 as a host, and a dual EWG-type electron transport layer (ETL), which has high electron transporting ability by containing at least two EWGs. Such a case can optimize the performance of the organic the electroluminescent element through the improvement of hole stability of the emissive layer (EML) and the maximization of electron transporting ability of the electron transport layer (ETL).

The electron transport layer is not particularly limited as long as it employs a compound containing at least two electron withdrawing groups (EWGs), and specifically, the electron transport layer may contain a compound represented by Chemical Formula 18 below.

[Chemical Formula 18]

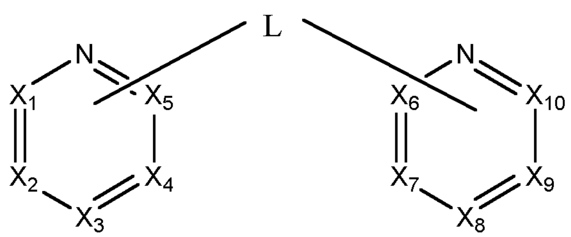

wherein Chemical Formula 18,

L may be selected from the group consisting of a $C_6$ to $C_{40}$ arylene group and a heteroarylene group having 5 to 40 nuclear atoms;

$X_1$ to $X_{10}$ may each be independently N or $C(R_1)$;

wherein when $C(R_1)$ is plural in number, the plurality of $R_1$ may be the same or different from each other and may each

be independently selected from the group consisting of a $C_1$ to $C_{40}$ alkyl group, a $C_2$ to $C_{40}$ alkenyl group, a $C_2$ to $C_{40}$ alkynyl group, a $C_6$ to $C_{40}$ aryl group, a heteroaryl group having 5 to 40 nuclear atoms, a $C_6$ to $C_{40}$ aryloxy group, a $C_1$ to $C_{40}$ alkyloxy group, a $C_6$ to $C_{40}$ arylamine group, a $C_3$ to $C_{40}$ cycloalkyl group, a heterocycloalkyl group having 3 to 40 nuclear atoms, a $C_1$ to $C_{40}$ alkylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylsilyl group; and

the arylene group and heteroarylene group of L, and the alkyl group, alkenyl group, alkynyl group, aryl group, heteroaryl group, aryloxy group, alkyloxy group, arylamine group, cycloalkyl group, heterocycloalkyl group, alkylsilyl group, alkylboron group, arylboron group, arylphosphine group, arylphosphine oxide group and arylsilyl group of $R_1$ may each be independently substituted with at least one substituent selected from the group consisting of deuterium (D), halogen, a cyano group, a nitro group, a $C_1$ to $C_{40}$ alkyl group, a $C_2$ to $C_{40}$ alkenyl group, a $C_2$ to $C_{40}$ alkynyl group, a $C_3$ to $C_{40}$ cycloalkyl group, a heterocycloalkyl group having 3 to 40 nuclear atoms, a $C_6$ to $C_{60}$ aryl group, a heteroaryl group having 5 to 60 nuclear atoms, a $C_1$ to $C_{40}$ alkyloxy group, a $C_6$ to $C_{60}$ aryloxy group, a $C_1$ to $C_{40}$ alkylsilyl group, a $C_6$ to $C_{60}$ arylsilyl group, a $C_1$ to $C_{40}$ alkylboron group, a $C_6$ to $C_{60}$ arylboron group, a $C_6$ to $C_{60}$ arylphosphine group, a $C_6$ to $C_{60}$ arylphosphine oxide group, and a $C_6$ to $C_{60}$ arylamine group, and when the substituents are plural in number, the substituents may be the same or different from each other.

In Chemical Formula 18, each of the $X_1$ to $X_5$ containing ring and the $X_6$ to $X_{10}$ containing ring is contained as an EWG. The $X_1$ to $X_5$ containing ring and the $X_6$ to $X_{10}$ containing ring may be the same or different from each other and may each be embodied as any one of substituents A-1 to A-5.

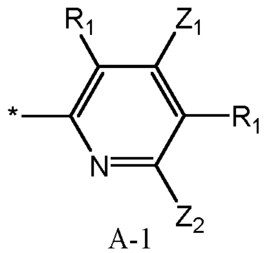
A-1

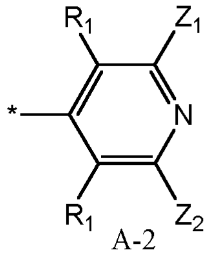
A-2

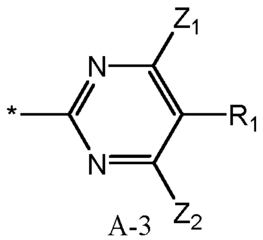
A-3

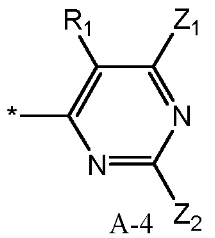
A-4

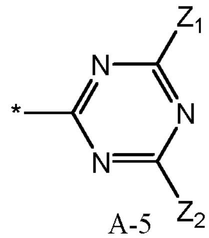
A-5

wherein the chemical formulas,

the mark * is a site linked to Chemical Formula 18.

$Z_1$ and $Z_2$ may be the same or different from each other and may each be independently selected from the group consisting of a $C_6$ to $C_{40}$ aryl group and a heteroaryl group having 5 to 40 nuclear atoms;

each $R_1$ may be independently selected from the group consisting of hydrogen, a $C_1$ to $C_{40}$ alkyl group, a $C_6$ to $C_{40}$ aryl group, and a heteroaryl group having 5 to 40 nuclear atoms; and

the aryl group and heteroaryl group of $Z_1$ to $Z_2$, the alkyl group, aryl group, and heteroaryl group of $R_1$ may each be independently substituted with at least one substituent selected from the group consisting of deuterium (D), halogen, a cyano group, a $C_6$ to $C_{40}$ aryl group, and a heteroaryl group having 5 to 40 nuclear atoms, and when the substituents are plural in number, the substituents may be the same or different from each other.

In an embodiment, $Z_1$ and $Z_2$ may be the same or different from each other and may each be independently further embodied as any one selected from the structural formulas below:

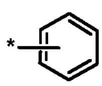

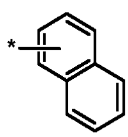

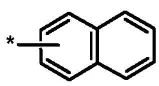

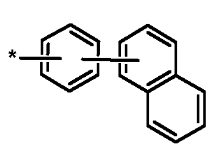

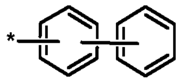
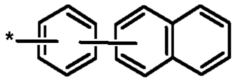
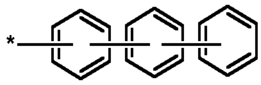
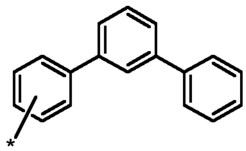
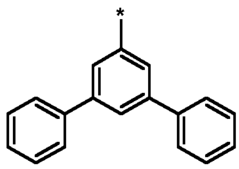
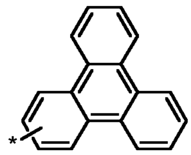
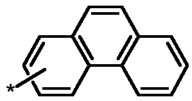
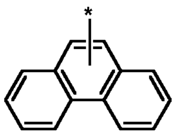
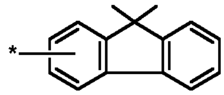
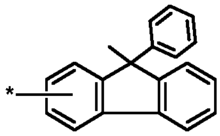
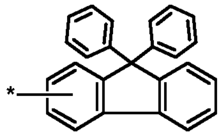
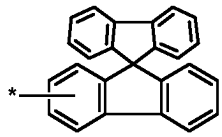
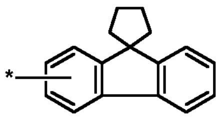
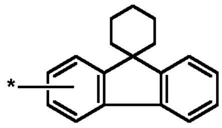
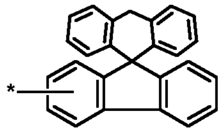
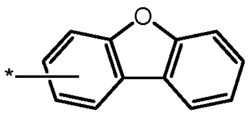
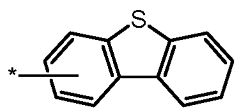
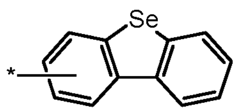
.

The above-described two EWGs are linked via a linker (L). These linkers are linkers of common divalent groups known in the art, and may be specifically selected from a $C_6$ to $C_{25}$ arylene groups and a heteroarylene group having 5 to 18 nuclear atoms.

In an embodiment, L may be further embodied as a linker selected from the following structural formulas. However, the linker is not limited thereto.

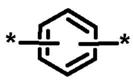
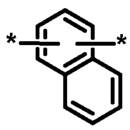
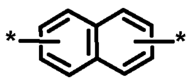
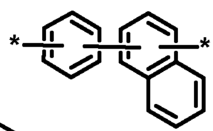
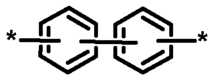
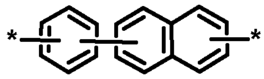
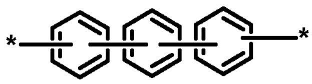
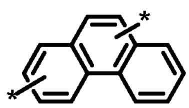
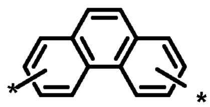
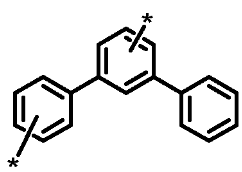
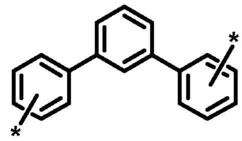
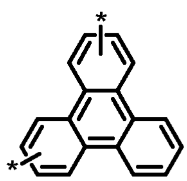

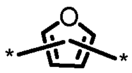 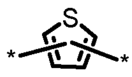 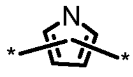 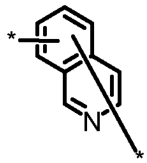 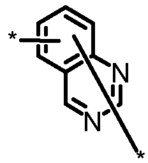

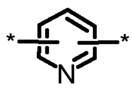 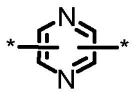 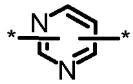 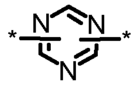

wherein the structural formulas,

the mark * is a site linked to Chemical Formula 18. Although not shown in the above-described structural formulas, at least one substituent (e.g., the same as the definition of $R_1$) known in the art may be substituted.

The structure of the organic electroluminescent element of the present invention including the above-described configuration is not particularly limited, and may have a configuration known in the art. As a non-limiting example, the organic electroluminescent element of the present invention may have a structure in which a substrate, an anode, a hole injection layer, a hole transport layer, an auxiliary emissive layer, an emissive layer, an electron transport layer, an electron injection layer, and a cathode are sequentially laminated. Particularly, in the structure of the organic electroluminescent element of the present invention, an insulating layer or an adhesive layer may be inserted at the interface between an electrode and an organic layer.

The organic electroluminescent element according to the present invention may be manufactured by forming organic layers and electrodes using materials and methods known in the art, except that at least one of the organic layers contains the compound represented by Chemical Formula 1.

The organic layer may be formed by vacuum deposition or solution application. Examples of the solution application may include spin coating, dip coating, doctor blading, inkjet printing, thermal transfer, and the like, but are not limited thereto.

The substrate used in manufacturing the organic electroluminescent element of the present invention is not particularly limited, but examples thereof may include a silicon wafer, quartz, a glass plate, a metal plate, a plastic film, a sheet, and the like.

Examples of an anode material include: a metal, such as vanadium, chromium, copper, zinc, or gold, or an alloy thereof; a metal oxide, such as zinc oxide, indium oxide, indium tin oxide (ITO), or indium zinc oxide (IZO); a combination of a metal and an oxide, such as ZnO:Al or SnO2:Sb; a conductive polymer, such as polythiophene, poly(3-methylthiophene), poly[3,4-(ethylene-1,2-dioxy)thiophene] (PEDT), polypyrrole, or polyaniline; and carbon black, but are not limited thereto.

Examples of a cathode material include a metal, such as magnesium, calcium, sodium, potassium, titanium, indium, yttrium, lithium, gadolinium, aluminum, silver, tin, or lead, or an alloy thereof; and a multilayer-structured material, such as LiF/Al or LiO2/Al, but are not limited thereto.

Additionally, the hole injection layer, the hole transport layer, and the electron injection layer are not particularly limited, and common materials known in the art may be used therefor.

Hereinafter, the present invention will be described in detail with reference to examples. However, the following examples are merely for illustrating the present invention and are not intended to limit the scope of the present invention.

## <Preparation Example 1-1> Synthesis of Cz-D1

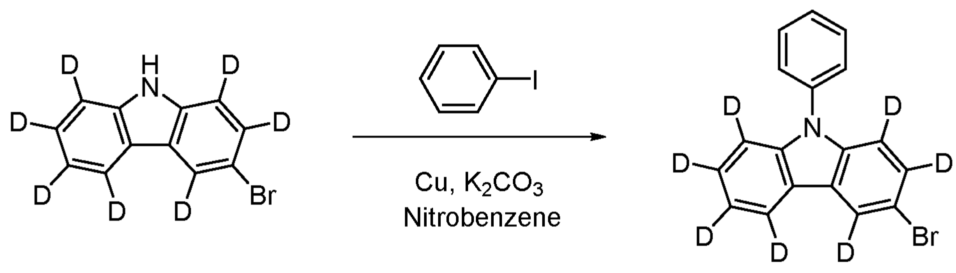


Cz-D1

Under a nitrogen atmosphere, 3-bromo-9H-carbazole-1,2,4,5,6,7,8-d7 (134.3 g, 530.6 mmol), Iodobenzene (130.0 g, 636.7 mmol), Cu (16.8 g, 265.3 mmol), $K_2CO_3$ (146.7 g, 1,061.3 mmol), and toluene (1000 ml) were mixed, and stirred at 110°C for 12 hours.

After the reaction was completed, extraction was conducted with ethyl acetate, moisture was removed over $MgSO_4$, and then purification was performed by column chromatography (hexane:EA=5:1 (v/v)), thereby obtaining Cz-D1 (125.7 g, yield: 72%).

Mass (calcd.: 329.25, found: 329 g/mol)

**<Preparation Example 1-2> Synthesis of Cz-D2**

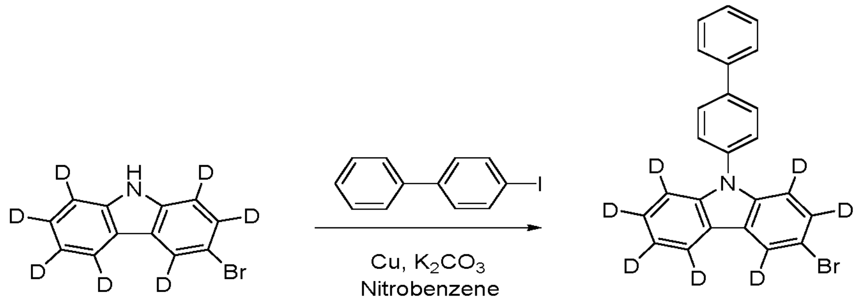


Cz-D2

The same procedure as in Preparation Example 1-1 was performed except that 4-iodo-1,1'-biphenyl (178.3 g, 636.7 mmol) was used instead of iodobenzene, thereby obtaining the target compound Cz-D2 (135.5 g, yield: 63%).

Mass (calcd.: 405.35, found: 405 g/mol)

**<Preparation Example 1-3> Synthesis of Cz-D3**

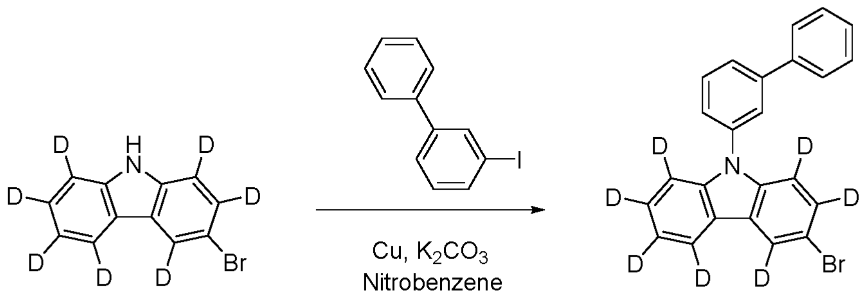


Cz-D3

The same procedure as in Preparation Example 1-1 was performed except that 3-iodo-1,1'-biphenyl (178.3 g, 636.7 mmol) was used instead of iodobenzene, thereby obtaining the target compound Cz-D3 (148.4 g, yield: 69%).

Mass (calcd.: 405.35, found: 405 g/mol)

**<Preparation Example 1-4> Synthesis of Cz-D4**

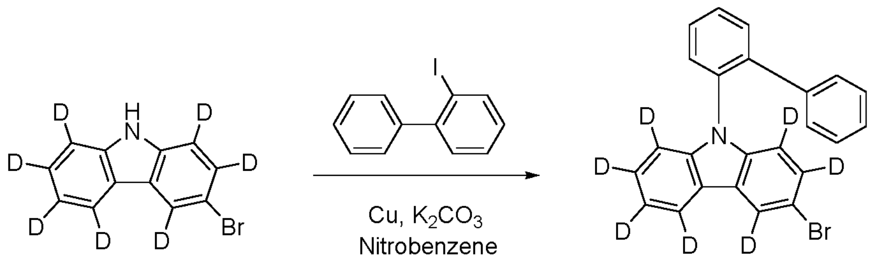


Cz-D4

The same procedure as in Preparation Example 1-1 was performed except that 2-iodo-1,1'-biphenyl (178.3 g, 636.7 mmol) was used instead of iodobenzene, thereby obtaining the target compound Cz-D4 (96.8 g, yield: 45%).
Mass (calcd.: 405.35, found: 405 g/mol)

**<Preparation Example 2-1> Synthesis of Cz-D5**

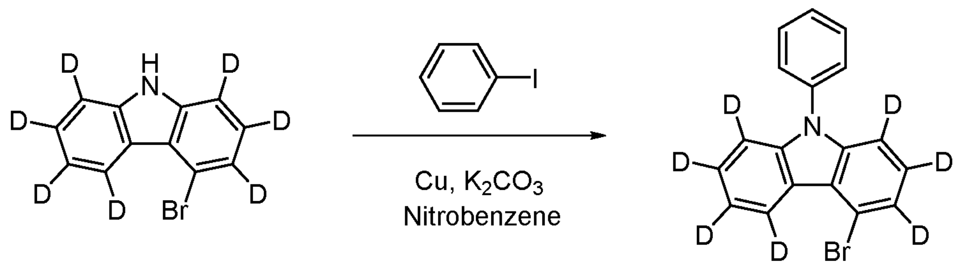


Cz-D5

The same procedure as in Preparation Example 1-1 was performed except that 4-bromo-9H-carbazole-1,2,4,5,6,7,8-d7 (134.3 g, 530.6 mmol) was used instead of 3-bromo-9H-carbazole-1,2,3,5,6,7,8-d7, thereby obtaining the target compound Cz-D5 (117.1 g, yield: 67%).
Mass (calcd.: 329.25, found: 329 g/mol)

**<Preparation Example 2-2> Synthesis of Cz-D6**

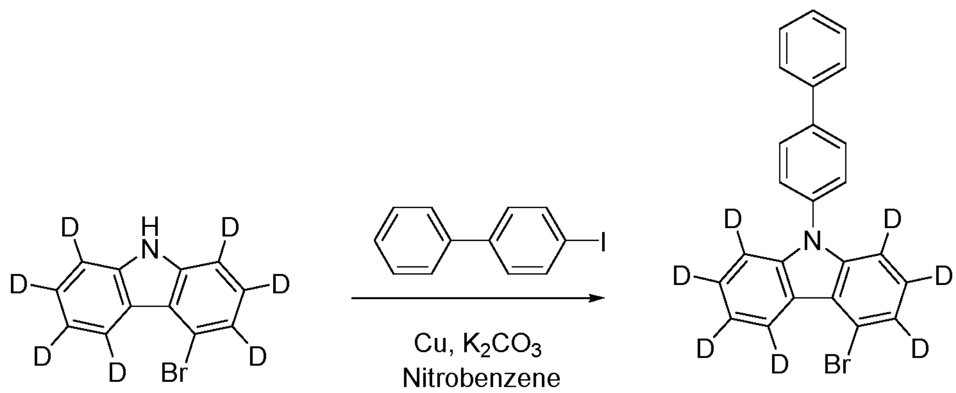


Cz-D6

The same procedure as in Preparation Example 2-1 was performed except that 4-iodo-1,1'-biphenyl (178.3 g, 636.7 mmol) was used instead of iodobenzene, thereby obtaining the target compound Cz-D6 (139.8 g, yield: 65%).
Mass (calcd.: 405.35, found: 405 g/mol)

**<Preparation Example 2-3> Synthesis of Cz-D7**

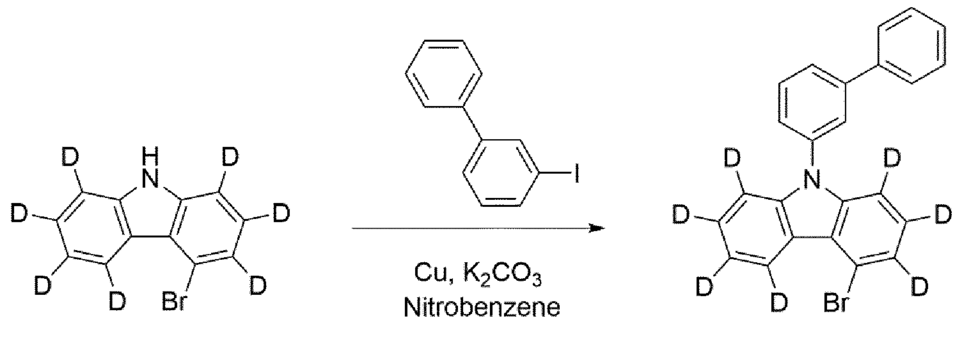


Cz-D7

The same procedure as in Preparation Example 2-1 was performed except that 3-iodo-1,1'-biphenyl (178.3 g, 636.7 mmol) was used instead of iodobenzene, thereby obtaining the target compound Cz-D7 (152.7 g, yield: 71%). Mass (calcd.: 405.35, found: 405 g/mol)

**<Preparation Example 2-4> Synthesis of Cz-D8**

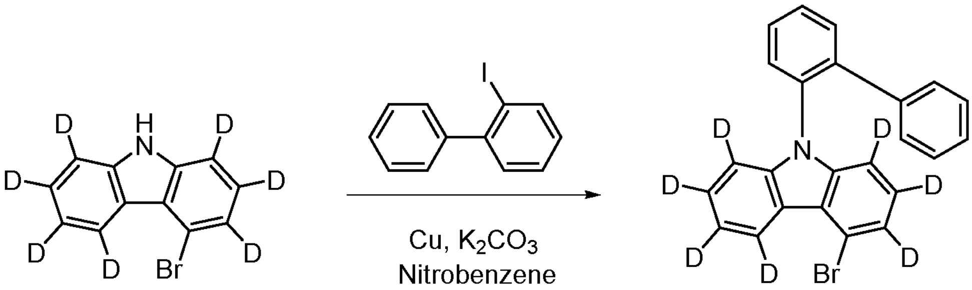


Cz-D8

The same procedure as in Preparation Example 2-1 was performed except that 2-iodo-1,1'-biphenyl (178.3 g, 636.7 mmol) was used instead of iodobenzene, thereby obtaining the target compound Cz-D8 (75.2 g, yield: 35%). Mass (calcd.: 405.35, found: 405 g/mol)

**<Preparation Example 3-1> Synthesis of Cz-D9**

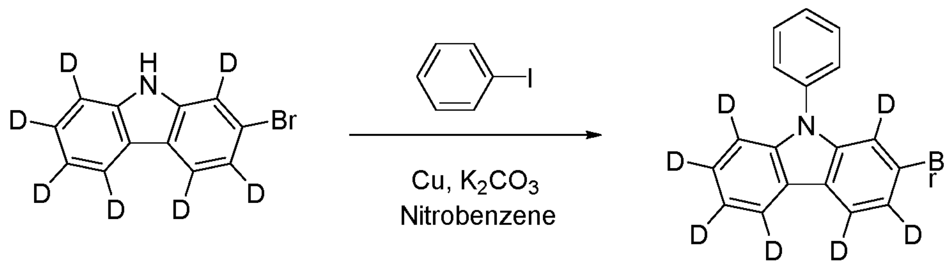


Cz-D9

The same procedure as in Preparation Example 1-1 was performed except that 2-bromo-9H-carbazole-1,3,4,5,6,7,8-d7 (134.3 g, 530.6 mmol) was used instead of 3-bromo-9H-carbazole-1,2,4,5,6,7,8-d7, thereby

obtaining the target compound Cz-D9 (134.5 g, yield: 77%).
Mass (calcd.: 329.25, found: 329 g/mol)

**<Preparation Example 3-2> Synthesis of Cz-D10**

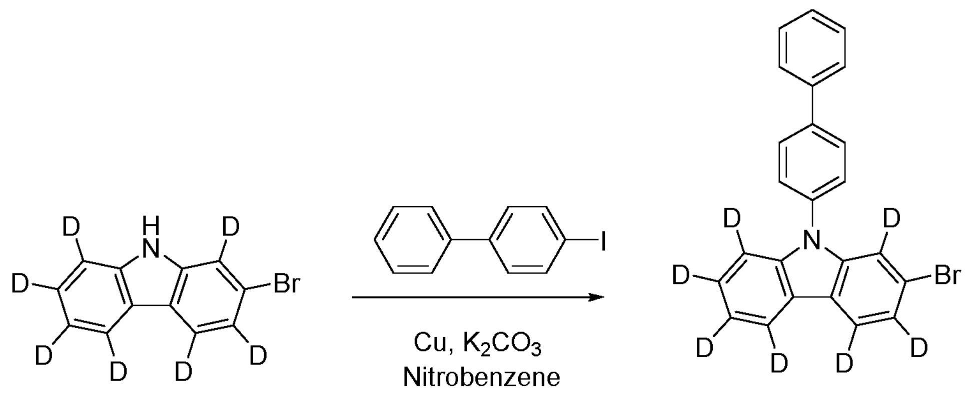


Cz-D10

The same procedure as in Preparation Example 3-1 was performed except that 4-iodo-1,1'-biphenyl (178.3 g, 636.7 mmol) was used instead of iodobenzene, thereby obtaining the target compound Cz-D10 (159.1 g, yield: 74%). Mass (calcd.: 405.35, found: 405 g/mol)

**<Preparation Example 3-3> Synthesis of Cz-D11**

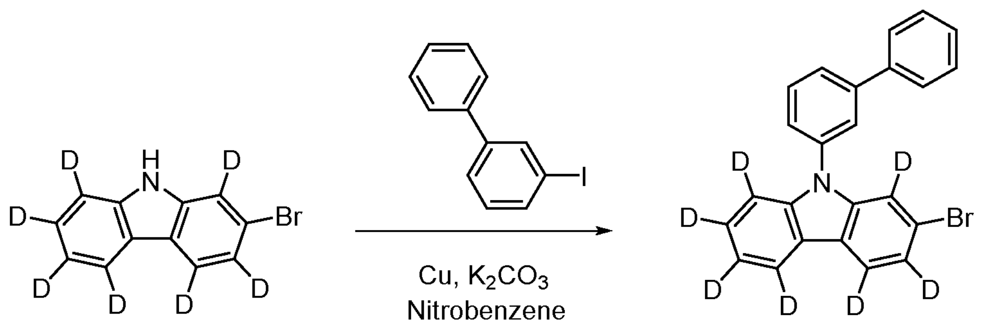


Cz-D11

The same procedure as in Preparation Example 3-1 was performed except that 3-iodo-1,1'-biphenyl (178.3 g, 636.7 mmol) was used instead of iodobenzene, thereby obtaining the target compound Cz-D11 (163.4 g, yield: 76%). Mass (calcd.: 405.35, found: 405 g/mol)

**<Preparation Example 3-4> Synthesis of Cz-D12**

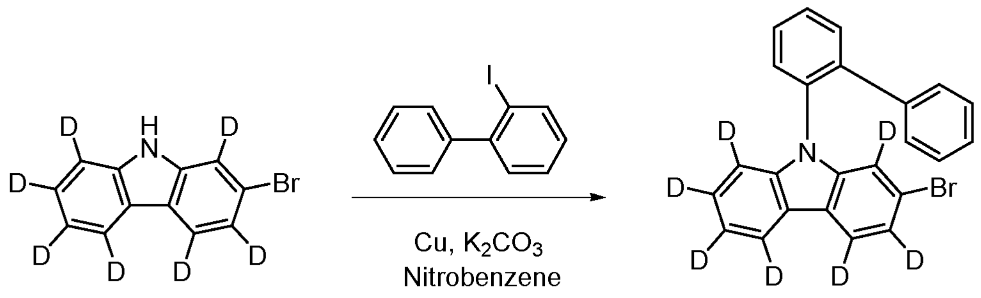


Cz-D12

The same procedure as in Preparation Example 3-1 was performed except that 2-iodo-1,1'-biphenyl (178.3 g, 636.7 mmol) was used instead of iodobenzene, thereby obtaining the target compound Cz-D12 (92.4 g, yield: 43%). Mass (calcd.: 405.35, found: 405 g/mol)

**<Preparation Example 4-1> Synthesis of Cz-D13**

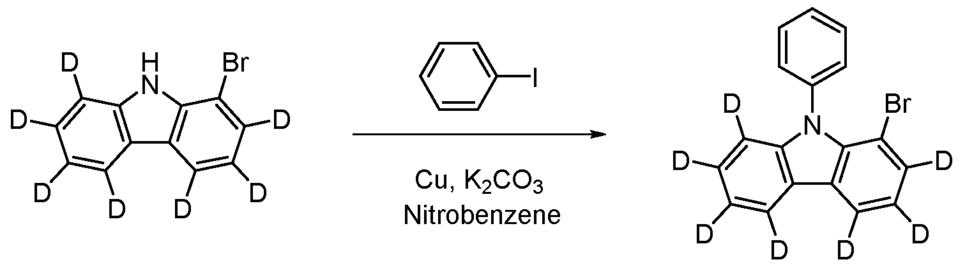


Cz-D13

The same procedure as in Preparation Example 1-1 was performed except that 1-bromo-9H-carbazole-2,3,4,5,6,7,8-d7 (134.3 g, 530.6 mmol) was used instead of 3-bromo-9H-carbazole-1,2,4,5,6,7,8-d7, thereby obtaining the target compound Cz-D13 (94.3 g, yield: 54%).
Mass (calcd.: 329.25, found: 329 g/mol)

**<Preparation Example 4-2> Synthesis of Cz-D14**

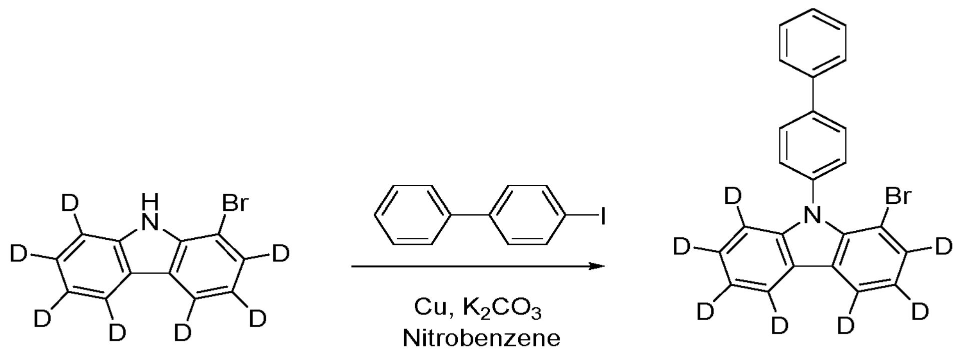


Cz-D14

The same procedure as in Preparation Example 4-1 was performed except that 4-iodo-1,1'-biphenyl (178.3 g, 636.7 mmol) was used instead of iodobenzene, thereby obtaining the target compound Cz-D14 (122.6 g, yield: 57%).
Mass (calcd.: 405.35, found: 405 g/mol)

**<Preparation Example 4-3> Synthesis of Cz-D15**

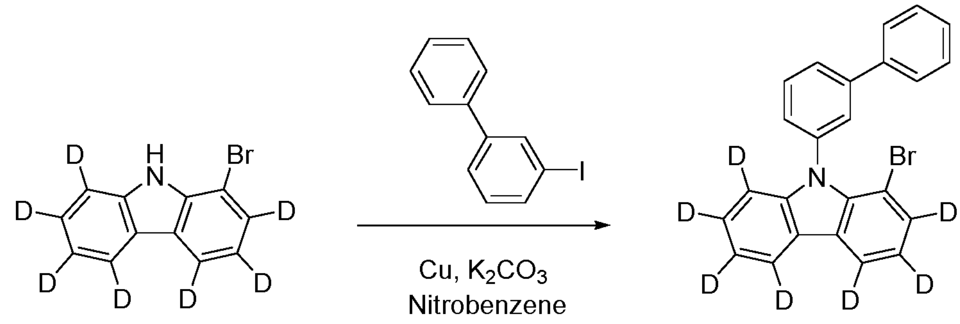


Cz-D15

The same procedure as in Preparation Example 4-1 was performed except that 3-iodo-1,1'-biphenyl (178.3 g, 636.7 mmol) was used instead of iodobenzene, thereby obtaining the target compound Cz-D15 (111.8 g, yield: 52%). Mass (calcd.: 405.35, found: 405 g/mol)

**<Preparation Example 4-4> Synthesis of Cz-D16**

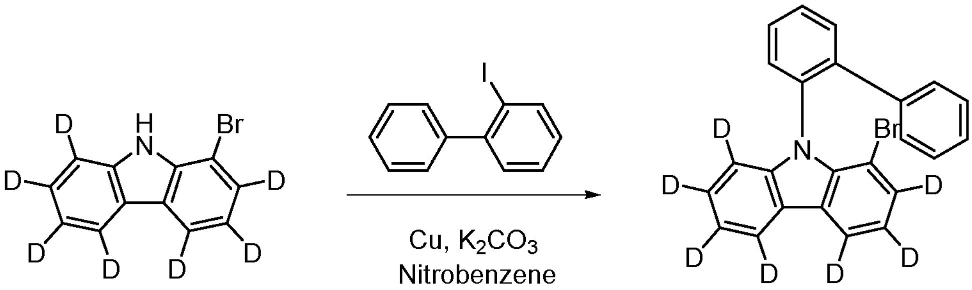


Cz-D16

The same procedure as in Preparation Example 4-1 was performed except that 2-iodo-1,1'-biphenyl (178.3 g, 636.7 mmol) was used instead of iodobenzene, thereby obtaining the target compound Cz-D16 (68.8 g, yield: 32%). Mass (calcd.: 405.35, found: 405 g/mol)

**<Preparation Example 5-1> Synthesis of BCz-D1**

<Step 5-1-1> Synthesis of 9-phenyl-3-(4,4,5,5-tetramethyl-1,3,2-dioxaborolan-2-yl)-9H-carbazole-1,2,4,5,6,7,8-d7

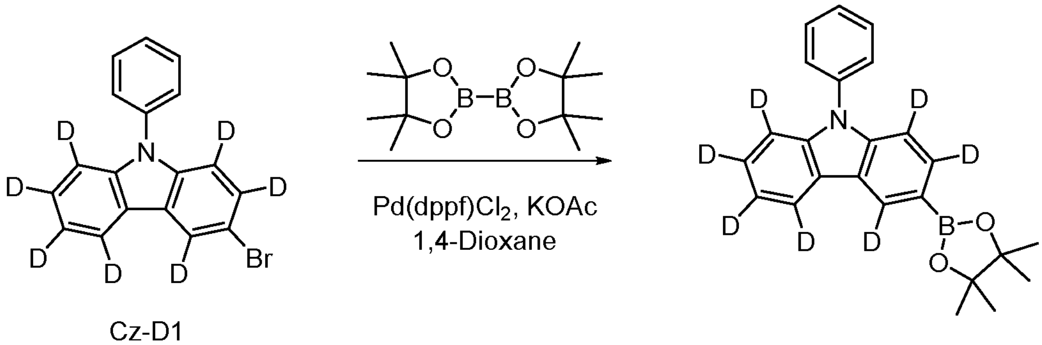


Cz-D1

Under a nitrogen atmosphere, Cz-D1 (100.0 g, 303.7 mmol), 4,4,4',4',5,5, 5',5'-octamethyl-2,2'-bi(1,3,2-dioxaborolane) (84.8 g, 334.1 mmol), Pd(dppf)$Cl_2$ (26.6 g, 30.3 mmol), KOAc (85.8 g, 911.1 mmol), and 1,4-Dioxane (1000 ml) were mixed, and stirred at 130°C for 12 hours. After the reaction was completed, extraction was conducted with ethyl acetate, moisture was removed over $MgSO_4$, and then purification was performed by column chromatography (hexane:EA=8:1 (v/v)), thereby obtaining 9-phenyl-3-(4,4,5,5-tetramethyl-1,3,2-dioxaborolan-2-yl)-9H-carbazole-1,2,4,5,6,7,8-d7 (96.0 g, yield: 84%).
Mass (calcd.: 376.3, found: 376 g/mol)

<Step 5-1-2> Synthesis of BCz-D1

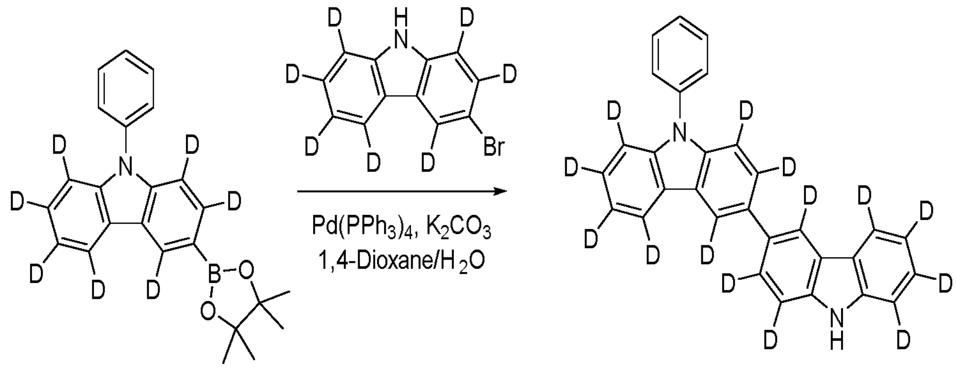


BCz-D1

Under a nitrogen atmosphere, 9-phenyl-3-(4,4,5,5-tetramethyl-1,3,2-dioxaborolan-2-yl)-9H-carbazole-1,2,4,5,6,7,8-d7 (96.0 g, 255.1 mmol), 3-bromo-9H-carbazole-1,2,4,5,6,7,8-d7 (77.5 g, 306.1 mmol), $Pd(PPh_3)_4$ (14.7 g, 12.7 mmol), $K_2CO_3$ (88.1 g, 637.8 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D1 (71.1 g, yield: 66%).
Mass (calcd.: 422.59, found: 422 g/mol)

**<Preparation Example 5-2> Synthesis of BCz-D2**

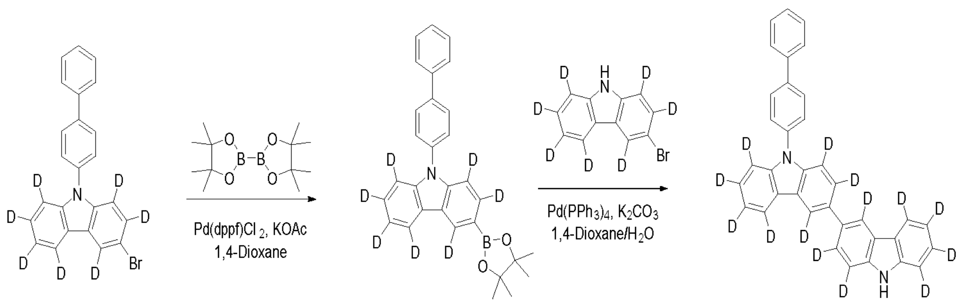


Cz-D2

BCz-D2

The same procedure as in Preparation Example 5-1 was performed except that Cz-D2 (100 g, 246.7 mmol) was used instead of Cz-D1, thereby obtaining the target compound BCz-D2 (66.4 g, yield: 54.0%).
Mass (calcd.: 498.69, found: 498 g/mol)

**<Preparation Example 5-3> Synthesis of BCz-D3**

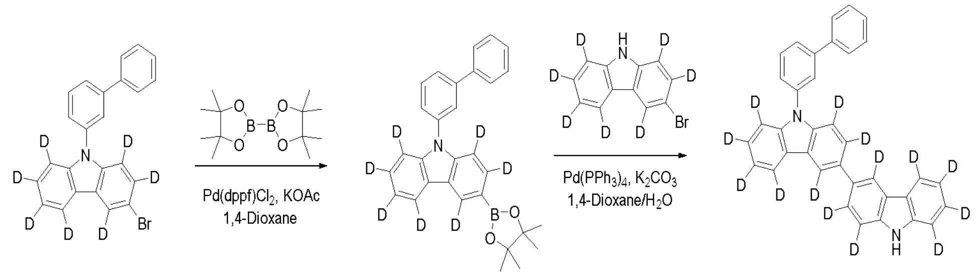


Cz-D3 BCz-D3

The same procedure as in Preparation Example 5-1 was performed except that Cz-D3 (100 g, 246.7 mmol) was used instead of Cz-D1, thereby obtaining the target compound BCz-D3 (59.7 g, yield: 48.5%).
Mass (calcd.: 498.69, found: 498 g/mol)

**<Preparation Example 5-4> Synthesis of BCz-D4**

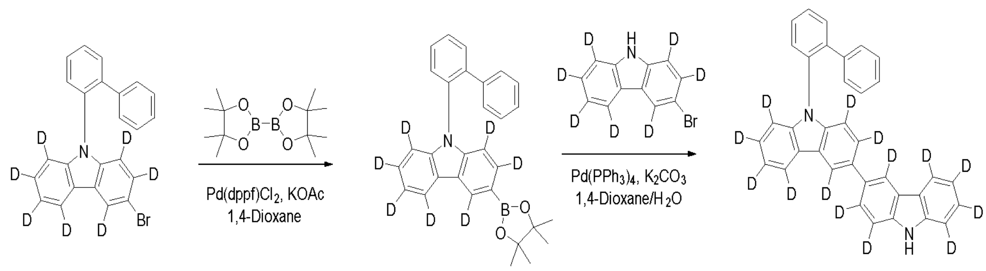


Cz-D4 BCz-D4

The same procedure as in Preparation Example 5-1 was performed except that Cz-D4 (100 g, 246.7 mmol) was used instead of Cz-D1, thereby obtaining the target compound BCz-D4 (59.4 g, yield: 48.3%).
Mass (calcd.: 498.69, found: 498 g/mol)

**<Preparation Example 6-1> Synthesis of BCz-D5**

<Step 6-1-1> Synthesis of 9-phenyl-4-(4,4,5,5-tetramethyl-1,3,2-dioxaborolan-2-yl)-9H-carbazole-1,2,3,5,6,7,8-d7

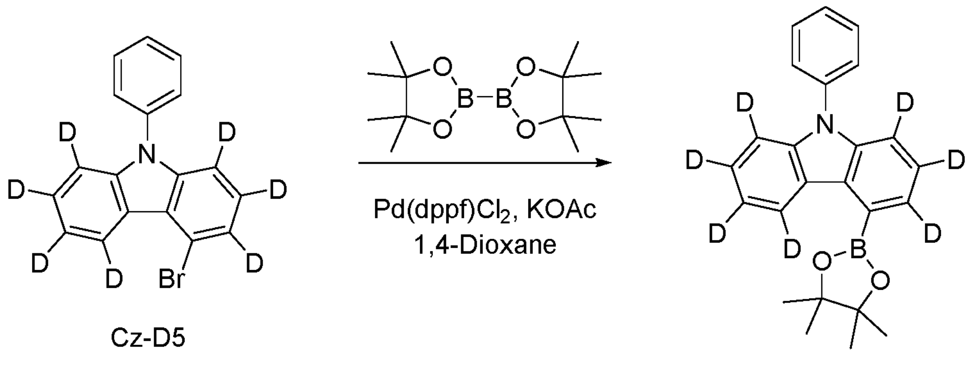


Cz-D5

Under a nitrogen atmosphere, Cz-D5 (100.0 g, 303.7 mmol), 4,4,4',4',5,5, 5',5'-octamethyl-2,2'-bi(1,3,2-dioxaborolane) (84.8 g, 334.1 mmol), Pd(dppf)$Cl_2$ (26.6 g, 30.3 mmol), KOAc (85.8 g, 911.1 mmol), and 1,4-Dioxane (1000 ml) were mixed, and stirred at 130°C for 12 hours.

After the reaction was completed, extraction was conducted with ethyl acetate, moisture was removed over $MgSO_4$, and then purification was performed by column chromatography (hexane:EA=8:1 (v/v)), thereby obtaining 9-phenyl-4-(4,4,5,5-tetramethyl-1,3,2-dioxaborolan-2-yl)-9H-carbazole-1,2,3,5,6,7,8-d7 (74.3 g, yield: 65%).
Mass (calcd.: 376.3, found: 376 g/mol)

<Step 6-1-2> Synthesis of BCz-D5

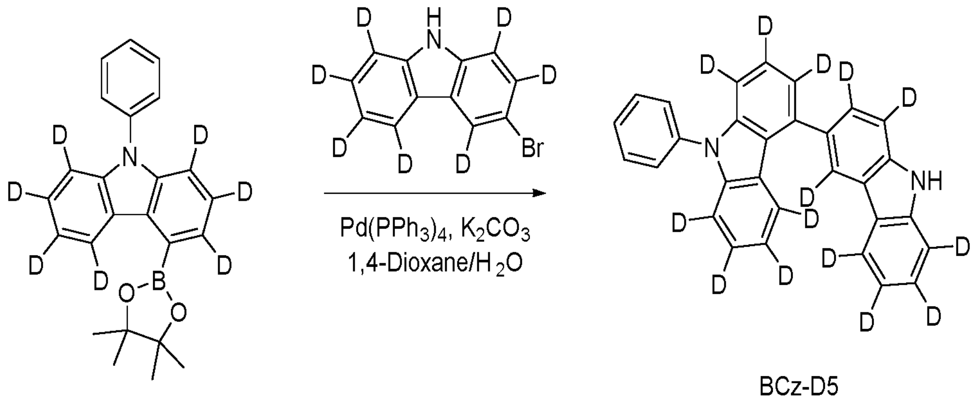


BCz-D5

Under a nitrogen atmosphere, 9-phenyl-4-(4,4,5,5-tetramethyl-1,3,2-dioxaborolan-2-yl)-9H-carbazole-1,2,3,5,6,7,8-d7 (74.3 g, 197.4 mmol) , 3-bromo-9H-carbazole-1,2,4,5,6,7,8-d7 (60.0 g, 236.9 mmol), $Pd(PPh_3)_4$ (11.4 g, 9.9 mmol) , $K_2CO_3$ (68.2 g, 493.5 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D5 (63.4 g, yield: 76%).
Mass (calcd.: 422.59, found: 422 g/mol)

**<Preparation Example 6-2> Synthesis of BCz-D6**

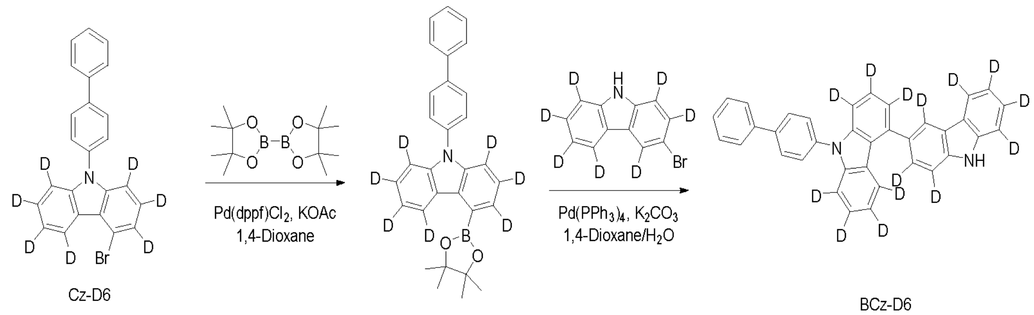


Cz-D6

BCz-D6

The same procedure as in Preparation Example 6-1 was performed except that Cz-D6 (100 g, 246.7 mmol) was used instead of Cz-D5, thereby obtaining the target compound BCz-D6 (55.4 g, yield: 45.1%).
Mass (calcd.: 498.69, found: 498 g/mol)

**<Preparation Example 6-3> Synthesis of BCz-D7**

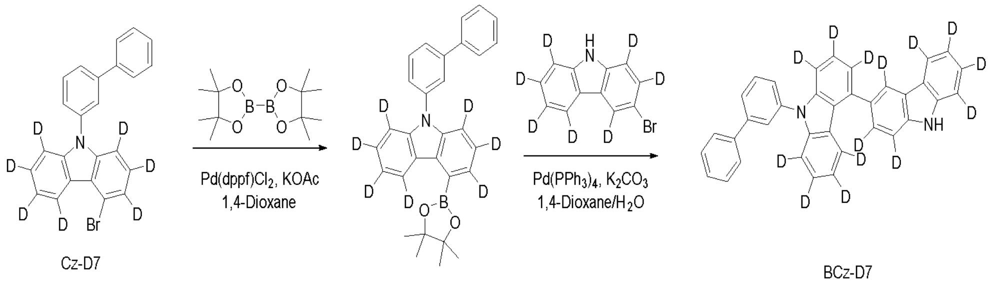


Cz-D7

BCz-D7

The same procedure as in Preparation Example 6-1 was performed except that Cz-D7 (100 g, 246.7 mmol) was used instead of Cz-D5, thereby obtaining the target compound BCz-D7 (43.1 g, yield: 35.1%).
Mass (calcd.: 498.69, found: 498 g/mol)

**<Preparation Example 6-4> Synthesis of BCz-D8**

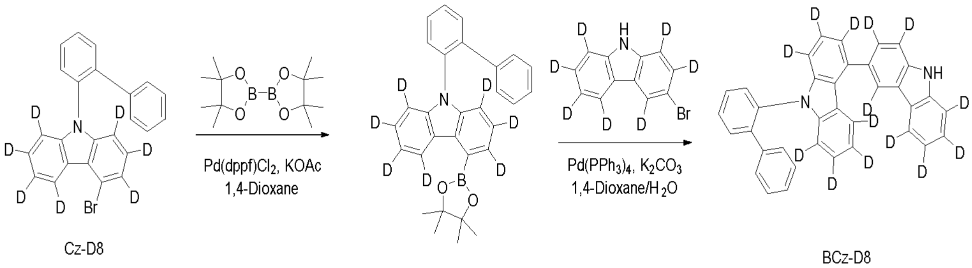


Cz-D8

BCz-D8

The same procedure as in Preparation Example 6-1 was performed except that Cz-D8 (100 g, 246.7 mmol) was used instead of Cz-D5, thereby obtaining the target compound BCz-D8 (28.2 g, yield: 22.9%).
Mass (calcd.: 498.69, found: 498 g/mol)

**<Preparation Example 7-1> Synthesis of BCz-D9**

<Step 7-1-1> Synthesis of 9-phenyl-2-(4,4,5,5-tetramethyl-1,3,2-dioxaborolan-2-yl)-9H-carbazole-1,3,4,5,6,7,8-d7

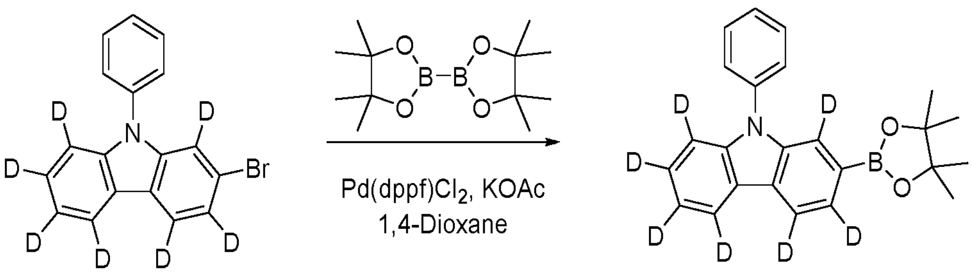


Cz-D9

Under a nitrogen atmosphere, Cz-D9 (100.0 g, 303.7 mmol), 4,4,4',4',5,5, 5',5'-octamethyl-2,2'-bi(1,3,2-dioxaborolane) (84.8 g, 334.1 mmol), Pd(dppf)$Cl_2$ (26.6 g, 30.3 mmol), KOAc (85.8 g, 911.1 mmol), and 1,4-Dioxane (1000 ml) were mixed, and stirred at 130°C for 12 hours.

After the reaction was completed, extraction was conducted with ethyl acetate, moisture was removed over $MgSO_4$, and then purification was performed by column chromatography (hexane:EA=8:1 (v/v)), thereby obtaining 9-phenyl-2-(4,4,5,5-tetramethyl-1,3,2-dioxaborolan-2-yl)-9H-carbazole-1,3,4,5,6,7,8-d7 (81.1 g, yield: 71%).
Mass (calcd.: 376.3, found: 376 g/mol)

<Step 7-1-2> Synthesis of BCz-D9

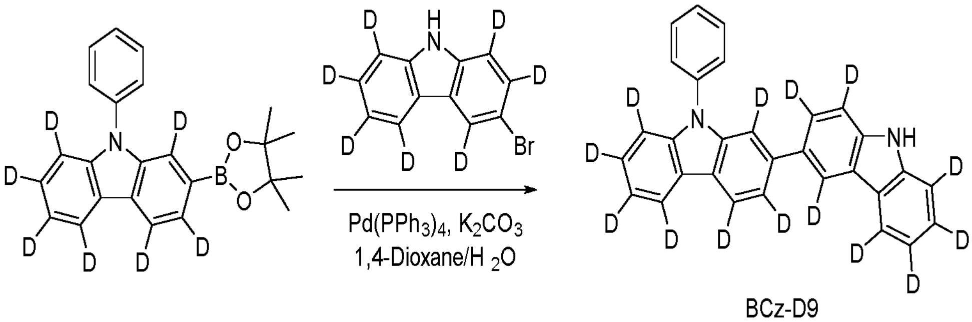


BCz-D9

Under a nitrogen atmosphere, 9-phenyl-2-(4,4,5,5-tetramethyl-1,3,2-dioxaborolan-2-yl)-9H-carbazole-1,3,4,5,6,7,8-d7 (81.1 g, 215.6 mmol), 3-bromo-9H-carbazole-1, 2, 4, 5, 6, 7, 8-d7 (65.5 g, 258.8 mmol), $Pd(PPh_3)_4$ (12.5 g, 10.8 mmol), $K_2CO_3$ (74.5 g, 539.1 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D9 (67.4 g, yield: 74%).
Mass (calcd.: 422.59, found: 422 g/mol)

**<Preparation Example 7-2> Synthesis of BCz-D10**

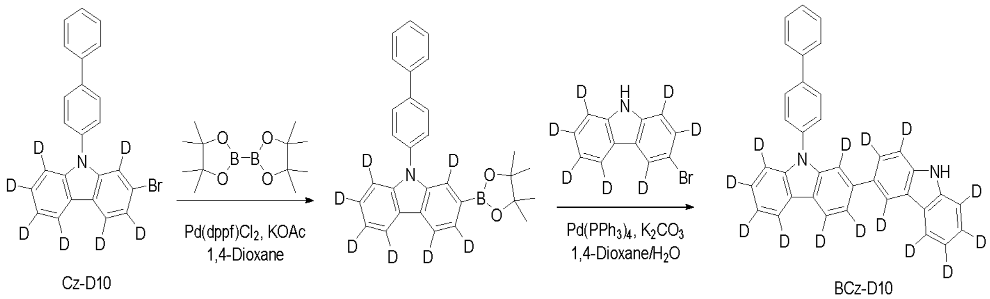


Cz-D10

BCz-D10

The same procedure as in Preparation Example 7-1 was performed except that Cz-D10 (100 g, 246.7 mmol) was used instead of Cz-D9, thereby obtaining the target compound BCz-D10 (65.2 g, yield: 53.0%).
Mass (calcd.: 498.69, found: 498 g/mol)

**<Preparation Example 7-3> Synthesis of BCz-D11**

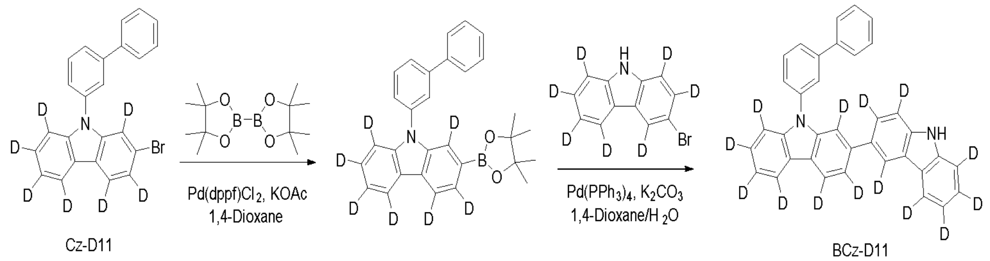


Cz-D11

BCz-D11

The same procedure as in Preparation Example 7-1 was performed except that Cz-D11 (100 g, 246.7 mmol) was

used instead of Cz-D9, thereby obtaining the target compound BCz-D11 (68.2 g, yield: 55.4%).
Mass (calcd.: 498.69, found: 498 g/mol)

**<Preparation Example 7-4> Synthesis of BCz-D12**

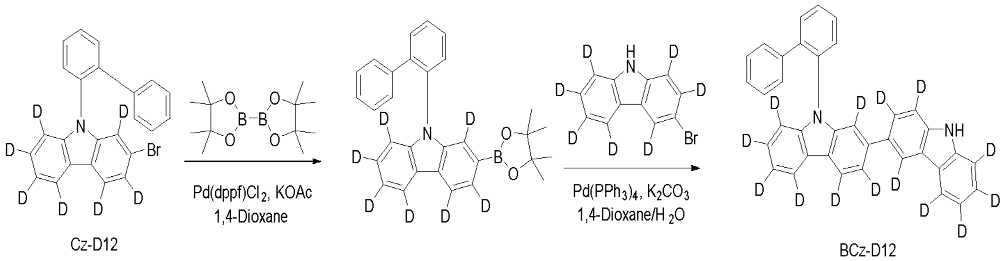


Cz-D12

BCz-D12

The same procedure as in Preparation Example 7-1 was performed except that Cz-D12 (100 g, 246.7 mmol) was used instead of Cz-D9, thereby obtaining the target compound BCz-D12 (51.1 g, yield: 41.6%).
Mass (calcd.: 498.69, found: 498 g/mol)

**<Preparation Example 8-1> Synthesis of BCz-D13**

<Step 8-1-1> Synthesis of 9-phenyl-1-(4,4,5,5-tetramethyl-1,3,2-dioxaborolan-2-yl)-9H-carbazole-2,3,4,5,6,7,8-d7

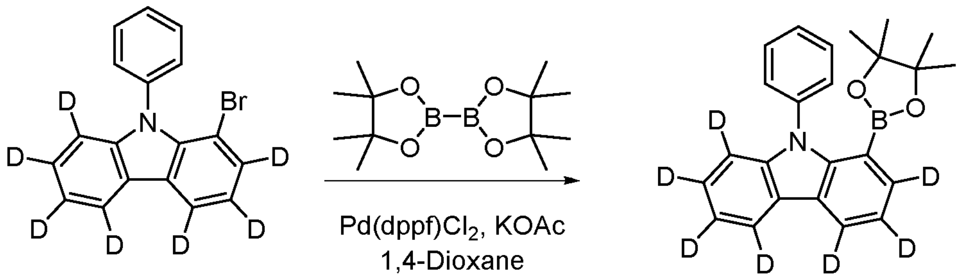


Cz-D13

Under a nitrogen atmosphere, Cz-D9 (100.0 g, 303.7 mmol), 4,4,4',4',5,5, 5',5'-octamethyl-2,2'-bi(1,3,2-dioxaborolane) (84.8 g, 334.1 mmol), Pd(dppf)$Cl_2$ (26.6 g, 30.3 mmol), KOAc (85.8 g, 911.1 mmol), and 1,4-Dioxane (1000 ml) were mixed, and stirred at 130°C for 12 hours.

After the reaction was completed, extraction was conducted with ethyl acetate, moisture was removed over $MgSO_4$, and then purification was performed by column chromatography (hexane:EA=8:1 (v/v)), thereby obtaining 9-phenyl-1-(4,4,5,5-tetramethyl-1,3,2-dioxaborolan-2-yl)-9H-carbazole-2,3,4,5,6,7,8-d7 (64.0 g, yield: 56%).
Mass (calcd.: 376.3, found: 376 g/mol)

<Step 8-1-2> Synthesis of BCz-D13

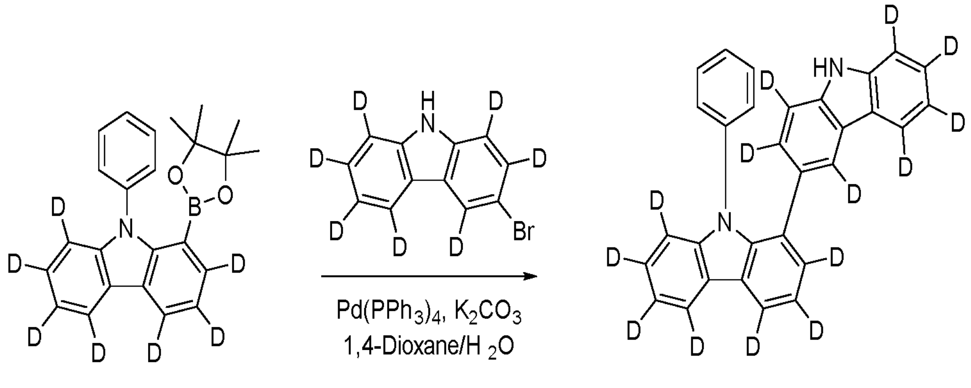


BCz-D13

Under a nitrogen atmosphere, 9-phenyl-1-(4,4,5,5-tetramethyl-1,3,2-dioxaborolan-2-yl)-9H-carbazole-2,3,4,5,6,7,8-d7 (64.0 g, 170.0 mmol) , 3-bromo-9H-carbazole-1, 2, 4, 5, 6, 7, 8-d7 (51.7 g, 204.1 mmol), $Pd(PPh_3)_4$ (9.8 g, 8.5 mmol), $K_2CO_3$ (58.8 g, 425.2 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D13 (37.3 g, yield: 52%).
Mass (calcd.: 422.59, found: 422 g/mol)

**<Preparation Example 8-2> Synthesis of BCz-D14**

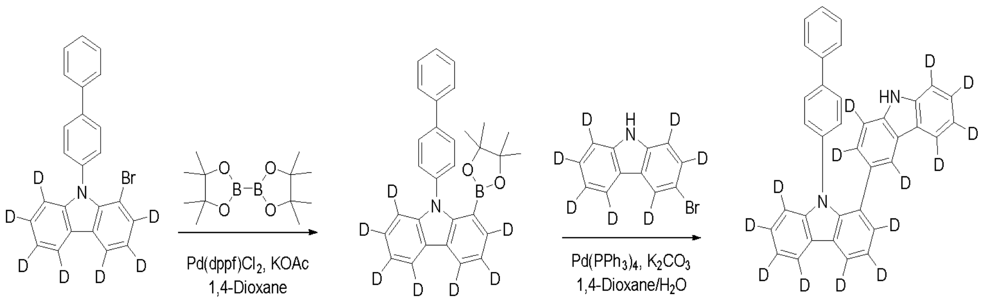


Cz-D14

BCz-D14

The same procedure as in Preparation Example 8-1 was performed except that Cz-D14 (100 g, 246.7 mmol) was used instead of Cz-D13, thereby obtaining the target compound BCz-D14 (33.2 g, yield: 27.0%).
Mass (calcd.: 498.69, found: 498 g/mol)

**<Preparation Example 8-3> Synthesis of BCz-D15**

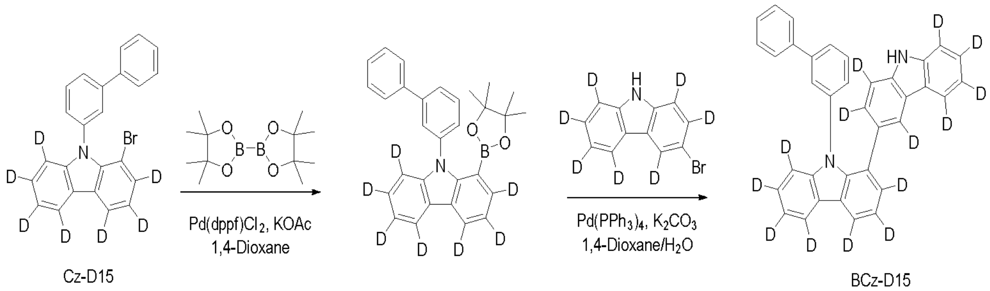


Cz-D15

BCz-D15

The same procedure as in Preparation Example 8-1 was performed except that Cz-D15 (100 g, 246.7 mmol) was

used instead of Cz-D13, thereby obtaining the target compound BCz-D15 (32.2 g, yield: 26.2%).
Mass (calcd.: 498.69, found: 498 g/mol)

<u>**<Preparation Example 8-4> Synthesis of BCz-D16**</u>

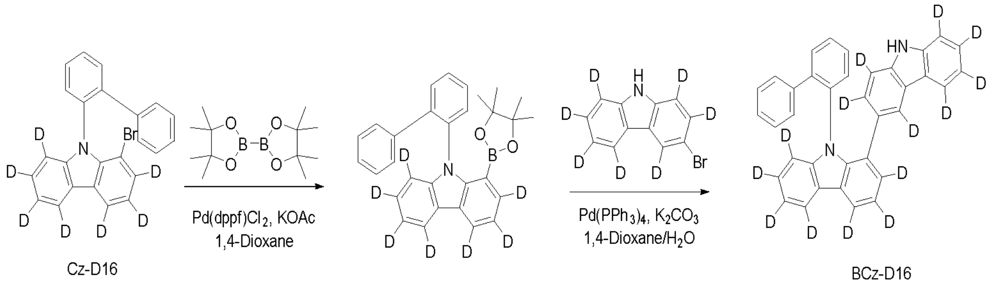

Cz-D16 BCz-D16

The same procedure as in Preparation Example 8-1 was performed except that Cz-D16 (100 g, 246.7 mmol) was used instead of Cz-D13, thereby obtaining the target compound BCz-D16 (27.0 g, yield: 21.9%).
Mass (calcd.: 498.69, found: 498 g/mol)

<u>**<Preparation Example 9-1> Synthesis of BCz-D17**</u>

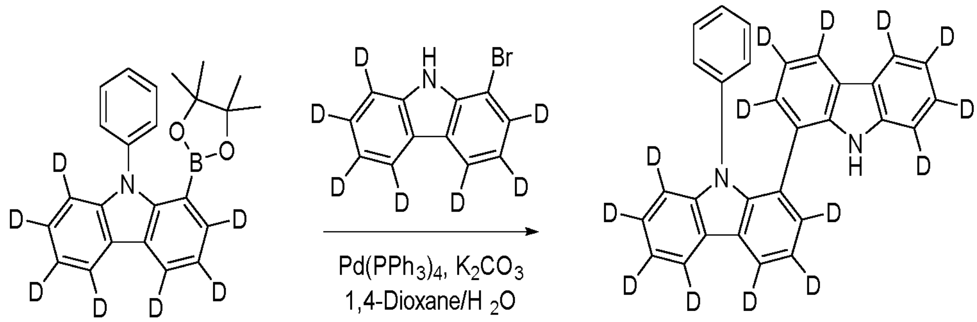

BCz-D17

Under a nitrogen atmosphere, 9-phenyl-1-(4,4,5,5-tetramethyl-1,3,2-dioxaborolan-2-yl)-9H-carbazole-2,3,4,5,6,7,8-d7 (100.0 g, 170.0 mmol), 1-bromo-9H-carbazole-2,3,4,5,6,7,8-d7 (80.7 g, 318.8 mmol), Pd($PPh_3$)$_4$ (15.3 g, 13.2 mmol), $K_2CO_3$ (91.8 g, 664.2 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D17 (50.5 g, yield: 45%).
Mass (calcd.: 422.59, found: 422 g/mol)

<u>**<Preparation Example 9-2> Synthesis of BCz-D18**</u>

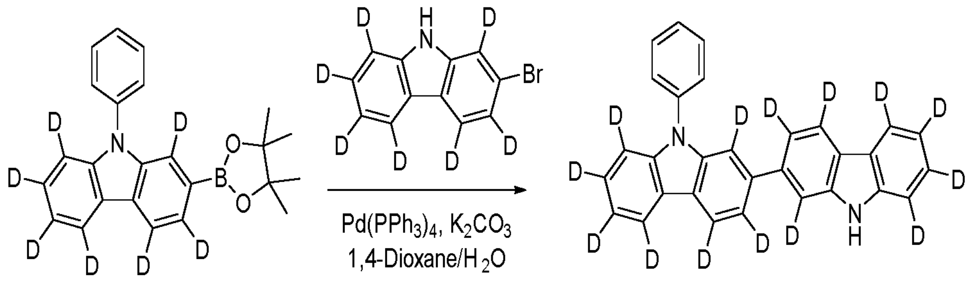


BCz-D18

Under a nitrogen atmosphere, 9-phenyl-2-(4,4,5,5-tetramethyl-1,3,2-dioxaborolan-2-yl)-9H-carbazole-1,3,4,5,6,7,8-d7 (100.0 g, 170.0 mmol), 2-bromo-9H-carbazole-1,3,4,5,6,7,8-d7 (80.7 g, 318.8 mmol), $Pd(PPh_3)_4$ (15.3 g, 13.2 mmol), $K_2CO_3$ (91.8 g, 664.2 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D18 (70.7 g, yield: 63%).
Mass (calcd.: 422.59, found: 422 g/mol)

**<Preparation Example 9-3> Synthesis of BCz-D19**

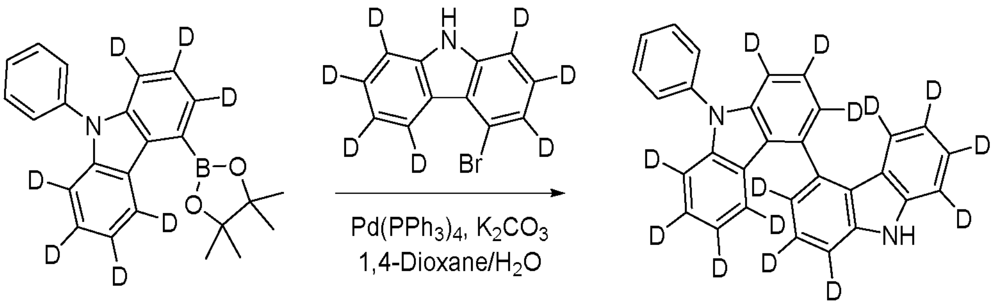


BCz-D19

Under a nitrogen atmosphere, 9-phenyl-4-(4,4,5,5-tetramethyl-1,3,2-dioxaborolan-2-yl)-9H-carbazole-1,2,3,5,6,7,8-d7 (100.0 g, 170.0 mmol), 4-bromo-9H-carbazole-1, 2, 3, 5, 6, 7, 8-d7 (80.7 g, 318.8 mmol), $Pd(PPh_3)_4$ (15.3 g, 13.2 mmol), $K_2CO_3$ (91.8 g, 664.2 mmol), and 1,4-dioxane/$H_2O$ (1000 ml/250 ml) were mixed, and stirred at 120°C for 4 hours.

After the reaction was completed, extraction was conducted with methylene chloride, and filtration was conducted with $MgSO_4$. The obtained organic layer was subjected to solvent removal, and then purification was performed by column chromatography (hexane:EA = 7:1 (v/v)), thereby obtaining BCz-D19 (59.5 g, yield: 53%).
Mass (calcd.: 422.59, found: 422 g/mol)

**[Synthesis Example 1] Synthesis of A-1**

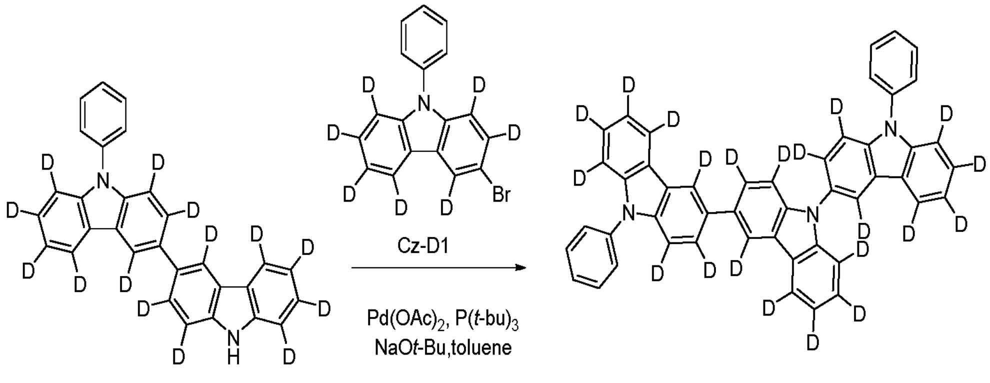


BCz-D1 A-1

Under a nitrogen atmosphere, BCz-D1 (10.0 g, 23.6 mmol), Cz-D1 (9.3 g, 28.3 mmol), $Pd(OAc)_2$ (1.36 g, 1.18 mmol), P(*t*-Bu)$_3$ (0.57 ml, 2.36 mmol), NaO(t-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound A-1 (13.0 g, yield: 82%).
Mass (calcd.: 670.93, found: 670 g/mol)

**[Synthesis Example 2] Synthesis of A-2**

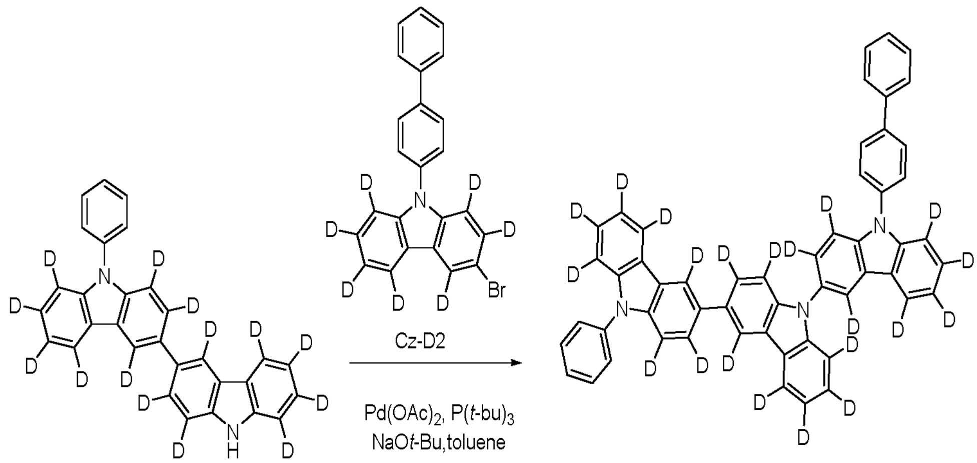


BCz-D1 A-2

The same procedure as in Synthesis Example 1 was performed except that Cz-D2 (10.0 g, 23.6 mmol) was used instead of Cz-D1, thereby obtaining the target compound A-2 (13.8 g, yield: 78%).
Mass (calcd.: 747.02, found: 747 g/mol)

**[Synthesis Example 3] Synthesis of A-3**

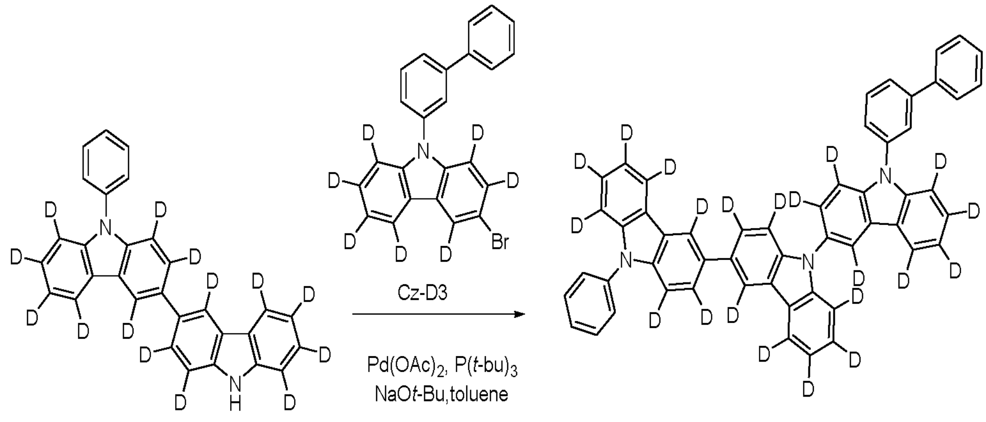


BCz-D1 A-3

The same procedure as in Synthesis Example 1 was performed except that Cz-D3 (10.0 g, 23.6 mmol) was used instead of Cz-D1, thereby obtaining the target compound A-3 (13.2 g, yield: 75%).
Mass (calcd.: 747.02, found: 747 g/mol)

## [Synthesis Example 4] Synthesis of A-4

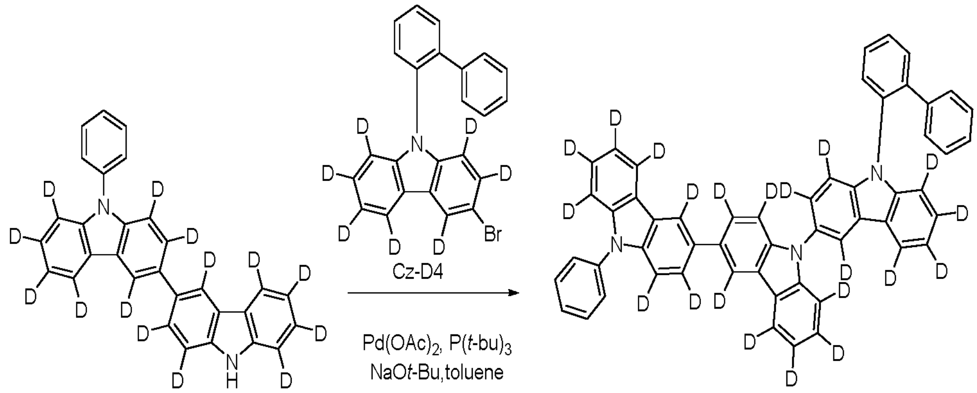


BCz-D1 A-4

The same procedure as in Synthesis Example 1 was performed except that Cz-D4 (10.0 g, 23.6 mmol) was used instead of Cz-D1, thereby obtaining the target compound A-4 (12.2 g, yield: 69%).
Mass (calcd.: 747.02, found: 747 g/mol)

## [Synthesis Example 5] Synthesis of A-5

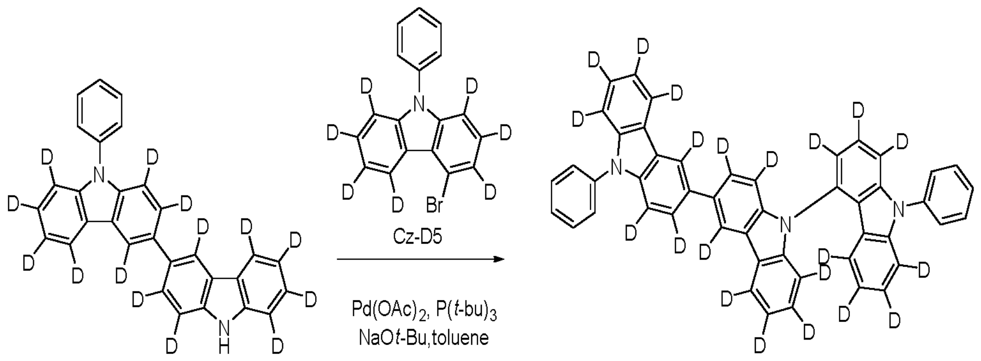


BCz-D1 A-5

The same procedure as in Synthesis Example 1 was performed except that Cz-D5 (9.3 g, 23.6 mmol) was used instead of Cz-D1, thereby obtaining the target compound A-5 (8.73 g, yield: 55%).
Mass (calcd.: 670.93, found: 670 g/mol)

**[Synthesis Example 6] Synthesis of A-6**

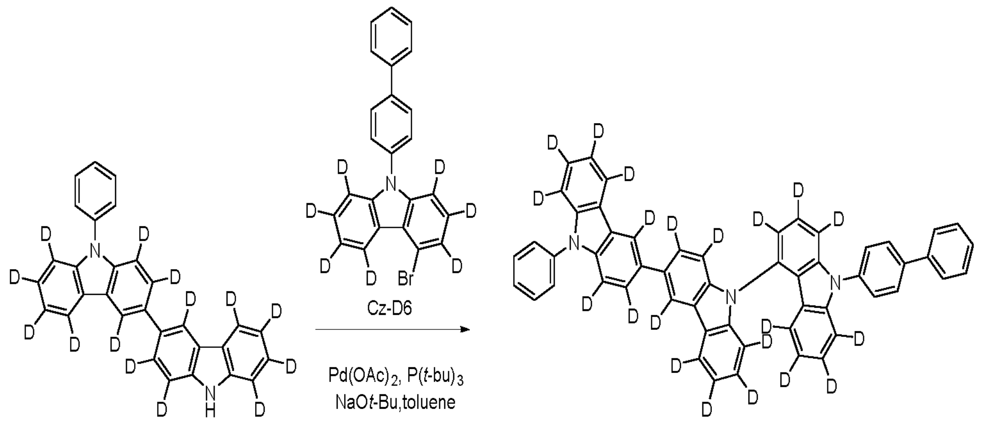


BCz-D1 A-6

The same procedure as in Synthesis Example 1 was performed except that Cz-D6 (10.0 g, 23.6 mmol) was used instead of Cz-D1, thereby obtaining the target compound A-6 (7.42 g, yield: 42%) .
Mass (calcd.: 747.02, found: 747 g/mol)

**[Synthesis Example 7] Synthesis of A-7**

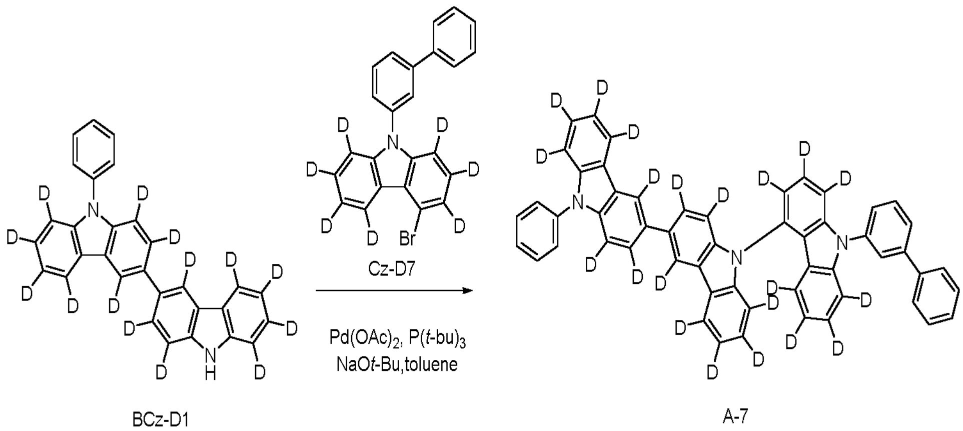


The same procedure as in Synthesis Example 1 was performed except that Cz-D7 (10.0 g, 23.6 mmol) was used instead of Cz-D1, thereby obtaining the target compound A-7 (8.83 g, yield: 50%).
Mass (calcd.: 747.02, found: 747 g/mol)

**[Synthesis Example 8] Synthesis of A-8**

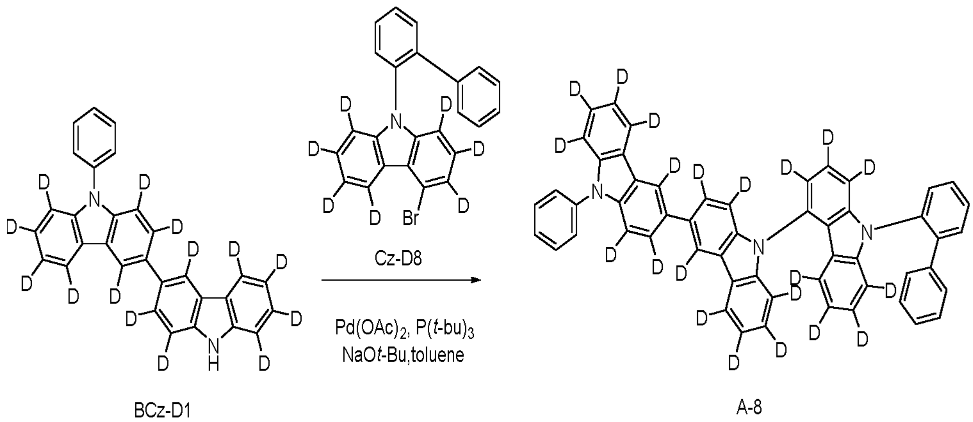


The same procedure as in Synthesis Example 1 was performed except that Cz-D8 (10.0 g, 23.6 mmol) was used instead of Cz-D1, thereby obtaining the target compound A-8 (9.89 g, yield: 56%).
Mass (calcd.: 747.02, found: 747 g/mol)

**[Synthesis Example 9] Synthesis of A-9**

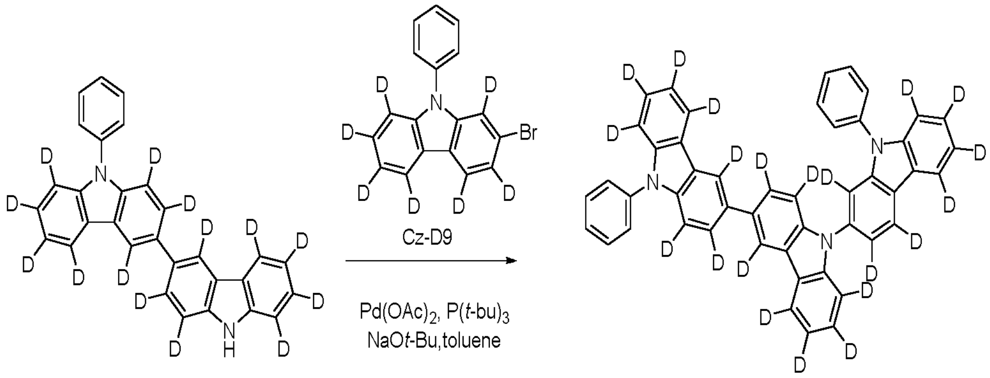


BCz-D1 A-9

The same procedure as in Synthesis Example 1 was performed except that Cz-D9 (9.3 g, 23.6 mmol) was used instead of Cz-D1, thereby obtaining the target compound A-9 (8.41 g, yield: 53%).
Mass (calcd.: 670.93, found: 670 g/mol)

**[Synthesis Example 10] Synthesis of A-10**

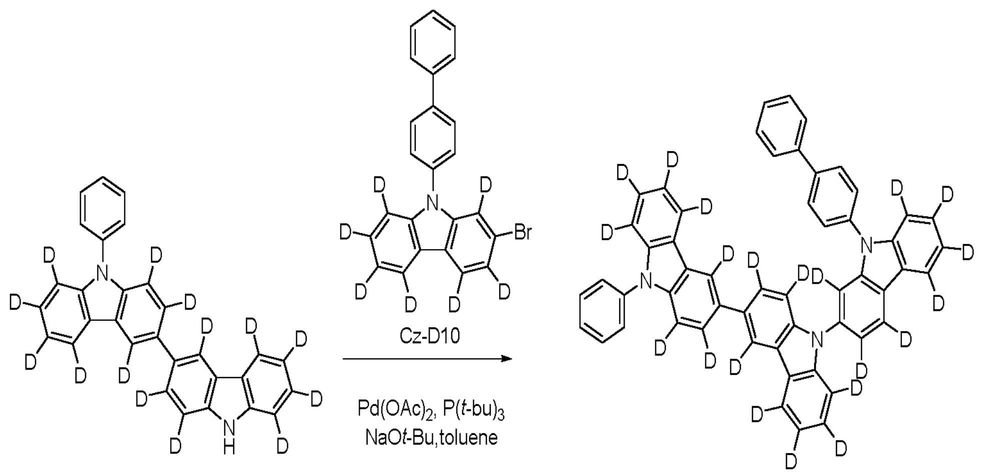


BCz-D1 A-10

The same procedure as in Synthesis Example 1 was performed except that Cz-D10 (10.0 g, 23.6 mmol) was used instead of Cz-D1, thereby obtaining the target compound A-10 (8.66 g, yield: 49%).
Mass (calcd.: 747.02, found: 747 g/mol)

**[Synthesis Example 11] Synthesis of A-11**

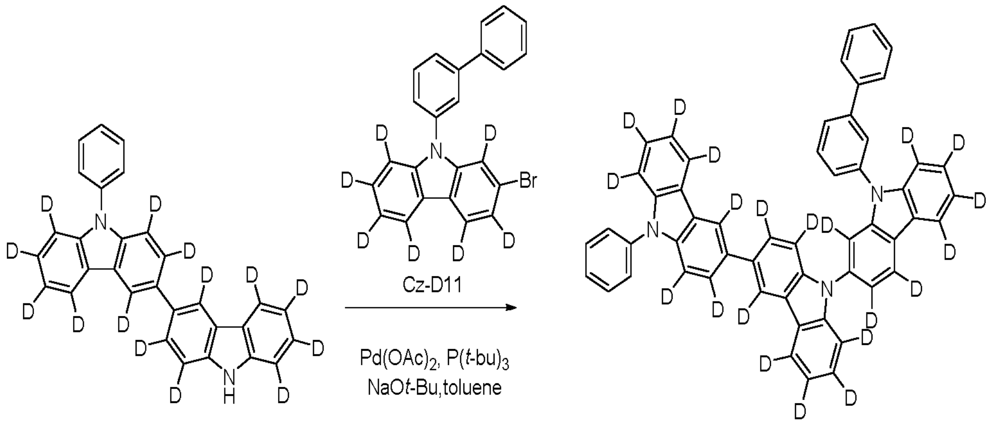


BCz-D1 A-11

The same procedure as in Synthesis Example 1 was performed except that Cz-D11 (10.0 g, 23.6 mmol) was used instead of Cz-D1, thereby obtaining the target compound A-11 (9.01 g, yield: 51%).
Mass (calcd.: 747.02, found: 747 g/mol)

## [Synthesis Example 12] Synthesis of A-12

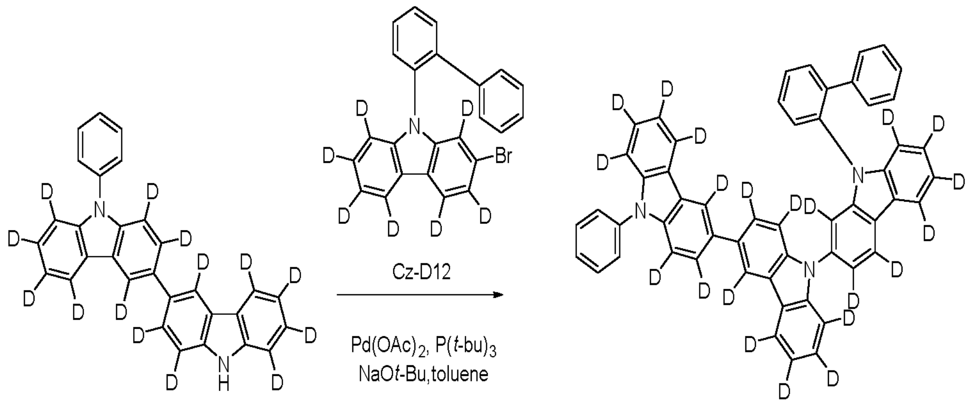


BCz-D1 A-12

The same procedure as in Synthesis Example 1 was performed except that Cz-D12 (10.0 g, 23.6 mmol) was used instead of Cz-D1, thereby obtaining the target compound A-12 (9.19 g, yield: 52%).
Mass (calcd.: 747.02, found: 747 g/mol)

## [Synthesis Example 13] Synthesis of A-13

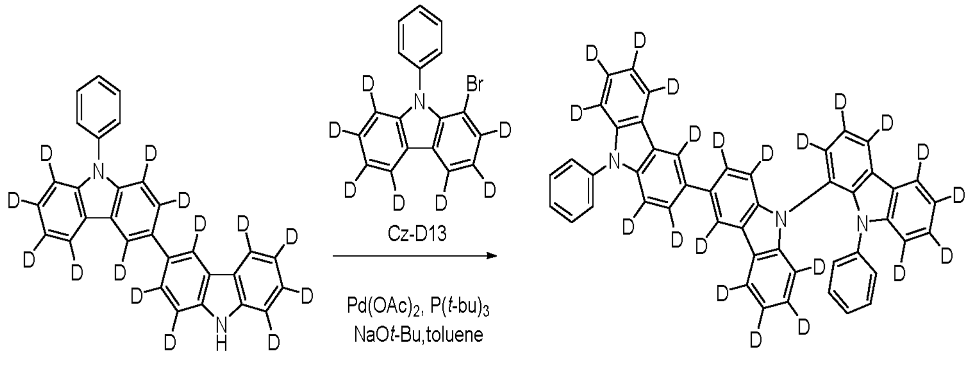


BCz-D1 A-13

The same procedure as in Synthesis Example 1 was performed except that Cz-D13 (9.3 g, 23.6 mmol) was used instead of Cz-D1, thereby obtaining the target compound A-13 (9.52 g, yield: 60%).
Mass (calcd.: 670.93, found: 670 g/mol)

**[Synthesis Example 14] Synthesis of A-14**

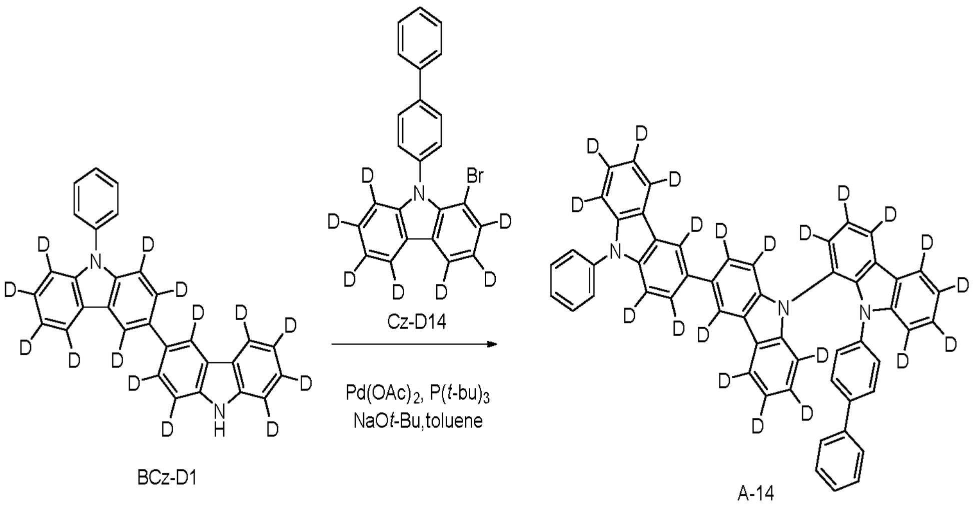


BCz-D1 A-14

The same procedure as in Synthesis Example 1 was performed except that Cz-D14 (10.0 g, 23.6 mmol) was used instead of Cz-D1, thereby obtaining the target compound A-14 (10.78 g, yield: 61%).
Mass (calcd.: 747.02, found: 747 g/mol)

**[Synthesis Example 15] Synthesis of A-15**

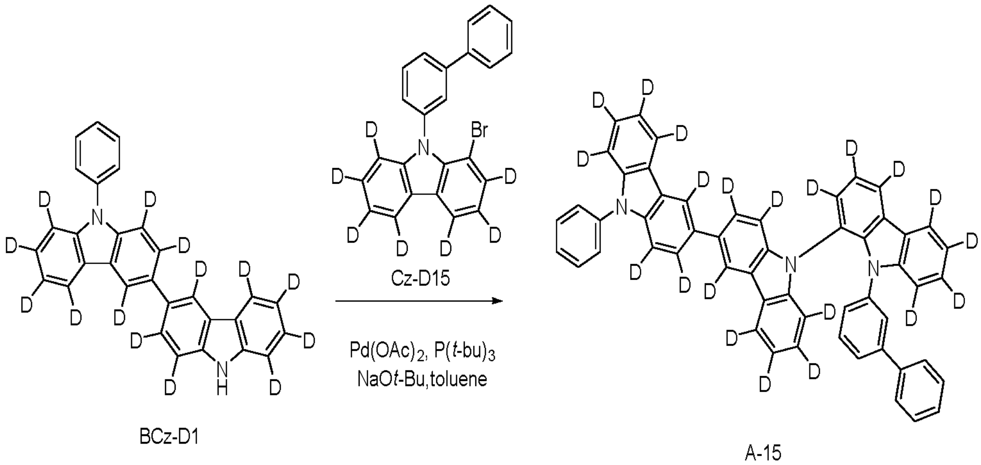


BCz-D1 A-15

The same procedure as in Synthesis Example 1 was performed except that Cz-D15 (10.0 g, 23.6 mmol) was used instead of Cz-D1, thereby obtaining the target compound A-15 (11.13 g, yield: 63%).
Mass (calcd.: 747.02, found: 747 g/mol)

**[Synthesis Example 16] Synthesis of A-16**

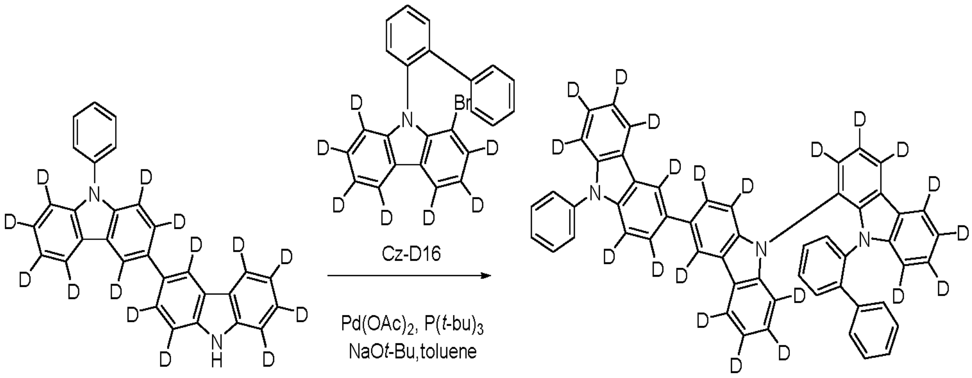


BCz-D1 A-16

The same procedure as in Synthesis Example 1 was performed except that Cz-D16 (10.0 g, 23.6 mmol) was used instead of Cz-D1, thereby obtaining the target compound A-16 (9.02 g, yield: 51%).
Mass (calcd.: 747.02, found: 747 g/mol)

**[Synthesis Example 17] Synthesis of B-1**

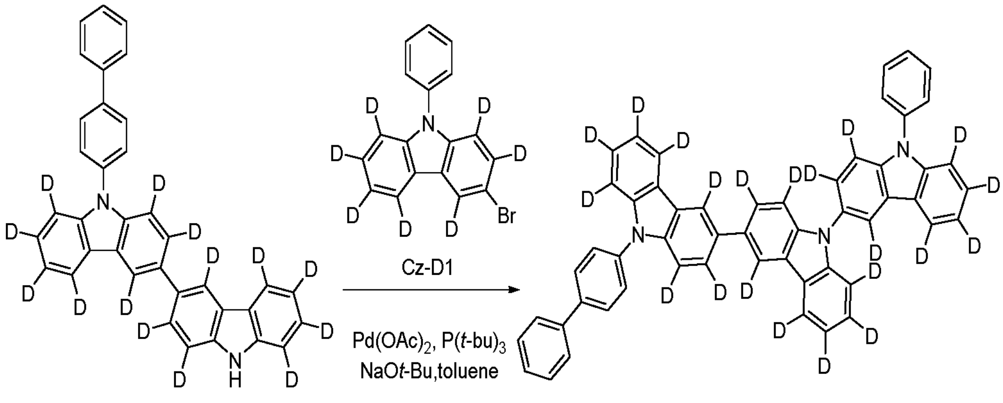


BCz-D2 B-1

Under a nitrogen atmosphere, BCz-D2 (10.0 g, 20.1 mmol), Cz-D1 (7.9 g, 24.1 mmol), Pd(OAc)$_2$ (1.15 g, 1.0 mmol), P($t$-Bu)$_3$ (0.49 ml, 2.0 mmol), NaO($t$-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound B-1 (10.2 g, yield: 62%).
Mass (calcd.: 747.02, found: 747 g/mol)

**[Synthesis Example 18] Synthesis of B-2**

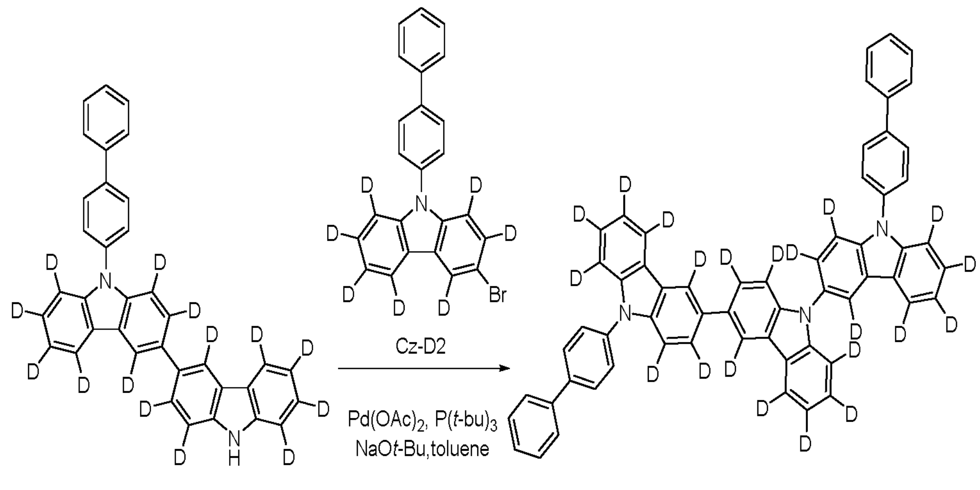


BCz-D2 B-2

The same procedure as in Synthesis Example 5 was performed except that Cz-D2 (9.75 g, 24.1 mmol) was used instead of Cz-D1, thereby obtaining the target compound B-2 (11.9 g, yield: 72%).
Mass (calcd.: 823.12, found: 823 g/mol)

**[Synthesis Example 19] Synthesis of B-3**

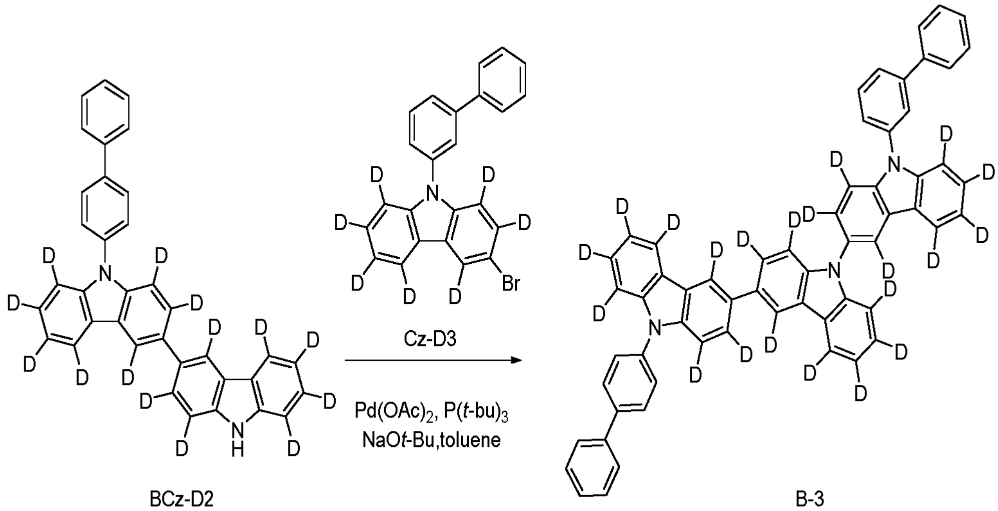


BCz-D2 B-3

The same procedure as in Synthesis Example 5 was performed except that Cz-D3 (9.75 g, 24.1 mmol) was used instead of Cz-D1, thereby obtaining the target compound B-3 (10.9 g, yield: 66%).
Mass (calcd.: 823.12, found: 823 g/mol)

**[Synthesis Example 20] Synthesis of B-4**

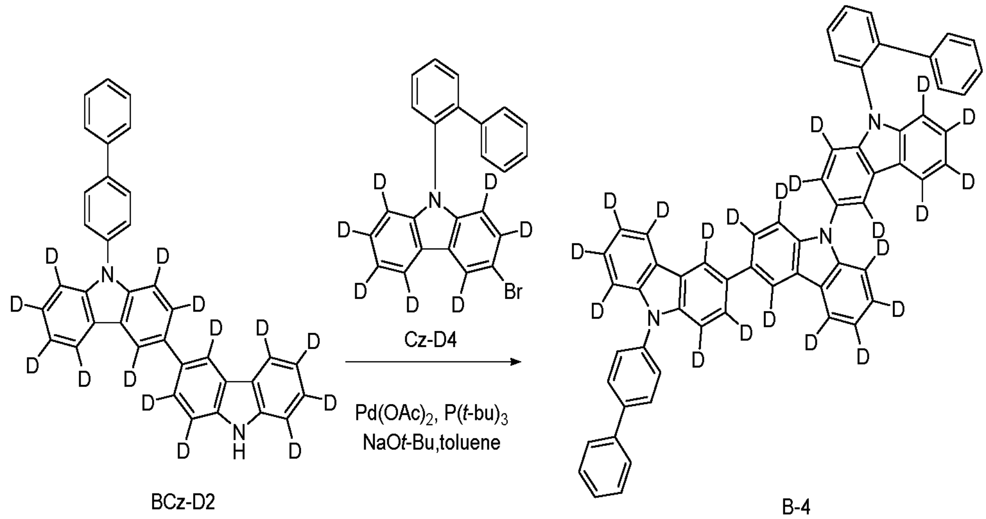


BCz-D2 B-4

The same procedure as in Synthesis Example 5 was performed except that Cz-D4 (9.75 g, 24.1 mmol) was used instead of Cz-D1, thereby obtaining the target compound B-4 (9.6 g, yield: 58%).
Mass (calcd.: 823.12, found: 823 g/mol)

**[Synthesis Example 21] Synthesis of B-5**

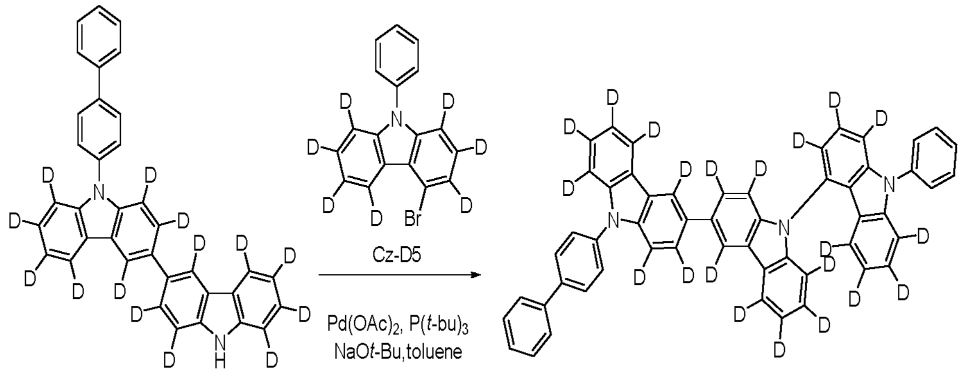


BCz-D2 B-5

The same procedure as in Synthesis Example 17 was performed except that Cz-D5 (7.9 g, 24.1 mmol) was used instead of Cz-D1, thereby obtaining the target compound B-5 (8.5 g, yield: 48%).
Mass (calcd.: 747.02, found: 747 g/mol)

**[Synthesis Example 22] Synthesis of B-6**

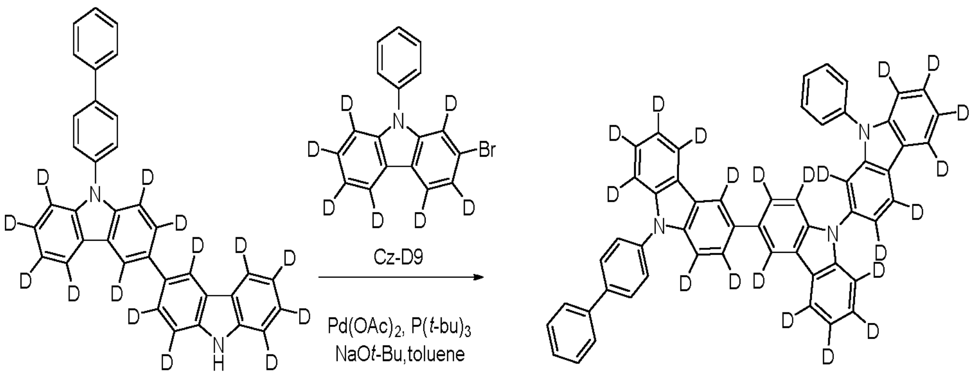


BCz-D2 B-6

The same procedure as in Synthesis Example 17 was performed except that Cz-D9 (7.9 g, 24.1 mmol) was used instead of Cz-D1, thereby obtaining the target compound B-6 (11.1 g, yield: 63%).
Mass (calcd.: 747.02, found: 747 g/mol)

**[Synthesis Example 23] Synthesis of B-7**

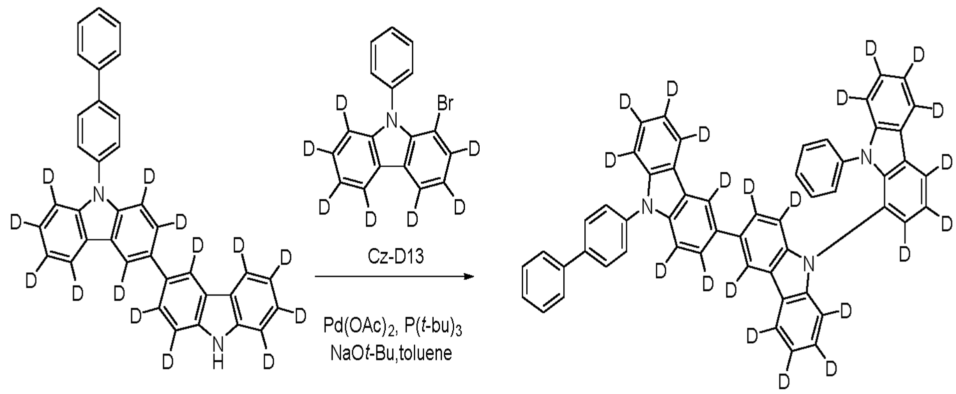


BCz-D2 B-7

The same procedure as in Synthesis Example 17 was performed except that Cz-D13 (7.9 g, 24.1 mmol) was used instead of Cz-D1, thereby obtaining the target compound B-7 (7.42 g, yield: 42%).
Mass (calcd.: 747.02, found: 747 g/mol)

**[Synthesis Example 24] Synthesis of C-1**

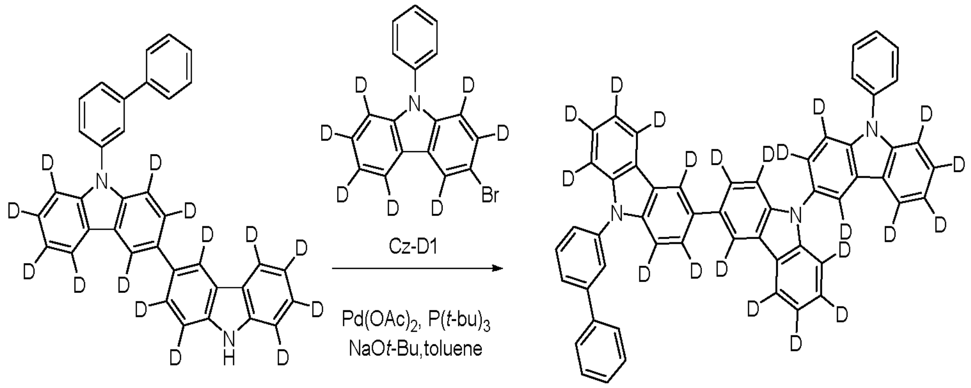


BCz-D3 C-1

Under a nitrogen atmosphere, BCz-D3 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound C-1 (9.4 g, yield: 63%).
Mass (calcd.: 747.02, found: 747 g/mol)

**[Synthesis Example 25] Synthesis of C-2**

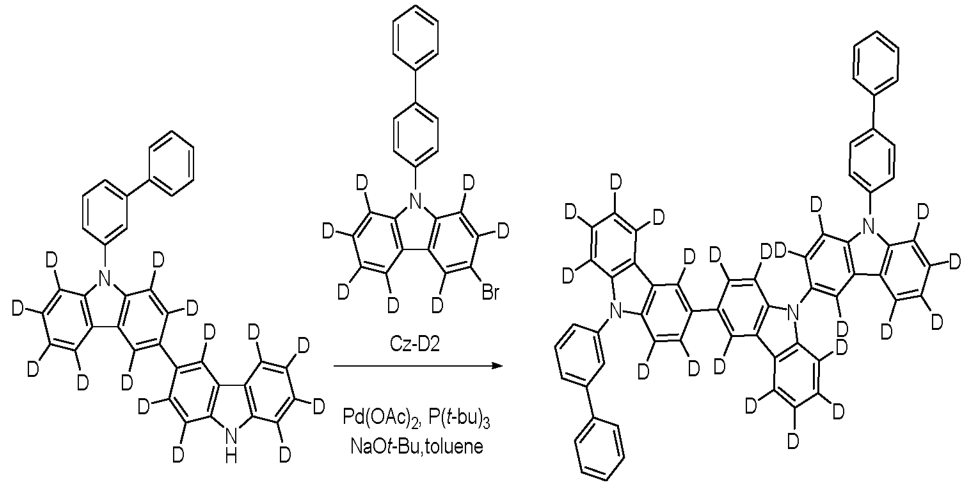


BCz-D3 C-2

The same procedure as in Synthesis Example 24 was performed except that Cz-D2 (9.75 g, 24.1 mmol) was used instead of Cz-D1, thereby obtaining the target compound C-2 (12.2 g, yield: 74%).
Mass (calcd.: 823.12, found: 823 g/mol)

**[Synthesis Example 26] Synthesis of C-3**

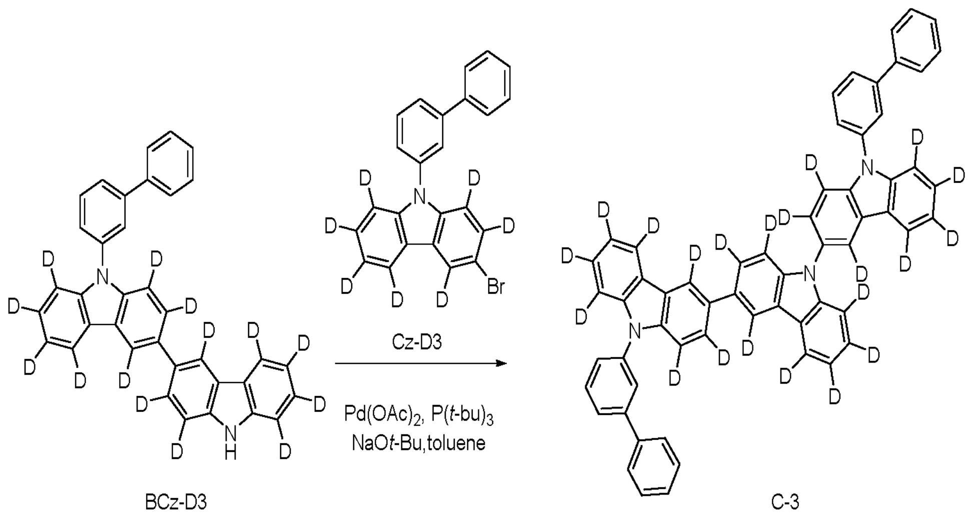


BCz-D3 C-3

The same procedure as in Synthesis Example 24 was performed except that Cz-D3 (9.75 g, 24.1 mmol) was used instead of Cz-D1, thereby obtaining the target compound C-3 (12.9 g, yield: 78%).
Mass (calcd.: 823.12, found: 823 g/mol)

**[Synthesis Example 27] Synthesis of C-4**

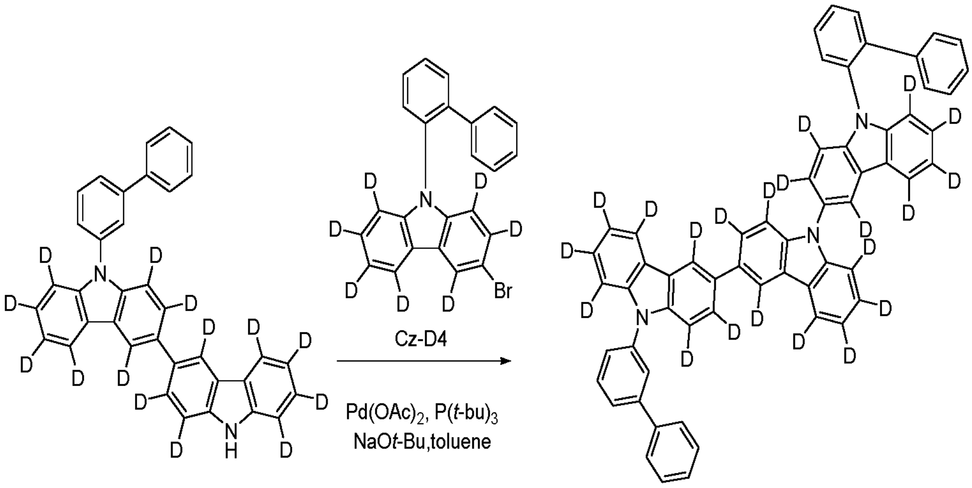


BCz-D3 C-4

The same procedure as in Synthesis Example 24 was performed except that Cz-D4 (9.75 g, 24.1 mmol) was used instead of Cz-D1, thereby obtaining the target compound C-4 (10.1 g, yield: 61%).
Mass (calcd.: 823.12, found: 823 g/mol)

**[Synthesis Example 28] Synthesis of C-5**

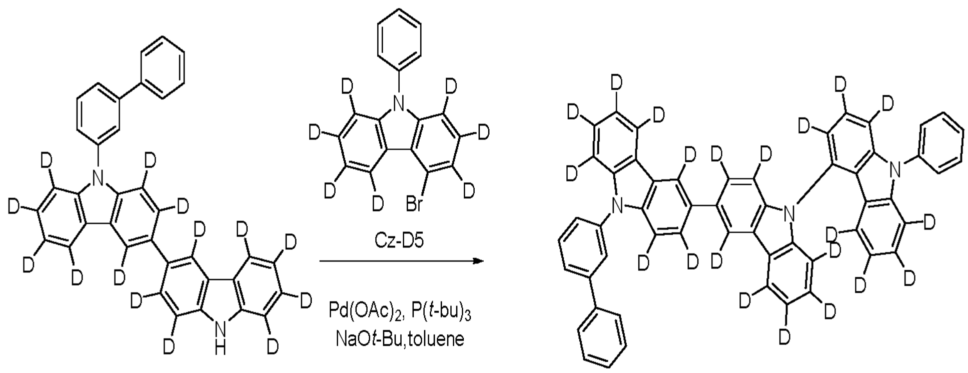


BCz-D3 C-5

The same procedure as in Synthesis Example 24 was performed except that Cz-D5 (7.9 g, 24.1 mmol) was used instead of Cz-D1, thereby obtaining the target compound C-5 (7.78 g, yield: 44%).
Mass (calcd.: 747.02, found: 747 g/mol)

**[Synthesis Example 29] Synthesis of C-6**

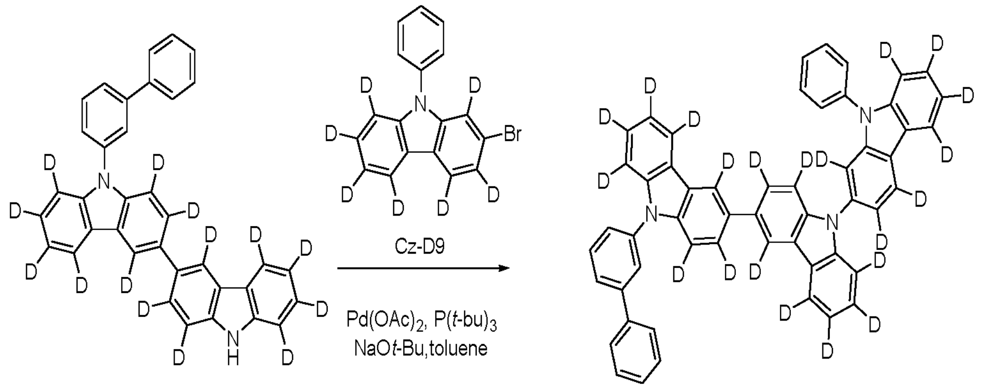


BCz-D3 C-6

The same procedure as in Synthesis Example 24 was performed except that Cz-D9 (7.9 g, 24.1 mmol) was used instead of Cz-D1, thereby obtaining the target compound C-6 (11.67 g, yield: 66%).
Mass (calcd.: 747.02, found: 747 g/mol)

**[Synthesis Example 30] Synthesis of C-7**

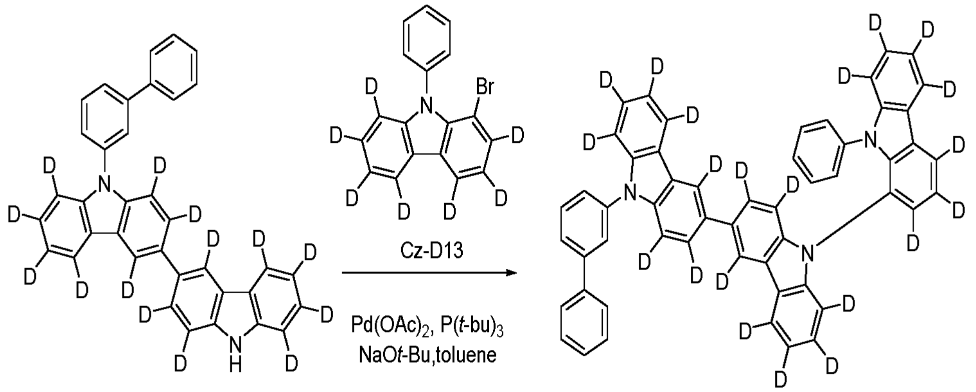


BCz-D3 C-7

The same procedure as in Synthesis Example 24 was performed except that Cz-D13 (7.9 g, 24.1 mmol) was used instead of Cz-D1, thereby obtaining the target compound C-7 (6.89 g, yield: 39%).
Mass (calcd.: 747.02, found: 747 g/mol)

**[Synthesis Example 31] Synthesis of D-1**

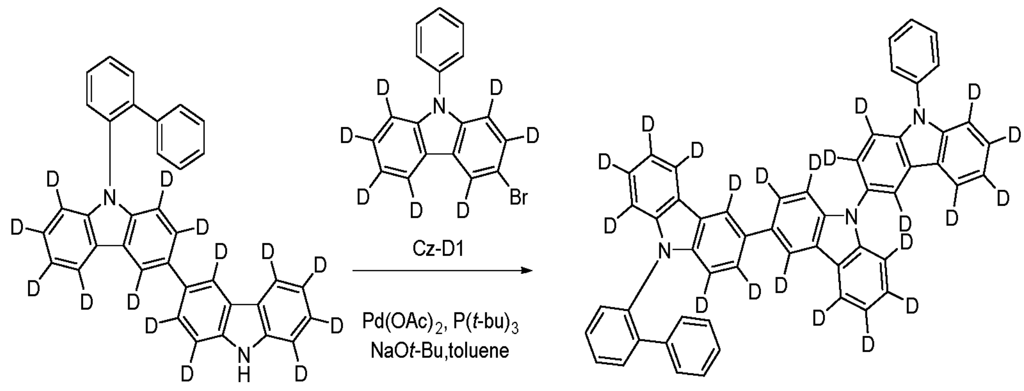


BCz-D4 D-1

Under a nitrogen atmosphere, BCz-D4 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound D-1 (8.1 g, yield: 54%).
Mass (calcd.: 747.02, found: 747 g/mol)

**[Synthesis Example 32] Synthesis of D-2**

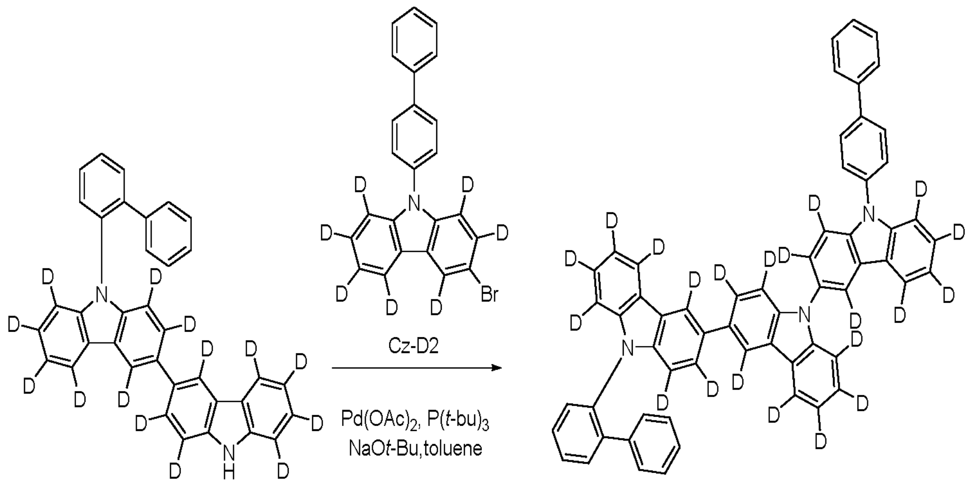


BCz-D4 D-2

The same procedure as in Synthesis Example 31 was performed except that Cz-D2 (9.75 g, 24.1 mmol) was used instead of Cz-D1, thereby obtaining the target compound D-2 (10.9 g, yield: 66%).
Mass (calcd.: 823.12, found: 823 g/mol)

**[Synthesis Example 33] Synthesis of D-3**

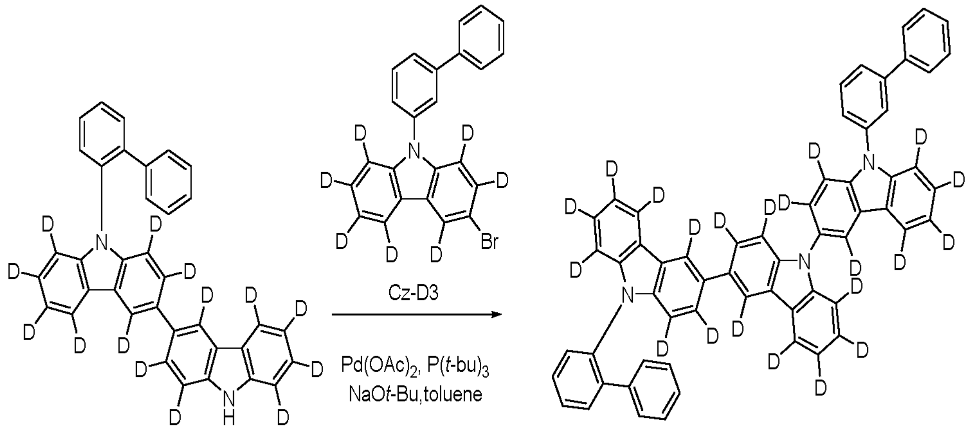


BCz-D4 D-3

The same procedure as in Synthesis Example 31 was performed except that Cz-D3 (9.75 g, 24.1 mmol) was used instead of Cz-D1, thereby obtaining the target compound D-3 (9.1 g, yield: 55%).
Mass (calcd.: 823.12, found: 823 g/mol)

**[Synthesis Example 34] Synthesis of D-4**

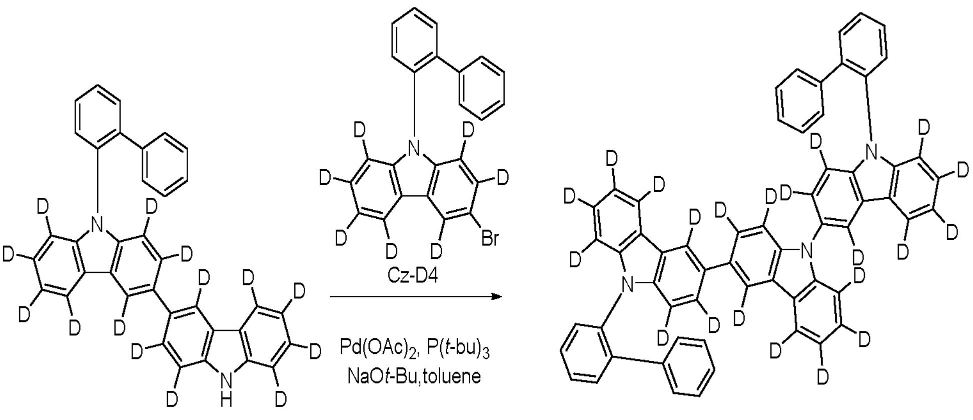


BCz-D4 D-4

The same procedure as in Synthesis Example 31 was performed except that Cz-D4 (9.75 g, 24.1 mmol) was used instead of Cz-D1, thereby obtaining the target compound D-4 (7.1 g, yield: 43%).
Mass (calcd.: 823.12, found: 823 g/mol)

**[Synthesis Example 35] Synthesis of D-5**

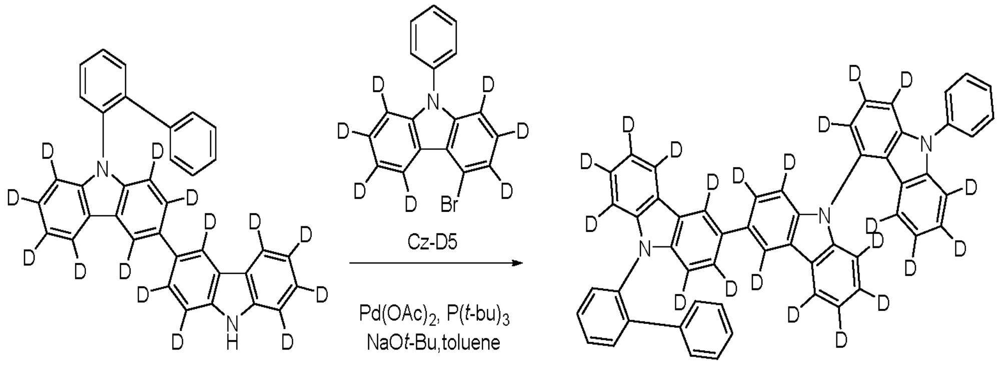


BCz-D4 D-5

The same procedure as in Synthesis Example 31 was performed except that Cz-D5 (7.9 g, 24.1 mmol) was used instead of Cz-D1, thereby obtaining the target compound D-5 (7.24 g, yield: 41%).
Mass (calcd.: 747.02, found: 747 g/mol)

**[Synthesis Example 36] Synthesis of D-6**

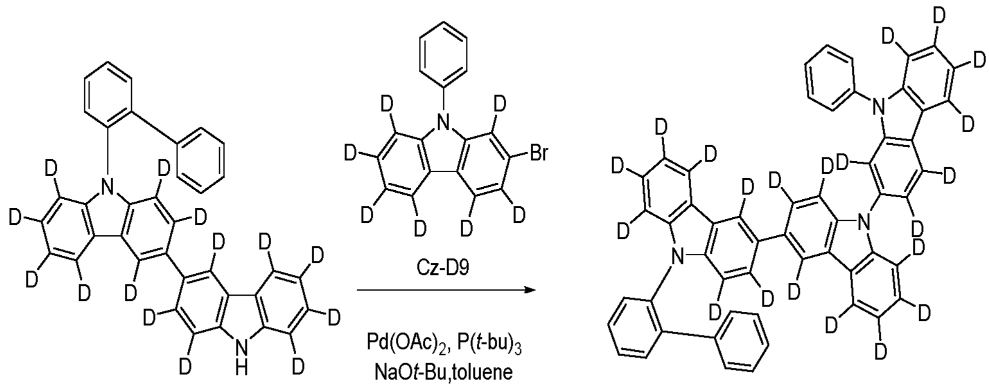


BCz-D4 D-6

The same procedure as in Synthesis Example 31 was performed except that Cz-D9 (7.9 g, 24.1 mmol) was used instead of Cz-D1, thereby obtaining the target compound D-6 (8.41 g, yield: 51%).
Mass (calcd.: 747.02, found: 747 g/mol)

**[Synthesis Example 37] Synthesis of D-7**

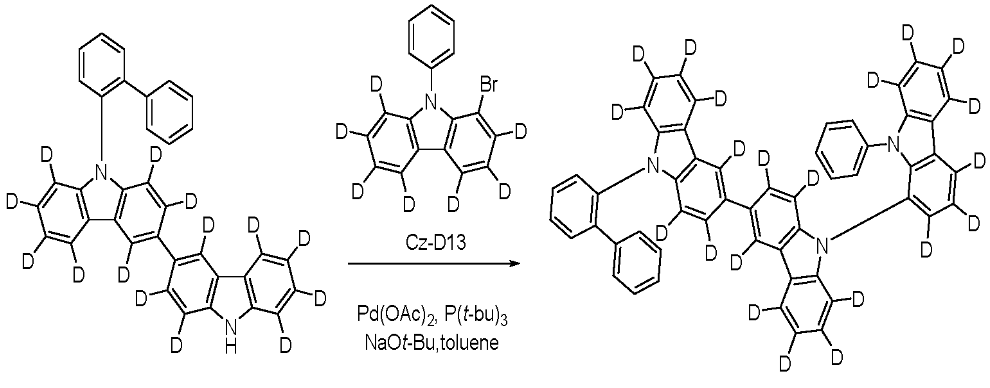


BCz-D4 D-7

The same procedure as in Synthesis Example 31 was performed except that Cz-D13 (7.9 g, 24.1 mmol) was used instead of Cz-D1, thereby obtaining the target compound D-7 (5.47 g, yield: 31%) .
Mass (calcd.: 747.02, found: 747 g/mol)

## [Synthesis Example 38] Synthesis of E-1

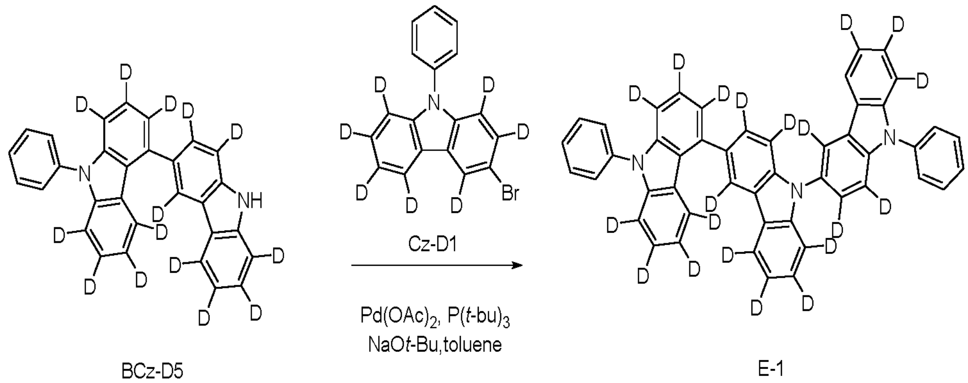


BCz-D5 E-1

Under a nitrogen atmosphere, BCz-D5 (10.0 g, 23.6 mmol), Cz-D1 (9.3 g, 28.3 mmol), $Pd(OAc)_2$ (1.36 g, 1.18 mmol), $P(t\text{-}Bu)_3$ (0.57 ml, 2.36 mmol), NaO(*t*-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound E-1 (11.43 g, yield: 72%).
Mass (calcd.: 670.93, found: 670 g/mol)

## [Synthesis Example 39] Synthesis of E-2

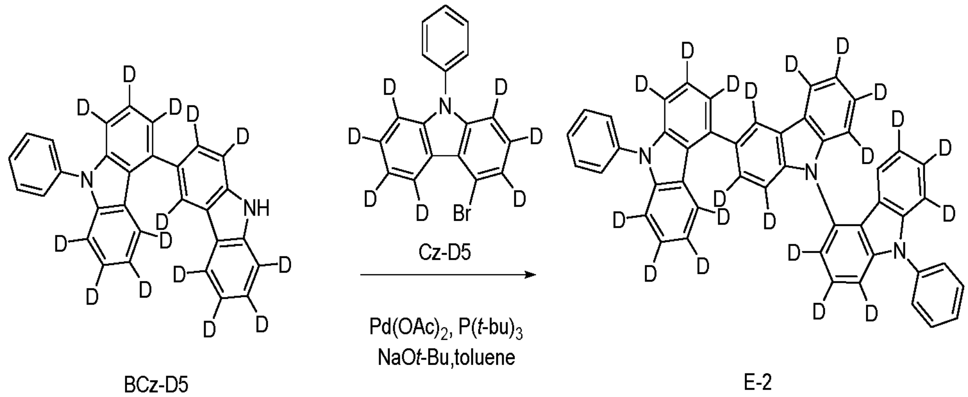


BCz-D5 E-2

The same procedure as in Synthesis Example 38 was performed except that Cz-D5 (9.3 g, 23.6 mmol) was used instead of Cz-D1, thereby obtaining the target compound E-2 (10.00 g, yield: 63%).
Mass (calcd.: 670.93, found: 670 g/mol)

**[Synthesis Example 40] Synthesis of E-3**

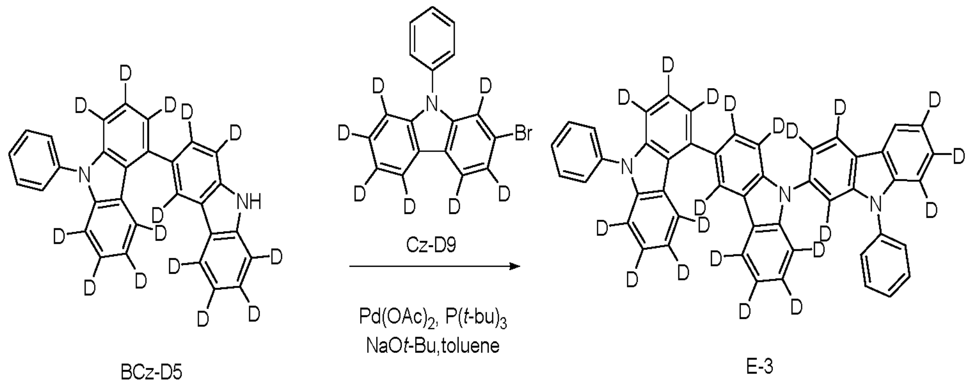


BCz-D5 E-3

The same procedure as in Synthesis Example 38 was performed except that Cz-D9 (9.3 g, 23.6 mmol) was used instead of Cz-D1, thereby obtaining the target compound E-3 (8.73 g, yield: 55%).
Mass (calcd.: 670.93, found: 670 g/mol)

**[Synthesis Example 41] Synthesis of E-4**

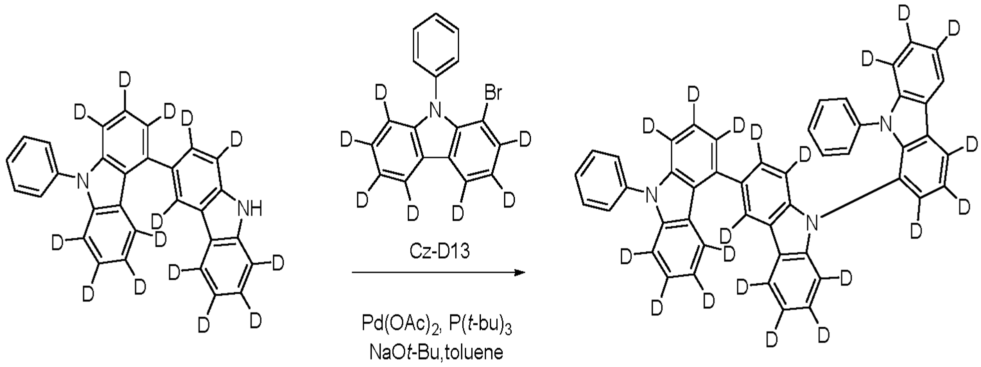


BCz-D5 E-4

The same procedure as in Synthesis Example 38 was performed except that Cz-D13 (9.3 g, 23.6 mmol) was used

instead of Cz-D1, thereby obtaining the target compound E-4 (9.21 g, yield: 58%).
Mass (calcd.: 670.93, found: 670 g/mol)

**[Synthesis Example 42] Synthesis of F-1**

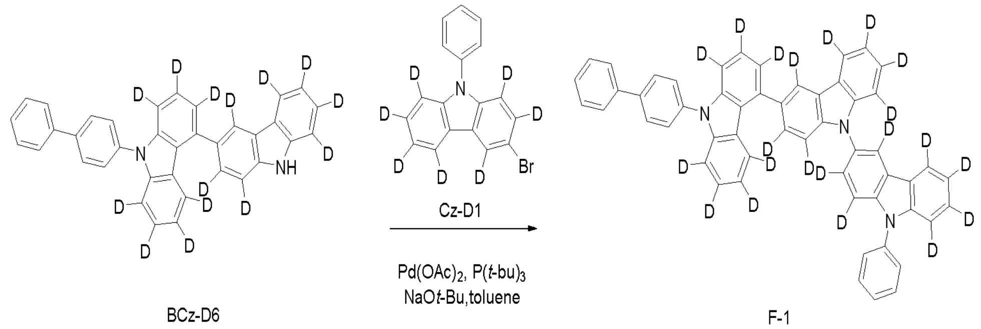


BCz-D6 F-1

Under a nitrogen atmosphere, BCz-D6 (10.0 g, 20.1 mmol), Cz-D1 (7.9 g, 24.1 mmol), Pd(OAc)$_2$ (1.15 g, 1.0 mmol), P($t$-Bu)$_3$ (0.49 ml, 2.0 mmol), NaO($t$-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound F-1 (13.79 g, yield: 78%).
Mass (calcd.: 747.02, found: 747 g/mol)

**[Synthesis Example 43] Synthesis of F-2**

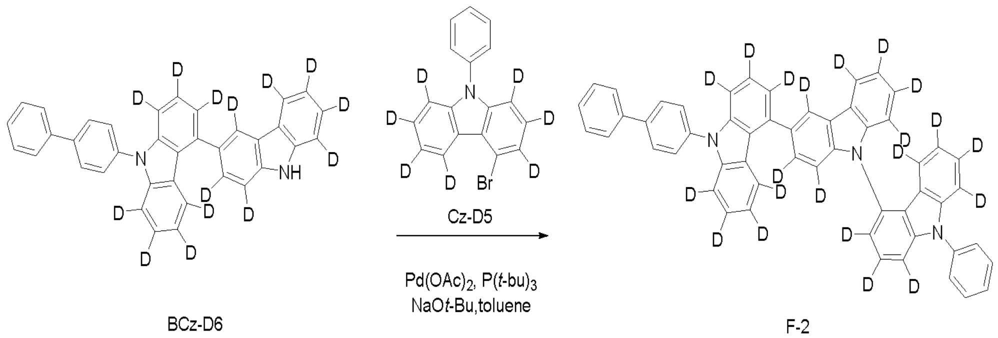


BCz-D6 F-2

The same procedure as in Synthesis Example 42 was performed except that Cz-D5 (7.9 g, 24.1 mmol) was used instead of Cz-D1, thereby obtaining the target compound F-2 (11.84 g, yield: 67%).
Mass (calcd.: 747.02, found: 747 g/mol)

**[Synthesis Example 44] Synthesis of F-3**

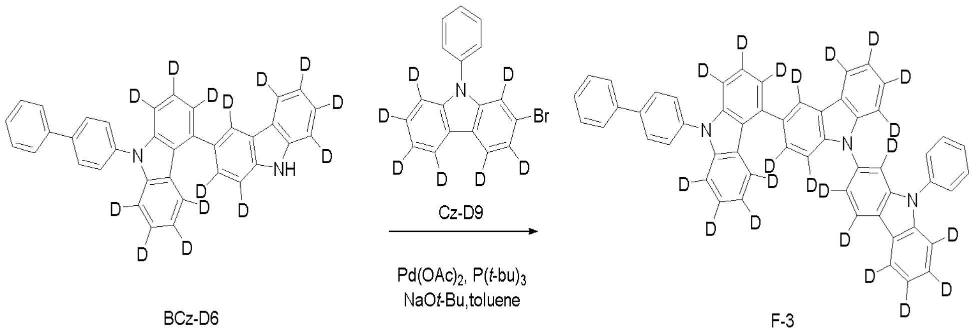


BCz-D6 F-3

The same procedure as in Synthesis Example 42 was performed except that Cz-D9 (7.9 g, 24.1 mmol) was used instead of Cz-D1, thereby obtaining the target compound F-3 (11.31 g, yield: 64%).
Mass (calcd.: 747.02, found: 747 g/mol)

**[Synthesis Example 45] Synthesis of F-4**

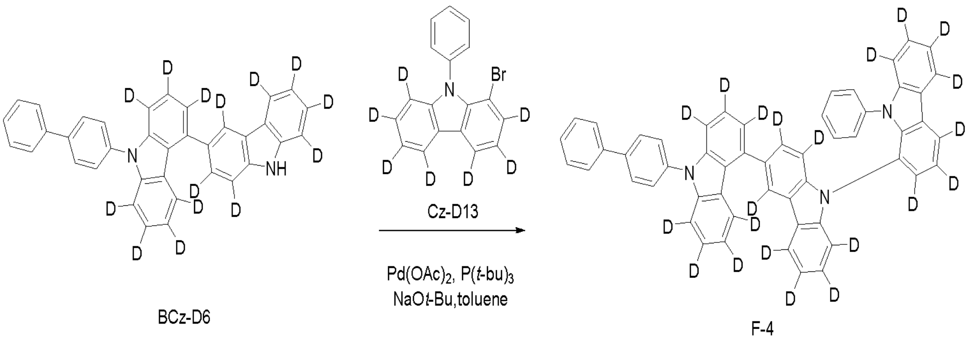


BCz-D6 F-4

The same procedure as in Synthesis Example 42 was performed except that Cz-D13 (7.9 g, 24.1 mmol) was used instead of Cz-D1, thereby obtaining the target compound F-4 (9.90 g, yield: 56%).
Mass (calcd.: 747.02, found: 747 g/mol)

**[Synthesis Example 46] Synthesis of G-1**

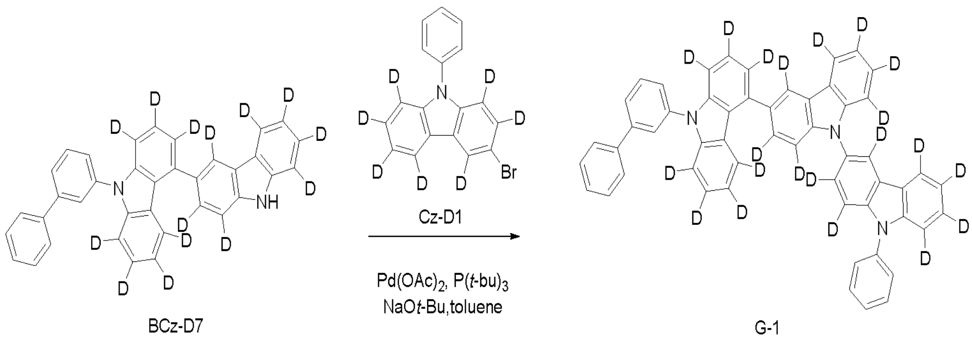


BCz-D7 G-1

Under a nitrogen atmosphere, BCz-D7 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound G-1 (12.90 g, yield: 73%)
Mass (calcd.: 747.02, found: 747 g/mol)

**[Synthesis Example 47] Synthesis of G-2**

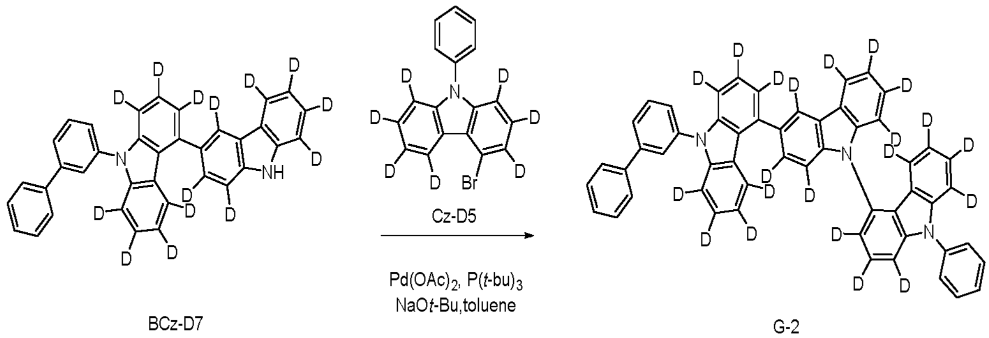


BCz-D7 G-2

The same procedure as in Synthesis Example 46 was performed except that Cz-D5 (7.9 g, 24.1 mmol) was used instead of Cz-D1, thereby obtaining the target compound G-2 (12.55 g, yield: 71%).
Mass (calcd.: 747.02, found: 747 g/mol)

**[Synthesis Example 48] Synthesis of G-3**

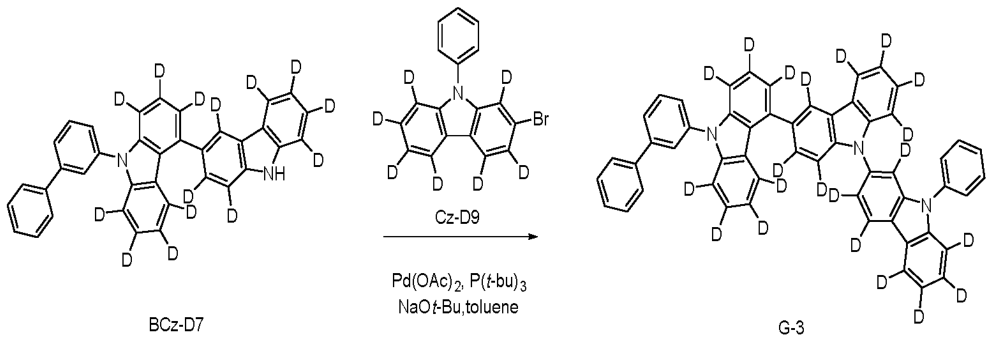


BCz-D7 G-3

The same procedure as in Synthesis Example 46 was performed except that Cz-D9 (7.9 g, 24.1 mmol) was used instead of Cz-D1, thereby obtaining the target compound G-3 (13.08 g, yield: 74%).
Mass (calcd.: 747.02, found: 747 g/mol)

**[Synthesis Example 49] Synthesis of G-4**

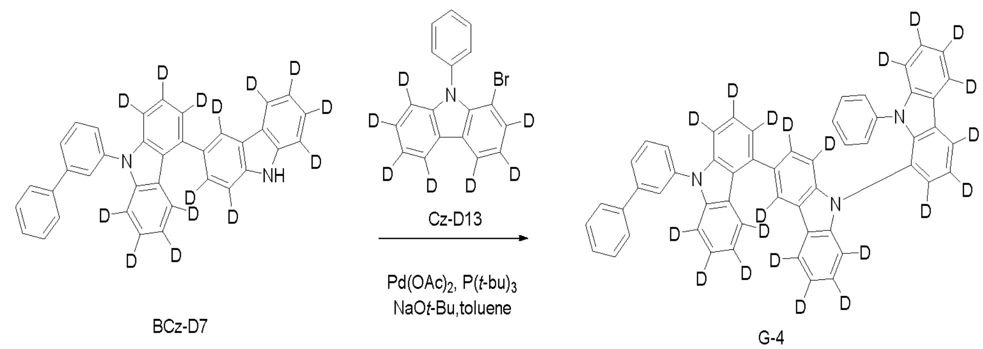


BCz-D7 G-4

The same procedure as in Synthesis Example 46 was performed except that Cz-D13 (7.9 g, 24.1 mmol) was used instead of Cz-D1, thereby obtaining the target compound G-4 (9.37 g, yield: 53%).

Mass (calcd.: 747.02, found: 747 g/mol)

**[Synthesis Example 50] Synthesis of H-1**

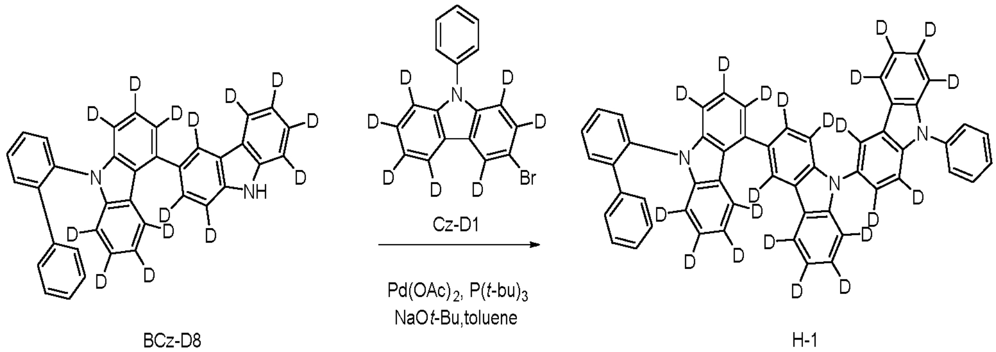


BCz-D8 H-1

Under a nitrogen atmosphere, BCz-D8 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound H-1 (13.42 g, yield: 76%)
Mass (calcd.: 747.02, found: 747 g/mol)

**[Synthesis Example 51] Synthesis of H-2**

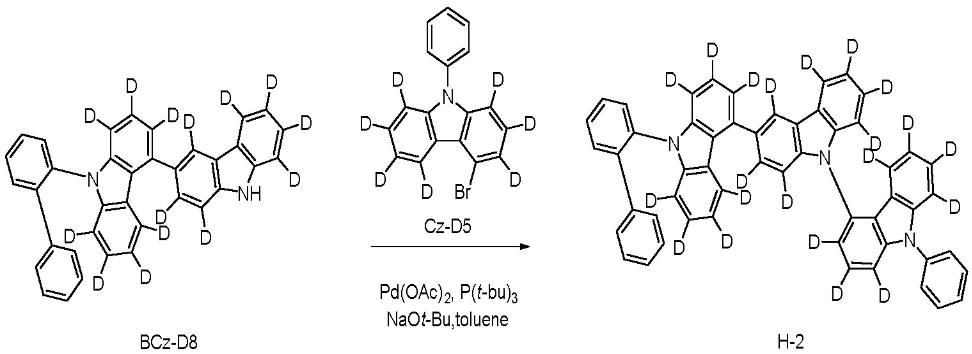


BCz-D8 H-2

The same procedure as in Synthesis Example 50 was performed except that Cz-D5 (7.9 g, 24.1 mmol) was used instead of Cz-D1, thereby obtaining the target compound H-2 (11.84 g, yield: 67%).
Mass (calcd.: 747.02, found: 747 g/mol)

**[Synthesis Example 52] Synthesis of H-3**

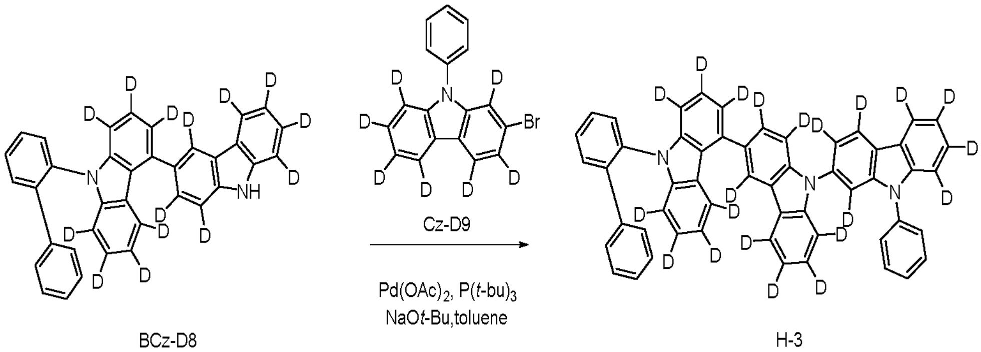


BCz-D8 H-3

The same procedure as in Synthesis Example 50 was performed except that Cz-D9 (7.9 g, 24.1 mmol) was used instead of Cz-D1, thereby obtaining the target compound H-3 (13.26 g, yield: 75%).
Mass (calcd.: 747.02, found: 747 g/mol)

**[Synthesis Example 53] Synthesis of H-4**

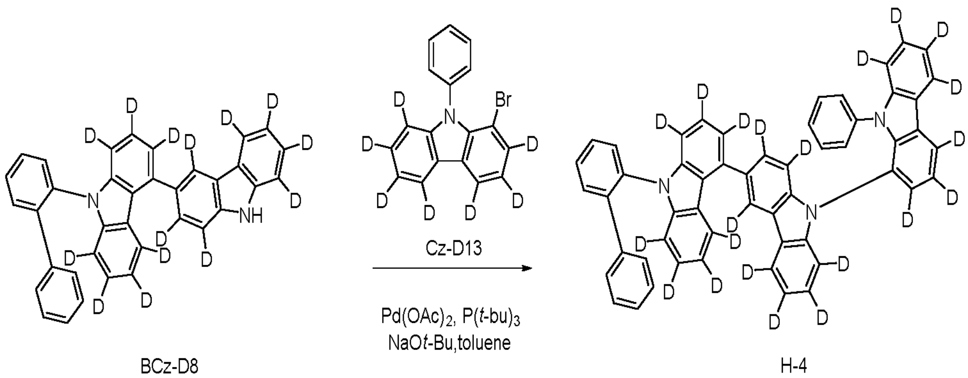


BCz-D8 H-4

The same procedure as in Synthesis Example 50 was performed except that Cz-D13 (7.9 g, 24.1 mmol) was used instead of Cz-D1, thereby obtaining the target compound H-4 (9.19 g, yield: 52%).
Mass (calcd.: 747.02, found: 747 g/mol)

**[Synthesis Example 54] Synthesis of I-1**

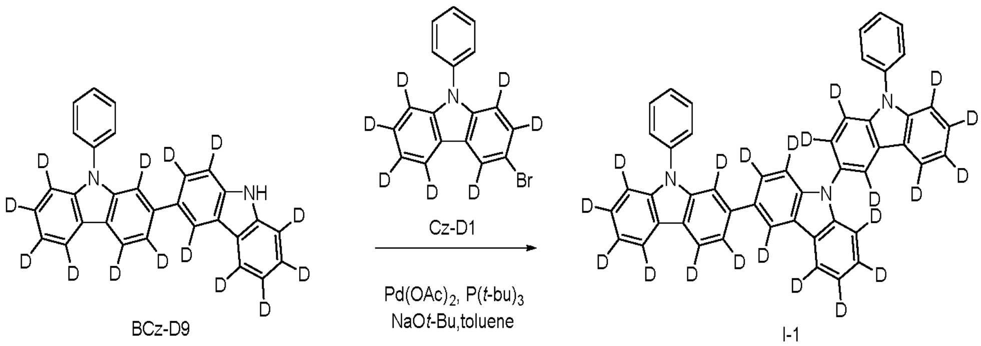


BCz-D9 I-1

Under a nitrogen atmosphere, BCz-D9 (10.0 g, 23.6 mmol), Cz-D1 (9.3 g, 28.3 mmol), $Pd(OAc)_2$ (1.36 g, 1.18 mmol), $P(t\text{-}Bu)_3$ (0.57 ml, 2.36 mmol), NaO(*t*-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound I-1 (11.27 g, yield: 71%).
Mass (calcd.: 670.93, found: 670 g/mol)

## [Synthesis Example 55] Synthesis of I-2

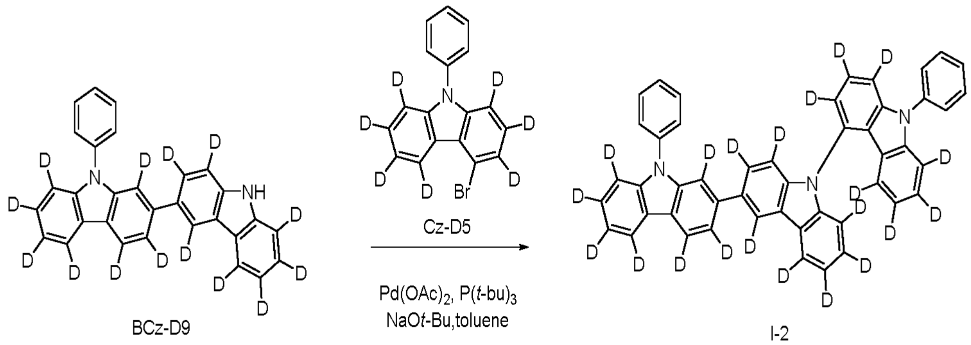


The same procedure as in Synthesis Example 54 was performed except that Cz-D5 (9.3 g, 23.6 mmol) was used instead of Cz-D1, thereby obtaining the target compound I-2 (10.63 g, yield: 67%).
Mass (calcd.: 670.93, found: 670 g/mol)

## [Synthesis Example 56] Synthesis of I-3

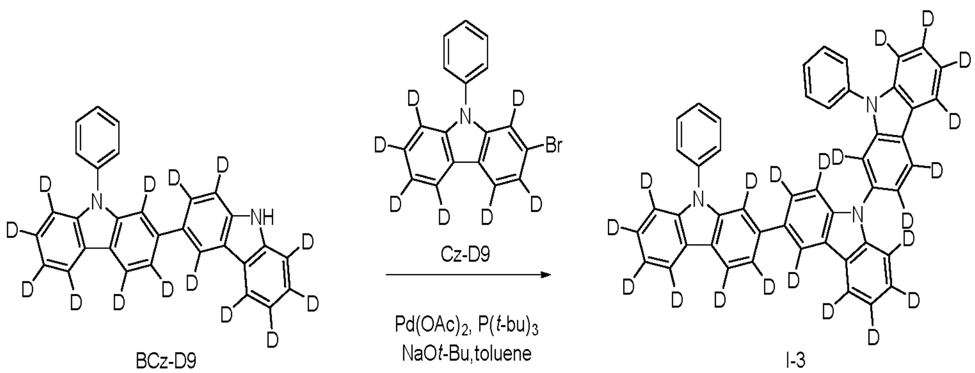


The same procedure as in Synthesis Example 54 was performed except that Cz-D9 (9.3 g, 23.6 mmol) was used instead of Cz-D1, thereby obtaining the target compound I-3 (8.25 g, yield: 52%).
Mass (calcd.: 670.93, found: 670 g/mol)

## [Synthesis Example 57] Synthesis of I-4

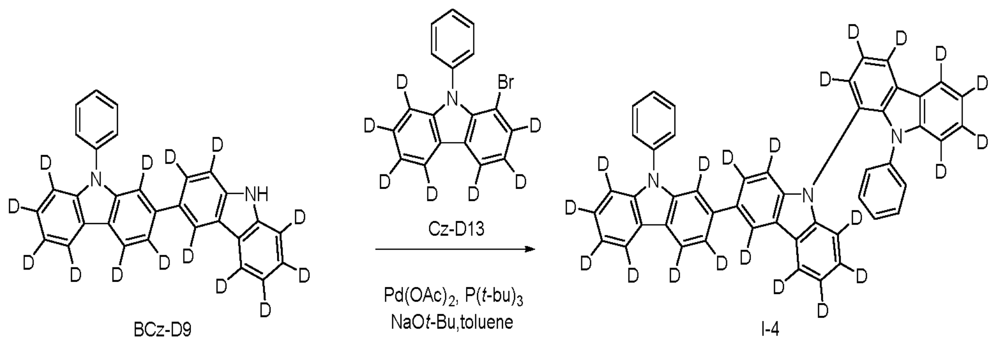


The same procedure as in Synthesis Example 54 was performed except that Cz-D13 (9.3 g, 23.6 mmol) was used

instead of Cz-D1, thereby obtaining the target compound I-4 (8.10 g, yield: 51%).
Mass (calcd.: 670.93, found: 670 g/mol)

**[Synthesis Example 58] Synthesis of J-1**

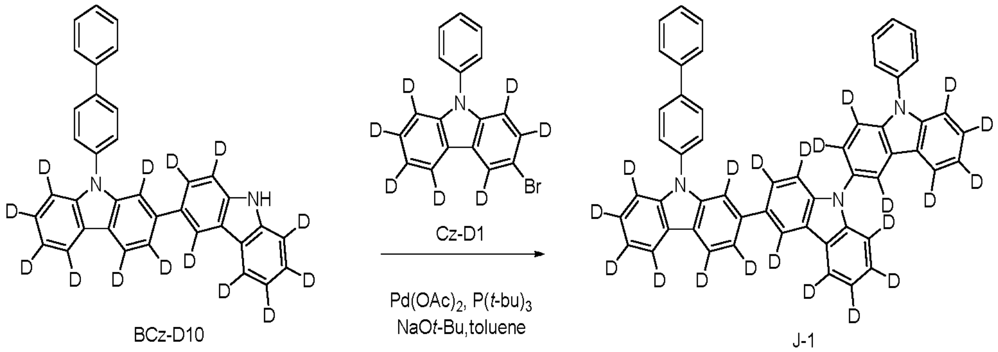


BCz-D10 J-1

Under a nitrogen atmosphere, BCz-D10 (10.0 g, 20.1 mmol), Cz-D1 (7.9 g, 24.1 mmol), $Pd(OAc)_2$ (1.15 g, 1.0 mmol), P(*t*-Bu)$_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound J-1 (12.90 g, yield: 73%).
Mass (calcd.: 747.02, found: 747 g/mol)

**[Synthesis Example 59] Synthesis of J-2**

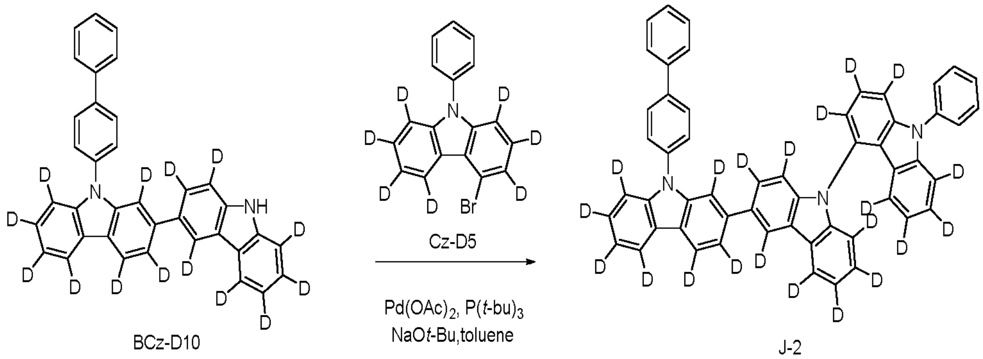


BCz-D10 J-2

The same procedure as in Synthesis Example 58 was performed except that Cz-D5 (7.9 g, 24.1 mmol) was used instead of Cz-D1, thereby obtaining the target compound J-2 (11.49 g, yield: 65%).
Mass (calcd.: 747.02, found: 747 g/mol)

**[Synthesis Example 60] Synthesis of J-3**

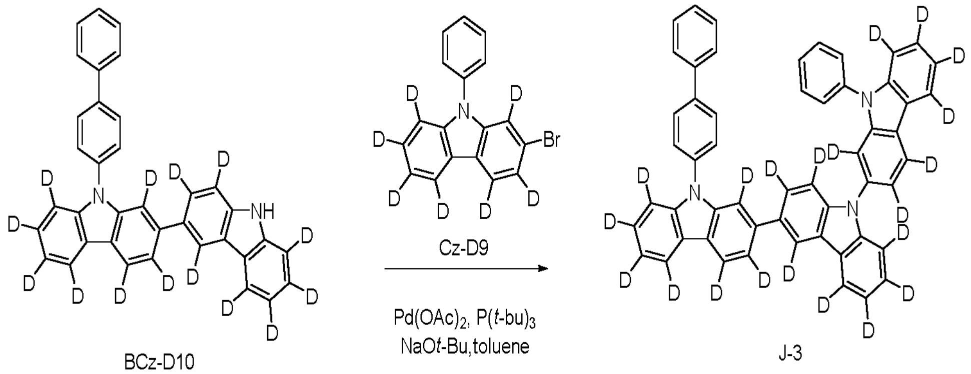


BCz-D10 J-3

The same procedure as in Synthesis Example 58 was performed except that Cz-D9 (7.9 g, 24.1 mmol) was used instead of Cz-D1, thereby obtaining the target compound J-3 (10.95 g, yield: 62%).
Mass (calcd.: 747.02, found: 747 g/mol)

**[Synthesis Example 61] Synthesis of J-4**

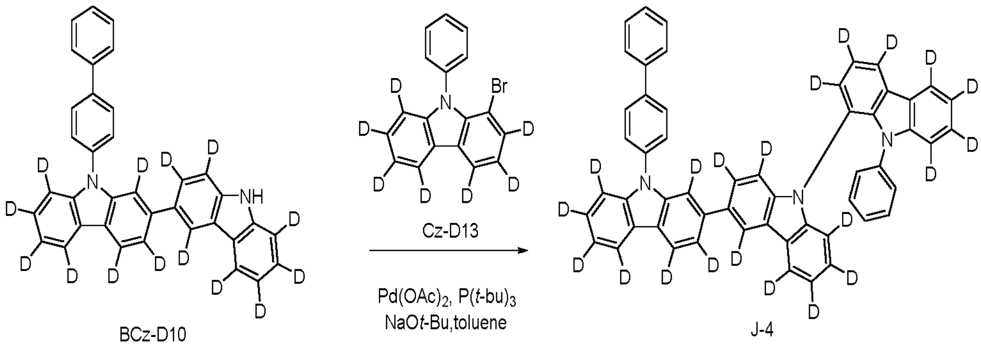


BCz-D10 J-4

The same procedure as in Synthesis Example 58 was performed except that Cz-D13 (7.9 g, 24.1 mmol) was used instead of Cz-D1, thereby obtaining the target compound J-4 (10.25 g, yield: 58%).
Mass (calcd.: 747.02, found: 747 g/mol)

**[Synthesis Example 62] Synthesis of K-1**

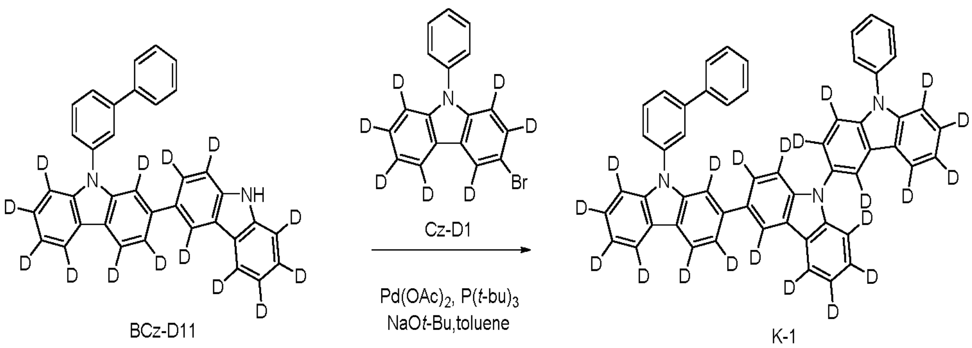


BCz-D11 K-1

Under a nitrogen atmosphere, BCz-D11 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound K-1 (12.37 g, yield: 70%)
Mass (calcd.: 747.02, found: 747 g/mol)

**[Synthesis Example 63] Synthesis of K-2**

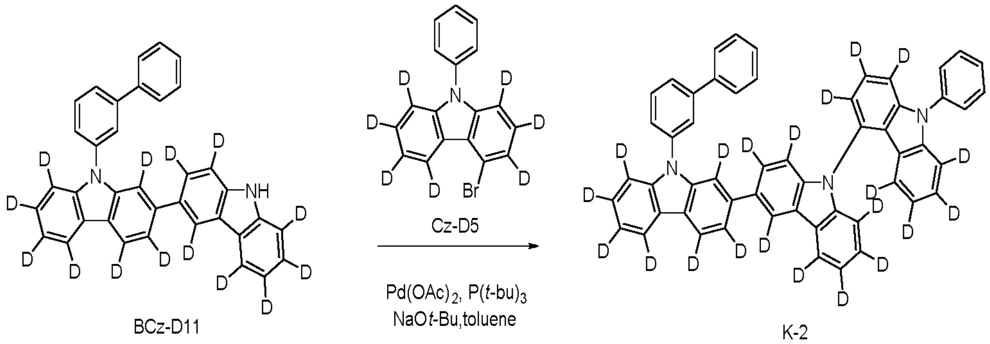


The same procedure as in Synthesis Example 62 was performed except that Cz-D5 (7.9 g, 24.1 mmol) was used instead of Cz-D1, thereby obtaining the target compound K-2 (13.61 g, yield: 77%).
Mass (calcd.: 747.02, found: 747 g/mol)

**[Synthesis Example 64] Synthesis of K-3**

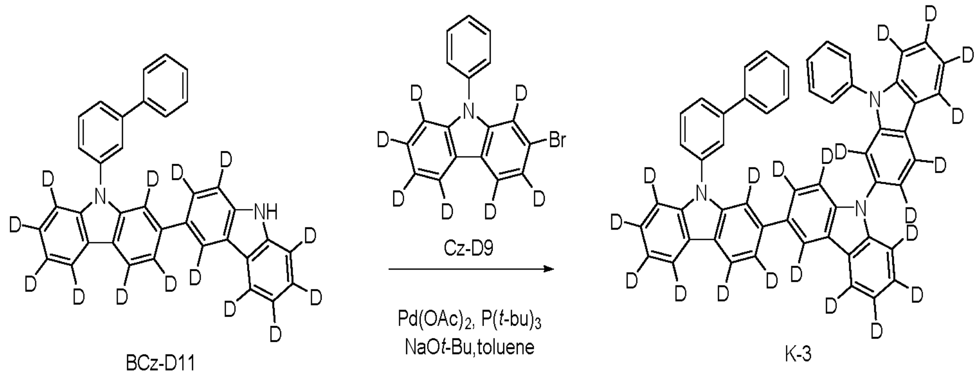


The same procedure as in Synthesis Example 62 was performed except that Cz-D9 (7.9 g, 24.1 mmol) was used instead of Cz-D1, thereby obtaining the target compound K-3 (12.55 g, yield: 71%).
Mass (calcd.: 747.02, found: 747 g/mol)

**[Synthesis Example 65] Synthesis of K-4**

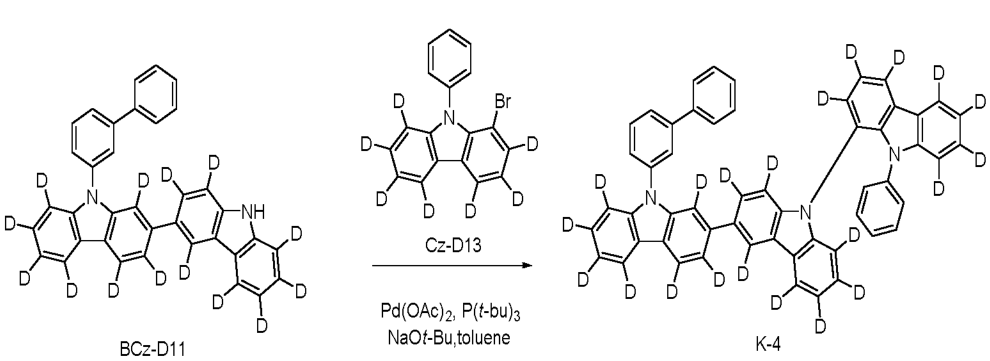


The same procedure as in Synthesis Example 62 was performed except that Cz-D13 (7.9 g, 24.1 mmol) was used

instead of Cz-D1, thereby obtaining the target compound K-4 (10.61 g, yield: 60%).
Mass (calcd.: 747.02, found: 747 g/mol)

**[Synthesis Example 66] Synthesis of L-1**

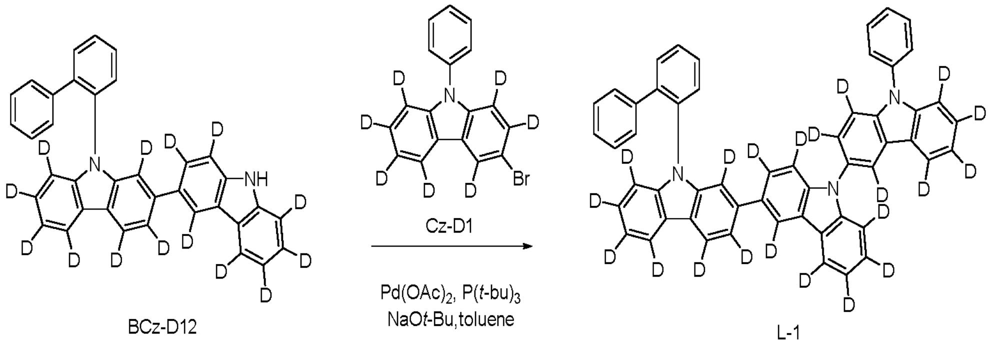


BCz-D12 L-1

Under a nitrogen atmosphere, BCz-D12 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound L-1 (13.42 g, yield: 76%)
Mass (calcd.: 747.02, found: 747 g/mol)

**[Synthesis Example 67] Synthesis of L-2**

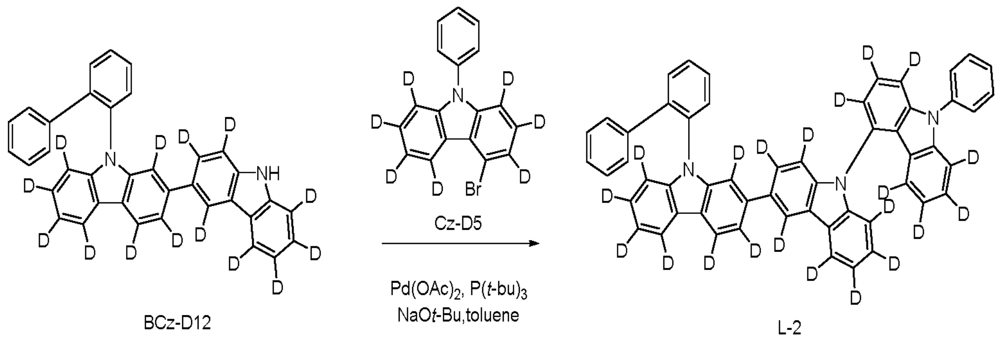


BCz-D12 L-2

The same procedure as in Synthesis Example 66 was performed except that Cz-D5 (7.9 g, 24.1 mmol) was used instead of Cz-D1, thereby obtaining the target compound L-2 (11.84 g, yield: 67%) .
Mass (calcd.: 747.02, found: 747 g/mol)

**[Synthesis Example 68] Synthesis of L-3**

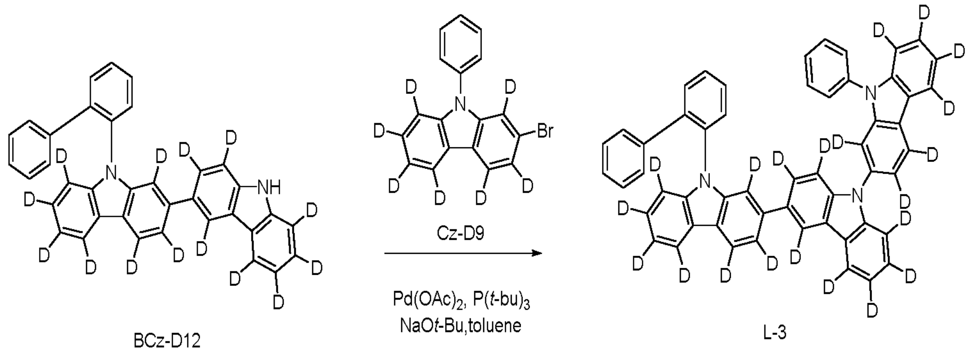


BCz-D12 L-3

The same procedure as in Synthesis Example 66 was performed except that Cz-D9 (7.9 g, 24.1 mmol) was used instead of Cz-D1, thereby obtaining the target compound L-3 (13.26 g, yield: 75%).
Mass (calcd.: 747.02, found: 747 g/mol)

**[Synthesis Example 69] Synthesis of L-4**

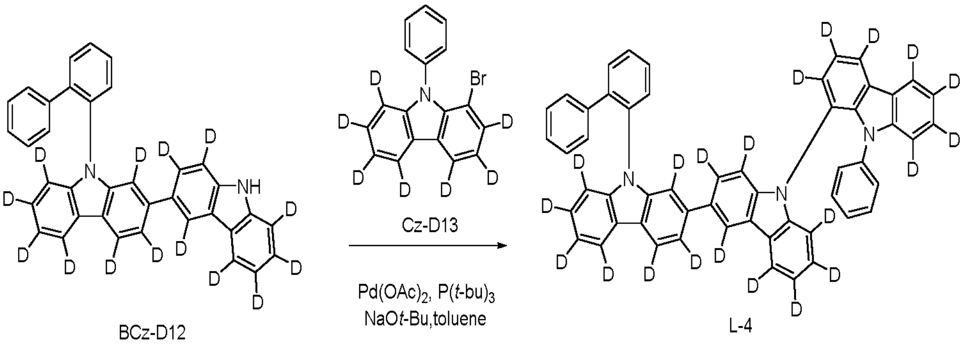


BCz-D12 L-4

The same procedure as in Synthesis Example 66 was performed except that Cz-D13 (7.9 g, 24.1 mmol) was used instead of Cz-D1, thereby obtaining the target compound L-4 (9.19 g, yield: 52%).
Mass (calcd.: 747.02, found: 747 g/mol)

**[Synthesis Example 70] Synthesis of M-1**

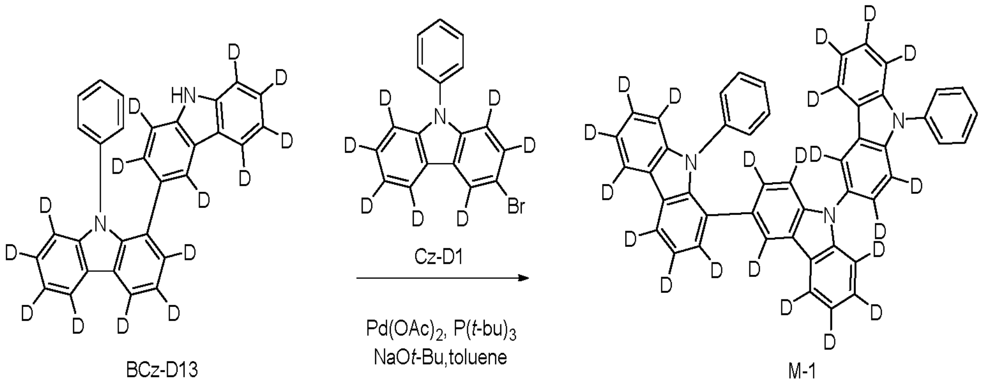


BCz-D13 M-1

Under a nitrogen atmosphere, BCz-D13 (10.0 g, 23.6 mmol), Cz-D1 (9.3 g, 28.3 mmol), $Pd(OAc)_2$ (1.36 g, 1.18 mmol), $P(t\text{-}Bu)_3$ (0.57 ml, 2.36 mmol), NaO(*t*-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound M-1 (10.16 g, yield: 64%).

Mass (calcd.: 670.93, found: 670 g/mol)

**[Synthesis Example 71] Synthesis of M-2**

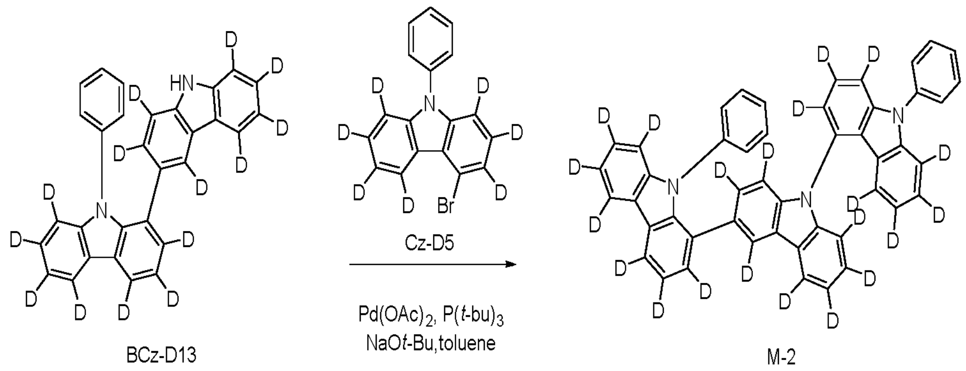


The same procedure as in Synthesis Example 70 was performed except that Cz-D5 (9.3 g, 23.6 mmol) was used instead of Cz-D1, thereby obtaining the target compound M-2 (9.37 g, yield: 59%).
Mass (calcd.: 670.93, found: 670 g/mol)

**[Synthesis Example 72] Synthesis of M-3**

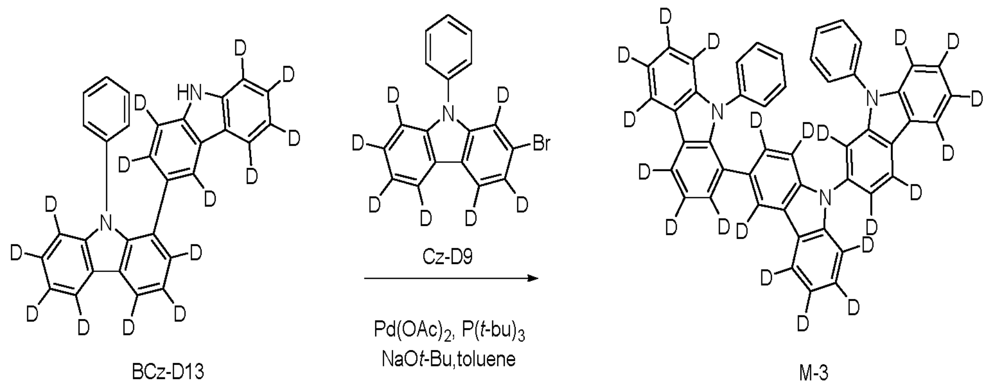


The same procedure as in Synthesis Example 70 was performed except that Cz-D9 (9.3 g, 23.6 mmol) was used instead of Cz-D1, thereby obtaining the target compound M-3 (7.78 g, yield: 49%).
Mass (calcd.: 670.93, found: 670 g/mol)

**[Synthesis Example 73] Synthesis of M-4**

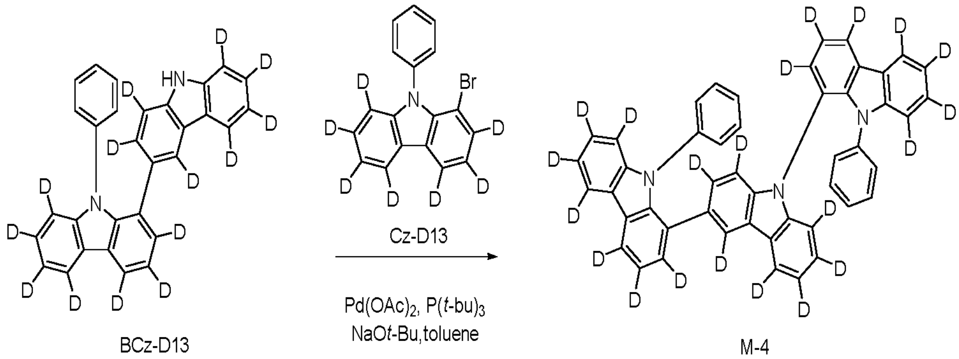


BCz-D13 M-4

The same procedure as in Synthesis Example 70 was performed except that Cz-D13 (9.3 g, 23.6 mmol) was used instead of Cz-D1, thereby obtaining the target compound M-4 (6.82 g, yield: 43%).
Mass (calcd.: 670.93, found: 670 g/mol)

**<u>[Synthesis Example 74] Synthesis of N-1</u>**

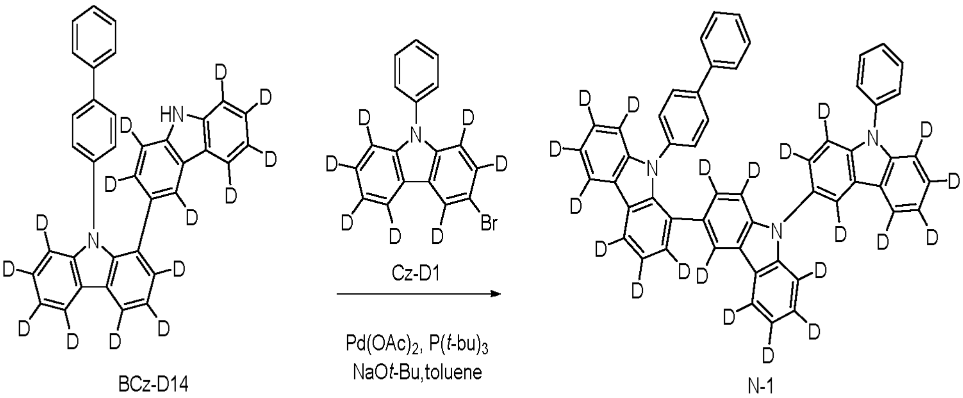


BCz-D14 N-1

Under a nitrogen atmosphere, BCz-D14 (10.0 g, 20.1 mmol), Cz-D1 (7.9 g, 24.1 mmol), $Pd(OAc)_2$ (1.15 g, 1.0 mmol), P($t$-Bu)$_3$ (0.49 ml, 2.0 mmol), NaO($t$-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound N-1 (11.49 g, yield: 65%).
Mass (calcd.: 747.02, found: 747 g/mol)

**<u>[Synthesis Example 75] Synthesis of N-2</u>**

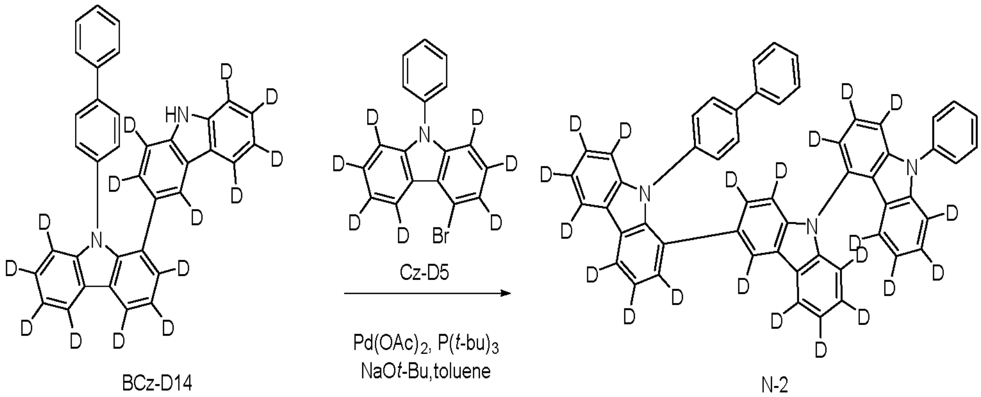


BCz-D14 N-2

The same procedure as in Synthesis Example 74 was performed except that Cz-D5 (7.9 g, 24.1 mmol) was used instead of Cz-D1, thereby obtaining the target compound N-2 (10.96 g, yield: 62%).
Mass (calcd.: 747.02, found: 747 g/mol)

**[Synthesis Example 76] Synthesis of N-3**

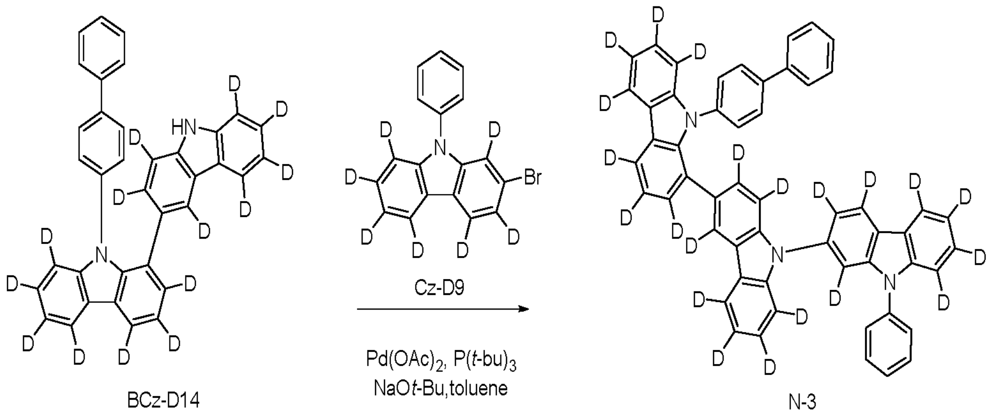


BCz-D14 N-3

The same procedure as in Synthesis Example 74 was performed except that Cz-D9 (7.9 g, 24.1 mmol) was used instead of Cz-D1, thereby obtaining the target compound N-3 (9.55 g, yield: 54%).
Mass (calcd.: 747.02, found: 747 g/mol)

**[Synthesis Example 77] Synthesis of N-4**

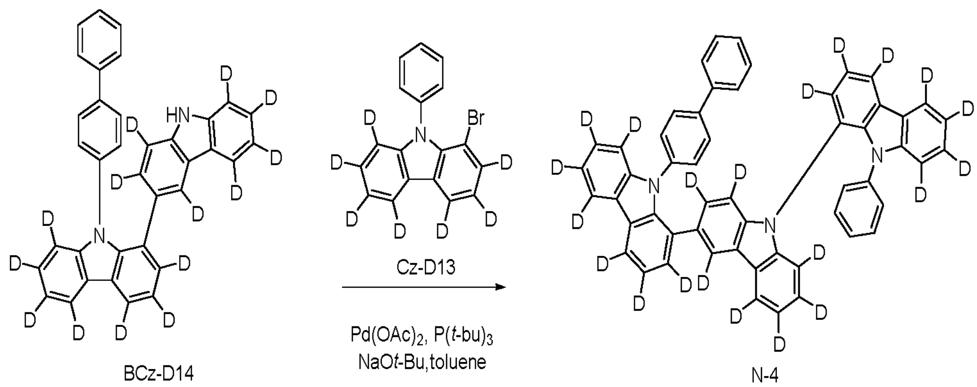


BCz-D14 N-4

The same procedure as in Synthesis Example 74 was performed except that Cz-D13 (7.9 g, 24.1 mmol) was used instead of Cz-D1, thereby obtaining the target compound N-4 (8.84 g, yield: 50%).
Mass (calcd.: 747.02, found: 747 g/mol)

**[Synthesis Example 78] Synthesis of O-1**

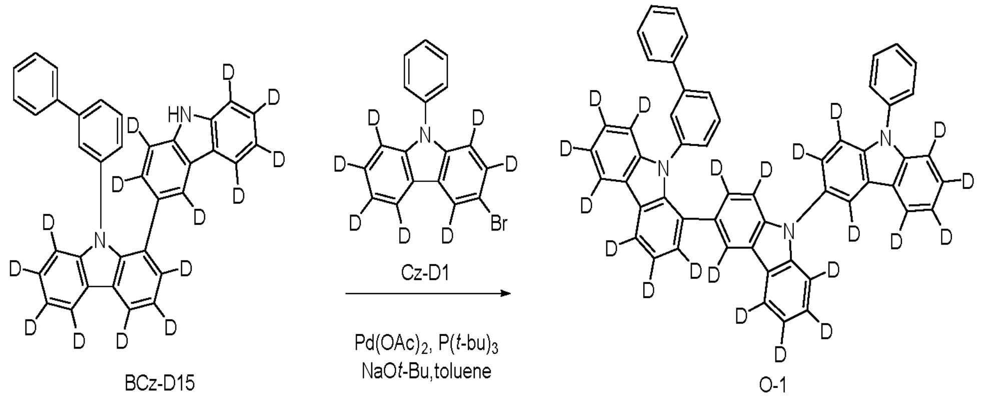

BCz-D15 O-1

Under a nitrogen atmosphere, BCz-D15 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound O-1 (11.14 g, yield: 63%)
Mass (calcd.: 747.02, found: 747 g/mol)

**[Synthesis Example 79] Synthesis of O-2**

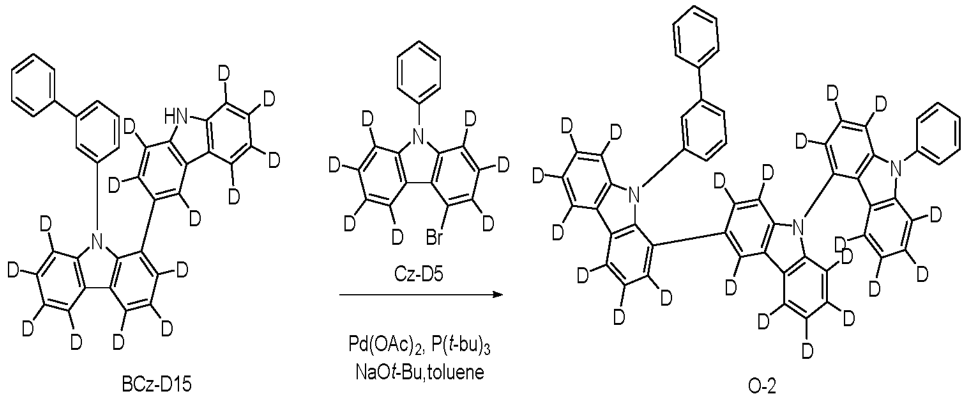

BCz-D15 O-2

The same procedure as in Synthesis Example 78 was performed except that Cz-D5 (7.9 g, 24.1 mmol) was used instead of Cz-D1, thereby obtaining the target compound O-2 (11.49 g, yield: 65%).
Mass (calcd.: 747.02, found: 747 g/mol)

**[Synthesis Example 80] Synthesis of O-3**

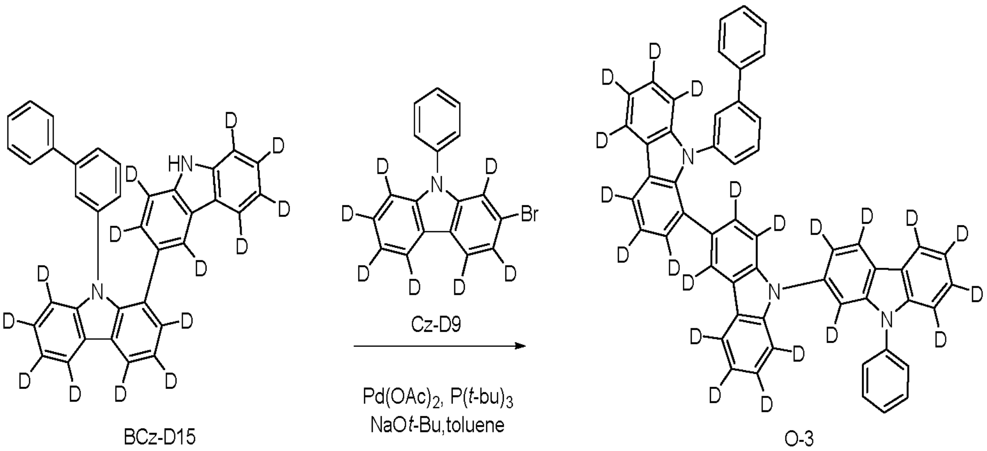


BCz-D15 O-3

The same procedure as in Synthesis Example 78 was performed except that Cz-D9 (7.9 g, 24.1 mmol) was used instead of Cz-D1, thereby obtaining the target compound O-3 (10.07 g, yield: 57%).
Mass (calcd.: 747.02, found: 747 g/mol)

## [Synthesis Example 81] Synthesis of O-4

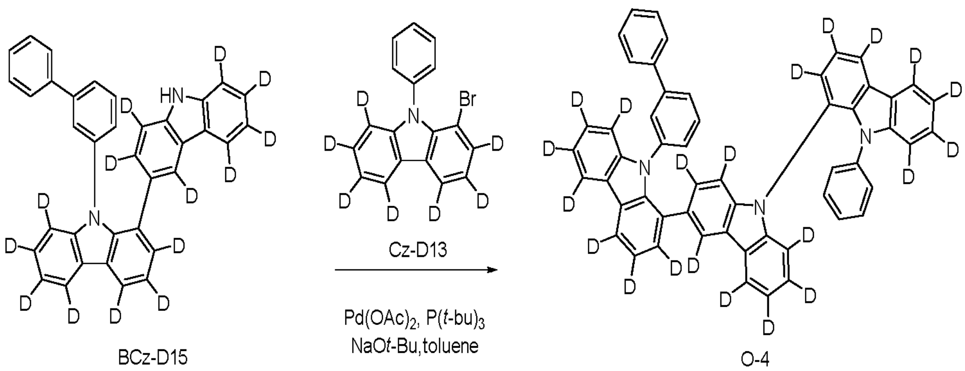


BCz-D15 O-4

The same procedure as in Synthesis Example 78 was performed except that Cz-D13 (7.9 g, 24.1 mmol) was used instead of Cz-D1, thereby obtaining the target compound O-4 (8.49 g, yield: 48%).
Mass (calcd.: 747.02, found: 747 g/mol)

## [Synthesis Example 82] Synthesis of P-1

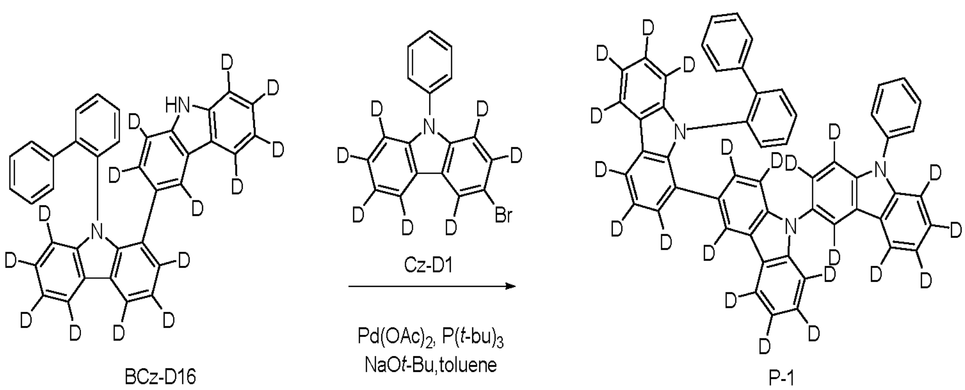


BCz-D16 P-1

Under a nitrogen atmosphere, BCz-D16 (10.0 g, 20.1 mmol), Cz-D1 (7.9 g, 24.1 mmol), $Pd(OAc)_2$ (1.15 g, 1.0 mmol), $P(t\text{-}Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound P-1 (10.95 g, yield: 62%)
Mass (calcd.: 747.02, found: 747 g/mol)

**[Synthesis Example 83] Synthesis of P-2**

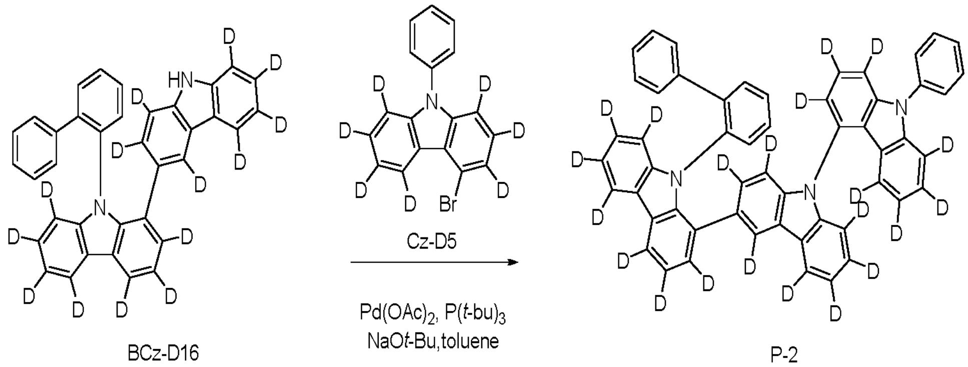


BCz-D16 P-2

The same procedure as in Synthesis Example 82 was performed except that Cz-D5 (7.9 g, 24.1 mmol) was used instead of Cz-D1, thereby obtaining the target compound P-2 (11.31 g, yield: 64%).
Mass (calcd.: 747.02, found: 747 g/mol)

**[Synthesis Example 84] Synthesis of P-3**

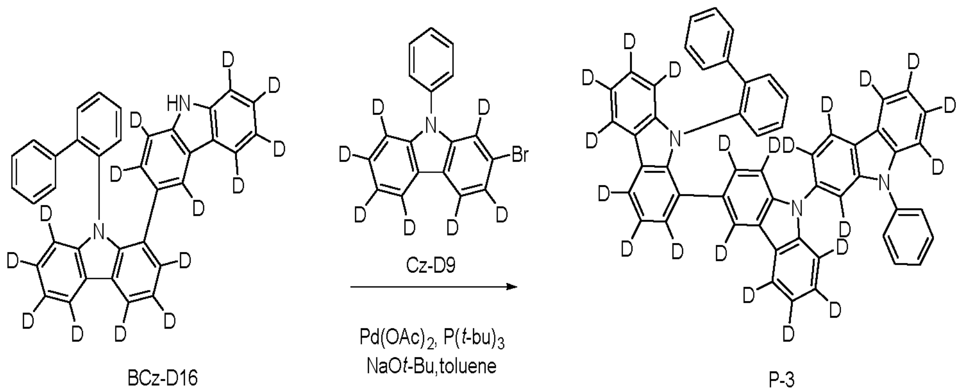


BCz-D16 P-3

The same procedure as in Synthesis Example 82 was performed except that Cz-D9 (7.9 g, 24.1 mmol) was used instead of Cz-D1, thereby obtaining the target compound P-3 (11.84 g, yield: 67%).
Mass (calcd.: 747.02, found: 747 g/mol)

**[Synthesis Example 85] Synthesis of P-4**

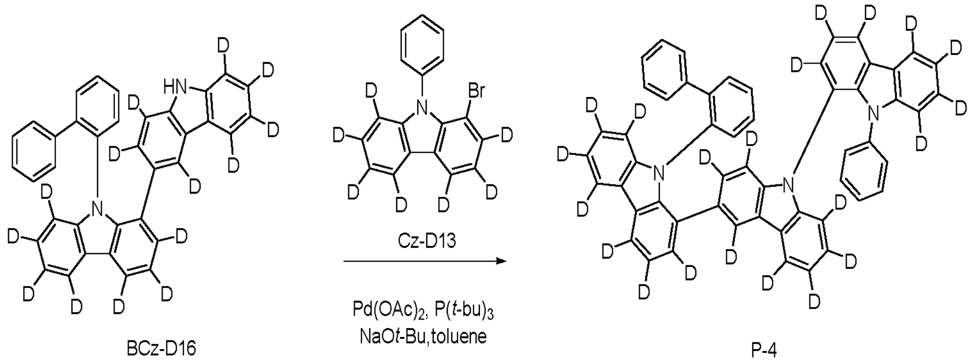


]　BCz-D16　P-4

The same procedure as in Synthesis Example 82 was performed except that Cz-D13 (7.9 g, 24.1 mmol) was used instead of Cz-D1, thereby obtaining the target compound P-4 (9.55 g, yield: 54%).
Mass (calcd.: 747.02, found: 747 g/mol)

**[Synthesis Example 86] Synthesis of Q-1**

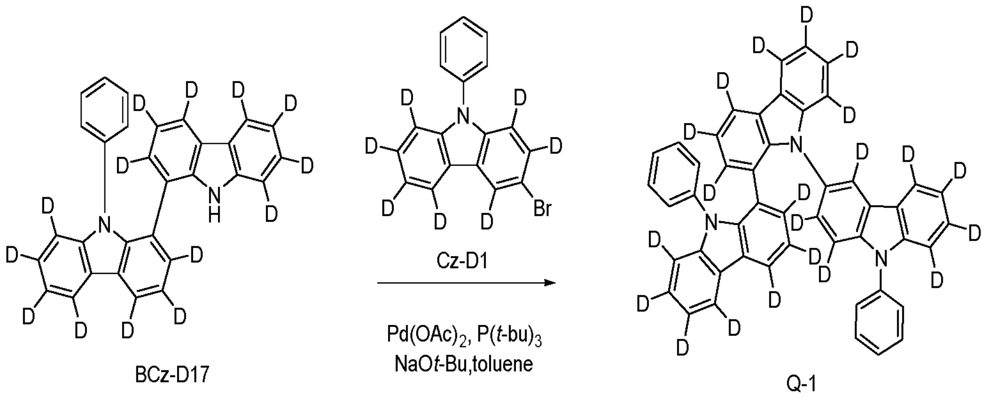


BCz-D17　Q-1

Under a nitrogen atmosphere, BCz-D17 (10.0 g, 23.6 mmol), Cz-D1 (9.3 g, 28.3 mmol), $Pd(OAc)_2$ (1.36 g, 1.18 mmol), $P(t\text{-}Bu)_3$ (0.57 ml, 2.36 mmol), NaO(*t*-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound Q-1 (6.67 g, yield: 42%).
Mass (calcd.: 670.93, found: 670 g/mol)

**[Synthesis Example 87] Synthesis of R-1**

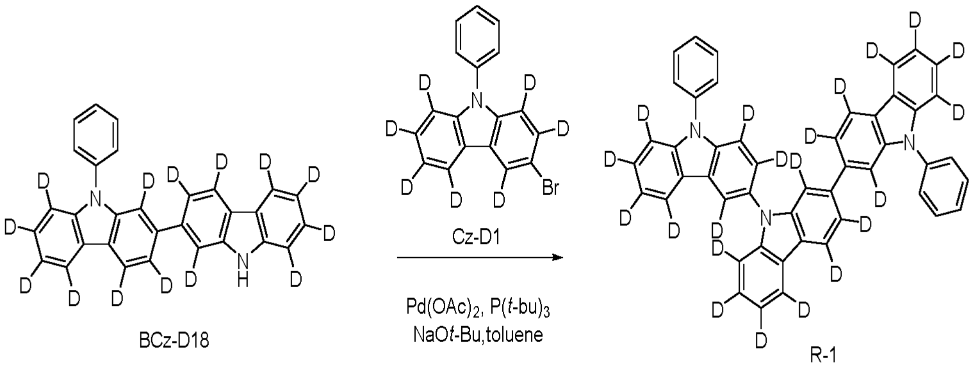


BCz-D18　R-1

Under a nitrogen atmosphere, BCz-D18 (10.0 g, 23.6 mmol), Cz-D1 (9.3 g, 28.3 mmol), $Pd(OAc)_2$ (1.36 g, 1.18 mmol), $P(t\text{-Bu})_3$ (0.57 ml, 2.36 mmol), NaO(*t*-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound R-1 (11.27 g, yield: 71%).
Mass (calcd.: 670.93, found: 670 g/mol)

**<u>[Synthesis Example 88] Synthesis</u>** <u>of S-1</u>

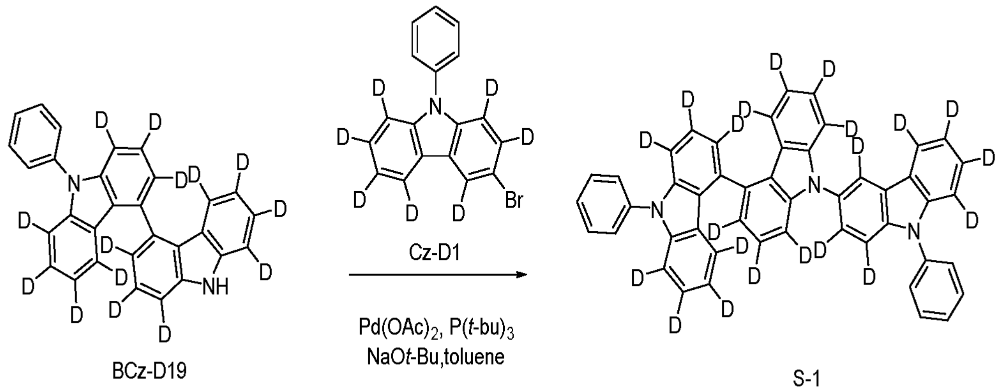


BCz-D19 S-1

Under a nitrogen atmosphere, BCz-D19 (10.0 g, 23.6 mmol), Cz-D1 (9.3 g, 28.3 mmol), $Pd(OAc)_2$ (1.36 g, 1.18 mmol), $P(t\text{-Bu})_3$ (0.57 ml, 2.36 mmol), NaO(*t*-Bu) (4.55 g, 47.3 mmol), and toluene (100 ml) were mixed, and stirred at 110°C for 5 hours. After the reaction was completed, toluene was concentrated, and the solid salt was filtered and then purified by recrystallization, thereby obtaining the target compound S-1 (11.75 g, yield: 74%).
Mass (calcd.: 670.93, found: 670 g/mol)

**<u>[Synthesis Example 89] Synthesis of T-1</u>**

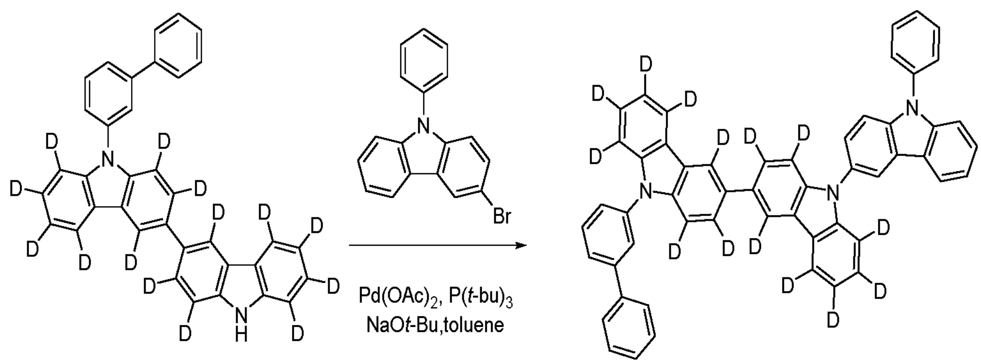


BCz-D3 T-1

Under a nitrogen atmosphere, BCz-D3 (10.0 g, 20.1 mmol), 3-bromo-9-phenyl-9H-carbazole (7.8 g, 24.1 mmol), $Pd(OAc)_2$ (1.15 g, 1.0 mmol), $P(t\text{-Bu})_3$ (0.49 ml, 2.0 mmol), NaO(t-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound T-1 (10.8 g, yield: 73%).
Mass (calcd.: 739.98, found: 739 g/mol)

**<u>[Synthesis Example 90] Synthesis of U-1</u>**

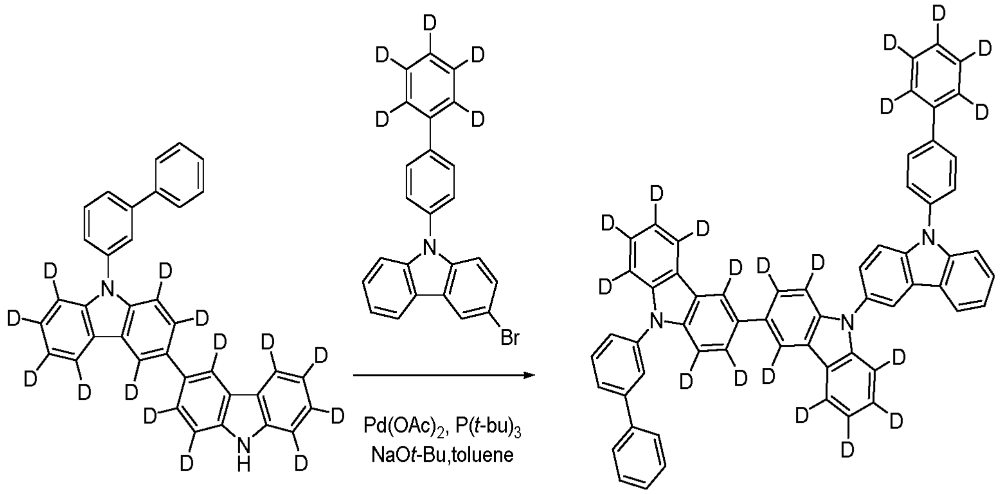


BCz-D3 U-1

Under a nitrogen atmosphere, BCz-D3 (10.0 g, 20.1 mmol), 9-([1,1'-biphenyl]-4-yl-2',3',4',5',6'-d5)-3-bromo-9H-carbazole (9.7 g, 24.1 mmol), $Pd(OAc)_2$ (1.15 g, 1.0 mmol), P(*t*-$Bu)_3$ (0.49 ml, 2.0 mmol), NaO(*t*-Bu) (3.85 g, 40.1 mmol), and toluene (100 ml) were mixed, and stirred at 110°C for 5 hours. After the reaction was completed, toluene was concentrated, and the solid salt was filtered and then purified by recrystallization, thereby obtaining the target compound U-1 (10.7 g, yield: 65%).
Mass (calcd.: 821.11, found: 821 g/mol)

**[Reference Examples]**

The structures of the compounds HT, HA, PA, $Ir(ppy)_3$, EA, ET, $(piq)_2Ir(acac)$, and CBP used in manufacturing organic EL elements according to examples and evaluation examples below are as follows.

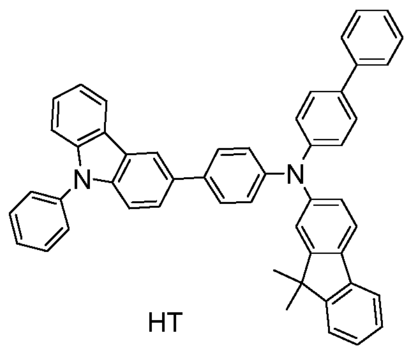

HT

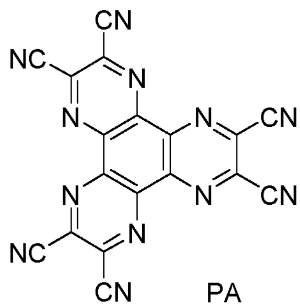

PA

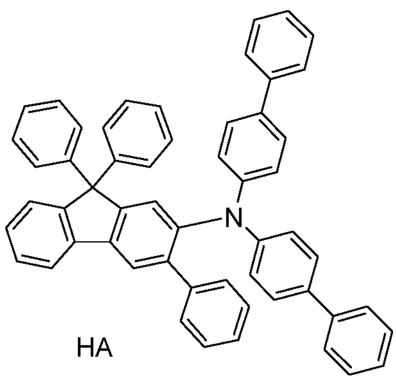

HA

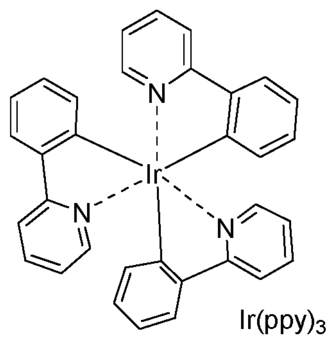

$Ir(ppy)_3$

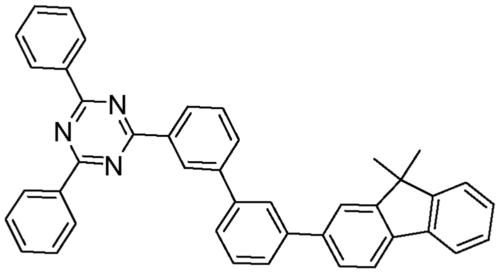

EA

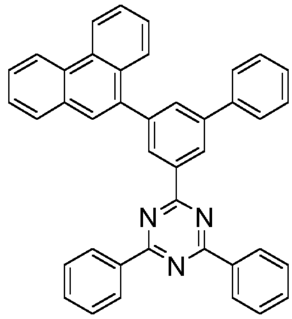

ET

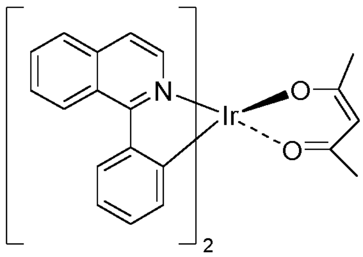

$(piq)_2Ir(acac)$

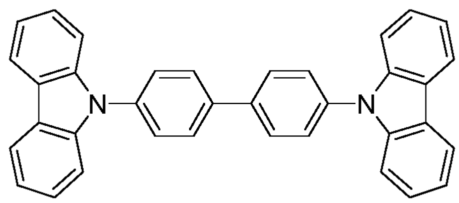

CBP

**[Example 1: Fabrication of green organic EL element]**

Compound A-1 synthesized in Synthesis Example 1 was subjected to high-purity sublimation purification by a commonly known method, and then a green organic EL element was fabricated as follows.

First, a glass substrate thin-film-coated with indium tin oxide (ITO) to a thickness of 1500 Å was ultrasonically washed with distilled water. After having washed with distilled water, the glass substrate was ultrasonically washed with a solvent, such as isopropyl alcohol, acetone, or methanol, dried, and then transferred to a UV OZONE cleaner (Power sonic 405, Hwashin Tech), and subsequently, the substrate was cleaned for 5 minutes using UV and transferred to a vacuum evaporator.

On the ITO transparent electrode thus prepared, HT + 2% PA (100 Å) / HT (1200 Å) / HA (300 Å) / 60% Compound A-1 + 30% Solus E-type HOST + 10% $Ir(ppy)_3$ (400 Å) / EA (50 Å) / ET + LiQ (300 Å_1:1) / LiF (10 Å) / Al (1000 Å) were laminated in that order, thereby fabricating an organic EL element.

**[Examples 2 to 88] Fabrication of green organic EL elements**

The same procedure as in Example 1 was performed except that Compounds A-2 to U-1 synthesized in Synthesis Examples 2 to 90, respectively, were used instead of Compound A-1 used as a light emission host material in the formation of an emissive layer, thereby fabricating green organic EL elements of Examples 2 to 90 (see Tables 1 and 2 below) .

**[Comparative Example 1] Fabrication of green organic EL element**

The same procedure as in Example 1 was performed except that Compound A-1-1 unsubstituted with deuterium and having the same structure was used instead of Compound A-1 used as a light emission host material in the formation of an emissive layer, thereby fabricating green organic EL element of Comparative Example 1. The structure of Compound A-1-1 used is as follows.

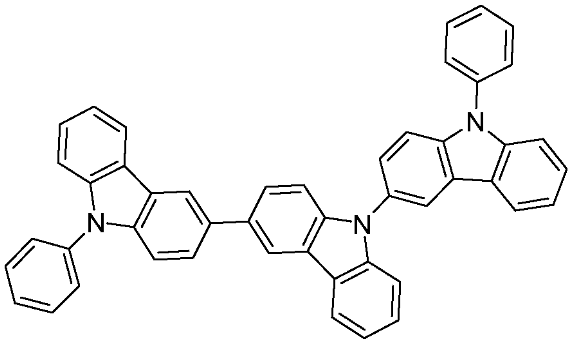

A-1-1

**[Comparative Example 2] Fabrication of green organic EL element**

The same procedure as in Example 2 was performed except that Compound A-2-1 unsubstituted with deuterium and having the same structure was used instead of Compound A-2 used as a light emission host material in the formation of an emissive layer, thereby fabricating green organic EL element of Comparative Example 2. The structure of Compound A-2-1 used is as follows.

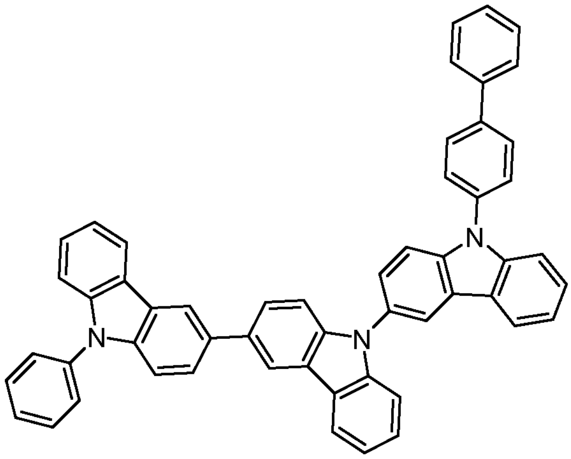

A-2-1

**[Comparative Example 3] Fabrication of green organic EL element**

The same procedure as in Example 3 was performed except that Compound A-3-1 unsubstituted with deuterium and having the same structure was used instead of Compound A-3 used as a light emission host material in the formation of an emissive layer, thereby fabricating green organic EL element of Comparative Example 3. The structure of Compound A-3-1 used is as follows.

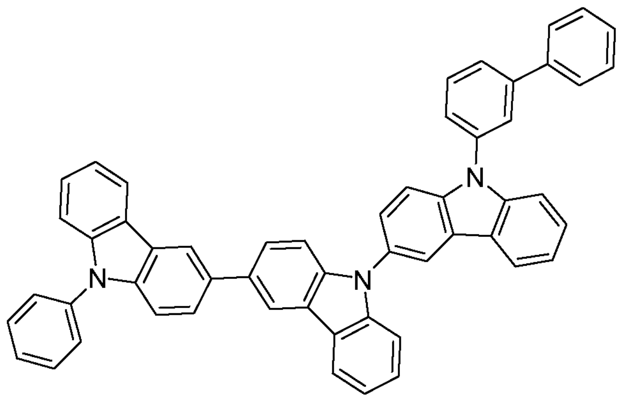

**A-3-1**

**[Comparative Example 4] Fabrication of green organic EL element**

The same procedure as in Example 4 was performed except that Compound A-4-1 unsubstituted with deuterium and having the same structure was used instead of Compound A-4 used as a light emission host material in the formation of an emissive layer, thereby fabricating green organic EL element of Comparative Example 4. The structure of Compound A-4-1 used is as follows.

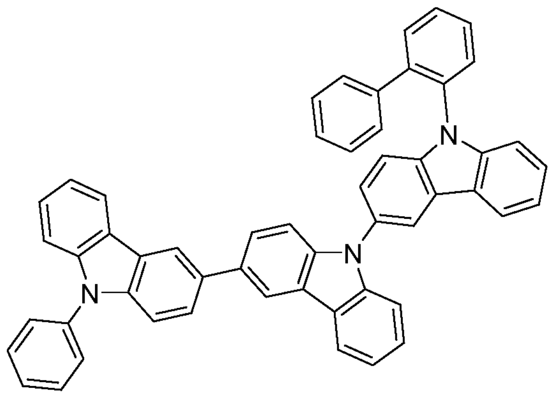

**A-4-1**

**[Evaluation Example 1]**

The green organic EL elements fabricated in Examples 1 to 4 and Comparative Examples 1 to 4 were measured for a driving voltage, a light emission peak, a current efficiency, and lifespan at a current density of (10) mA/cm$^2$, and the results are shown in Table 1 below.

TABLE 1

| Sample | Host | Driving voltage (V) | EL peak (nm) | Current efficiency (cd/A) | Lifespan (T97, hrs) |
|---|---|---|---|---|---|
| Example 1 | A-1 | 5.42 | 516 | 52.3 | 300 |
| Example 2 | A-2 | 5.32 | 515 | 50.9 | 310 |
| Example 3 | A-3 | 5.31 | 516 | 51.8 | 304 |
| Example 4 | A-4 | 5.40 | 518 | 53.2 | 301 |
| Comparative Example 1 | A-1-1 | 6.93 | 516 | 38.2 | 200 |
| Comparative Example 2 | A-2-1 | 6.83 | 517 | 37.2 | 210 |
| Comparative Example 3 | A-3-1 | 6.44 | 518 | 38.6 | 201 |

(continued)

| Sample | Host | Driving voltage (V) | EL peak (nm) | Current efficiency (cd/A) | Lifespan (T97, hrs) |
|---|---|---|---|---|---|
| Comparative Example 4 | A-4-1 | 6.52 | 515 | 38.9 | 180 |

As shown in Table 1, the green organic EL elements of Examples 1 to 4 using Compounds A-1 to A-4 according to the present invention as materials for an emissive layer were markedly superior in view of element efficiency, driving voltage, and lifespan characteristics, compared with the green organic EL elements of Comparative Examples 1 to 4 using Compounds A-1-1 to A-4-1 unsubstituted with deuterium (D) and having the same structures as the inventive compounds.

**[Evaluation Example 2]**

The green organic EL elements fabricated in Examples 5 to 90 were measured for a driving voltage, current efficiency, a light emission peak, and lifespan at a current density of (10) mA/$cm^2$, and the results are shown in Table 2 below.

TABLE 2

| Sample | Host | Driving voltage | EL peak | Current efficiency | Lifespan |
|---|---|---|---|---|---|
| | | (V) | (nm) | (cd/A) | (hr, $T_{97}$) |
| Example 5 | A-5 | 5,31 | 517 | 50.4 | 290 |
| Example 6 | A-6 | 5.33 | 518 | 51, 4 | 293 |
| Example 7 | A-7 | 5.34 | 517 | 55.2 | 293 |
| Example 8 | A-8 | 5.36 | 515 | 55.1 | 301 |
| Example 9 | A-9 | 5.52 | 518 | 51.4 | 301 |
| Example 10 | A-10 | 5.53 | 518 | 51.2 | 294 |
| Example 11 | A-11 | 5.43 | 517 | 52.3 | 294 |
| Example 12 | A-12 | 5.23 | 515 | 52.4 | 299 |
| Example 13 | A-13 | 5.43 | 518 | 52.6 | 298 |
| Example 14 | A-14 | 5.54 | 518 | 55.1 | 301 |
| Example 15 | A-15 | 5.56 | 517 | 50.1 | 310 |
| Example 16 | A-16 | 5.44 | 515 | 51.1 | 311 |
| Example 17 | B-1 | 5.56 | 518 | 52.2 | 314 |
| Example 18 | B-2 | 5.53 | 518 | 55.1 | 300 |
| Example 19 | B-3 | 5.54 | 517 | 49.8 | 300 |
| Example 20 | B-4 | 5.41 | 515 | 49.9 | 305 |
| Example 21 | B-5 | 5.46 | 518 | 52.1 | 304 |
| Example 22 | B-6 | 5.62 | 518 | 53.2 | 299 |
| Example 23 | B-7 | 5.24 | 518 | 55.4 | 280 |
| Example 24 | C-1 | 5.23 | 517 | 55.1 | 289 |
| Example 25 | C-2 | 5.24 | 515 | 51.2 | 281 |
| Example 26 | C-3 | 5.32 | 518 | 51.4 | 284 |
| Example 27 | C-4 | 5.39 | 518 | 52.3 | 283 |
| Example 28 | C-5 | 5.32 | 515 | 55.4 | 290 |
| Example 29 | C-6 | 5.33 | 518 | 54.4 | 310 |
| Example 30 | C-7 | 5.34 | 518 | 49.8 | 311 |

(continued)

| Sample | Host | Driving voltage | EL peak | Current efficiency | Lifespan |
|---|---|---|---|---|---|
| | | (V) | (nm) | (cd/A) | (hr, $T_{97}$) |
| Example 31 | D-1 | 5.42 | 518 | 49.7 | 305 |
| Example 32 | D-2 | 5.23 | 517 | 49.9 | 304 |
| Example 33 | D-3 | 5.24 | 515 | 49.3 | 303 |
| Example 34 | D-4 | 5.25 | 515 | 51.1 | 302 |
| Example 35 | D-5 | 5.42 | 518 | 51.2 | 300 |
| Example 36 | D-6 | 5.34 | 518 | 52.2 | 301 |
| Example 37 | D-7 | 5.33 | 517 | 53.2 | 308 |
| Example 38 | E-1 | 5.42 | 515 | 54.3 | 290 |
| Example 39 | E-2 | 5.61 | 518 | 52.1 | 291 |
| Example 40 | E-3 | 5.63 | 515 | 53.4 | 299 |
| Example 41 | E-4 | 5.62 | 514 | 55.5 | 298 |
| Example 42 | F-1 | 5.34 | 515 | 58.2 | 297 |
| Example 43 | F-2 | 5.42 | 518 | 52.1 | 296 |
| Example 44 | F-3 | 5.44 | 519 | 52.4 | 297 |
| Example 45 | F-4 | 5.46 | 511 | 55.4 | 298 |
| Example 46 | G-1 | 5.41 | 515 | 55.3 | 298 |
| Example 47 | G-2 | 5.41 | 520 | 55.2 | 300 |
| Example 48 | G-3 | 5.44 | 511 | 54.4 | 301 |
| Example 49 | G-4 | 5.46 | 514 | 53.2 | 300 |
| Example 50 | H-1 | 5.44 | 515 | 54.4 | 302 |
| Example 51 | H-2 | 5.51 | 515 | 52.3 | 300 |
| Example 52 | H-3 | 5.52 | 518 | 55.1 | 300 |
| Example 53 | H-4 | 5.55 | 513 | 52.3 | 299 |
| Example 54 | I-1 | 5.66 | 518 | 53.9 | 310 |
| Example 55 | I-2 | 5.61 | 515 | 52.8 | 310 |
| Example 56 | I-3 | 5.41 | 518 | 52.8 | 301 |
| Example 57 | I-4 | 5.29 | 518 | 51.7 | 301 |
| Example 58 | J-1 | 5.28 | 517 | 52.4 | 300 |
| Example 59 | J-2 | 5.22 | 515 | 53.2 | 299 |
| Example 60 | J-3 | 5.28 | 515 | 55.6 | 296 |
| Example 61 | J-4 | 5.28 | 518 | 52.4 | 294 |
| Example 62 | K-1 | 5.29 | 518 | 55.4 | 293 |
| Example 63 | K-2 | 5.34 | 511 | 55.2 | 290 |
| Example 64 | K-3 | 5.33 | 514 | 53.2 | 291 |
| Example 65 | K-4 | 5.36 | 515 | 55.6 | 294 |
| Example 66 | L-1 | 5.43 | 515 | 51.1 | 299 |
| Example 67 | L-2 | 5.33 | 518 | 50.4 | 300 |
| Example 68 | L-3 | 5.43 | 511 | 48.9 | 301 |

(continued)

| Sample | Host | Driving voltage (V) | EL peak (nm) | Current efficiency (cd/A) | Lifespan (hr, $T_{97}$) |
|---|---|---|---|---|---|
| Example 69 | L-4 | 5.51 | 514 | 48.8 | 302 |
| Example 70 | M-1 | 5.24 | 515 | 49.9 | 300 |
| Example 71 | M-2 | 5.34 | 515 | 47.9 | 300 |
| Example 72 | M-3 | 5.23 | 518 | 48.2 | 301 |
| Example 73 | M-4 | 5.67 | 513 | 48.3 | 304 |
| Example 74 | N-1 | 5.86 | 518 | 51.1 | 310 |
| Example 75 | N-2 | 5.43 | 515 | 51.2 | 311 |
| Example 76 | N-3 | 5.44 | 518 | 53.2 | 300 |
| Example 77 | N-4 | 5.64 | 517 | 54.2 | 290 |
| Example 78 | O-1 | 5.43 | 517 | 55.1 | 293 |
| Example 79 | O-2 | 5.44 | 515 | 51.4 | 294 |
| Example 80 | O-3 | 5.64 | 518 | 51.5 | 295 |
| Example 81 | O-4 | 5.55 | 517 | 55.2 | 299 |
| Example 82 | P-1 | 5.52 | 518 | 53.2 | 299 |
| Example 83 | P-2 | 5.61 | 517 | 52.1 | 301 |
| Example 84 | P-3 | 5.34 | 515 | 48.8 | 301 |
| Example 85 | P-4 | 5.42 | 513 | 48.9 | 290 |
| Example 86 | Q-1 | 5.33 | 514 | 49.1 | 290 |
| Example 87 | R-1 | 5.23 | 515 | 48.2 | 291 |
| Example 88 | S-1 | 5.42 | 516 | 51.4 | 290 |
| Example 89 | U-1 | 5.31 | 514 | 51.2 | 300 |
| Example 90 | T-1 | 5.28 | 517 | 54.4 | 289 |

As shown in Table 2, the green organic EL elements of Examples 5 to 90 using Compounds A-1 to S-1 according to the present invention as materials for an emissive layer were also superior in view of element efficiency, driving voltage, and lifespan characteristics, as in the above-described green organic EL elements of Examples 1 to 4.

**[Example 91] Fabrication of red organic EL element**

Compound A-1 synthesized in Synthesis Example 1 was subjected to high-purity sublimation purification by a commonly known method and then a red organic electroluminescent element was fabricated as follows.

First, a glass substrate thin-film-coated with indium tin oxide (ITO) to a thickness of 1500 Å was ultrasonically washed with distilled water. After having washed with distilled water, the glass substrate was ultrasonically washed with a solvent, such as isopropyl alcohol, acetone, or methanol, dried, and then transferred to a UV OZONE cleaner (Power sonic 405, Hwashin Tech), and subsequently, the substrate was cleaned for 5 minutes using UV and transferred to a vacuum evaporator.

On the ITO transparent electrode thus prepared, HT + 2% PA (100 Å) / HT (1200 Å) / HA (300 Å) / 50% Compound A-1 + 40% Solus E-type HOST + 10% $(piq)_2Ir(acac)$ (400 Å) / EA (50 Å) / ET + LiQ (300 Å_1:1) / LiF (10 Å) / Al (1000 Å) were laminated in that order, thereby fabricating an organic EL element.

**[Examples 92 to 106] Fabrication of red organic EL elements**

The same procedure as in Example 89 was performed except that Compounds A-2 to A-16 synthesized in Synthesis Examples 2 to 16, respectively, were used instead of Compound A-1 used as a light emission host material in the formation of an emissive layer, thereby fabricating red organic EL elements (see Table 3 below).

**[Comparative Example 5]**

The same procedure as in Example 91 was performed except that CBP was used instead of Compound A-1 used as a light emission host material in the formation of an emissive layer, thereby fabricating a red organic electroluminescent element of Comparative Example 5.

**[Evaluation Example 3]**

The organic electroluminescent elements fabricated in Examples 91 to 106 and Comparative Example 5 were measured for a driving voltage and current efficiency at a current density of 10 $mA/cm^3$, and the results are shown in Table 3 below.

TABLE 3

| Sample | Host | Driving voltage (V) | Current efficiency (cd/A) |
|---|---|---|---|
| Example 91 | A-1 | 4.65 | 11.8 |
| Example 92 | A-2 | 4.34 | 12.1 |
| Example 93 | A-3 | 4.56 | 12.4 |
| Example 94 | A-4 | 4.54 | 11.9 |
| Example 95 | A-5 | 4.43 | 11.5 |
| Example 96 | A-6 | 4.61 | 12.4 |
| Example 97 | A-7 | 4.56 | 12.6 |
| Example 98 | A-8 | 4.66 | 12.5 |
| Example 99 | A-9 | 4.34 | 11.6 |
| Example 100 | A-10 | 4.56 | 11.7 |
| Example 101 | A-11 | 4.46 | 11.8 |
| Example 102 | A-12 | 4.78 | 11.9 |
| Example 103 | A-13 | 4.74 | 11.5 |
| Example 104 | A-14 | 4.87 | 11.8 |
| Example 105 | A-15 | 4.74 | 11.5 |
| Example 106 | A-16 | 4.87 | 11.8 |
| Comparative Example 5 | CBP | 5.25 | 8.2 |

As shown in Table 3 above, the red organic EL elements of Examples 91 to 106 using Compounds A-1 to A-16 according to the present invention as materials for an emissive layer were superior in view of element efficiency and driving voltage compared with the conventional red organic electroluminescent element of Comparative Example 5 using CBP.

**[Examples 107 to 110] Fabrication of green organic EL elements**

Compound A-1 synthesized in Synthesis Example 1 was subjected to high-purity sublimation purification by a commonly known method, and then a green organic EL element was fabricated as follows.

First, a glass substrate thin-film-coated with indium tin oxide (ITO) to a thickness of 1200 Å was ultrasonically washed with distilled water. After having washed with distilled water, the glass substrate was ultrasonically washed with a solvent, such as isopropyl alcohol, acetone, or methanol, dried, and then transferred to a UV OZONE cleaner (Power sonic 405, Hwashin Tech), and subsequently, the substrate was cleaned for 5 minutes using UV and transferred to a vacuum evaporator.

On the ITO transparent electrode thus prepared, HT + 2% PA (100 Å) / HT (1200 Å) / HA (300 Å) / 60% Compound A-1 + 30% Solus E-type HOST + 10% $Ir(ppy)_3$ (400 Å) / EA (50 Å) / ET-1 to ET-4 + LiQ (300 Å._1:1) / LiF (10 Å) / Al (1000 Å) were laminated in that order, thereby fabricating an organic EL element.

Particularly, Compounds ET-1, ET-2, ET-3, and ET-4 used as materials for an electron transport layer are dual

electro transfer type materials, and the structures thereof are as follows.

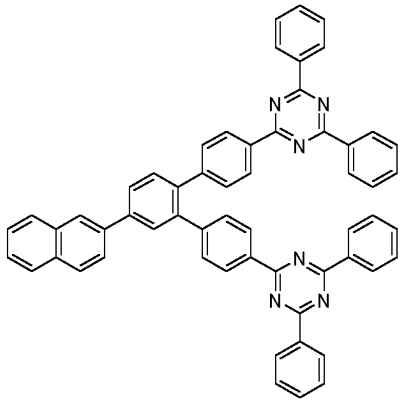

ET-1

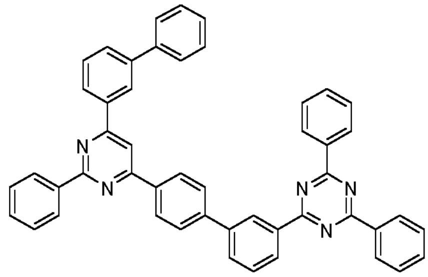

ET-2

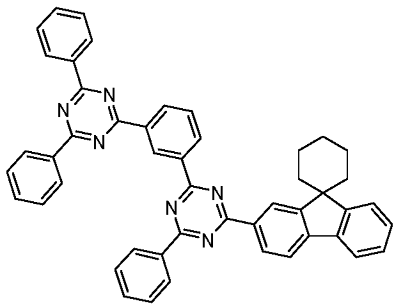

ET-3

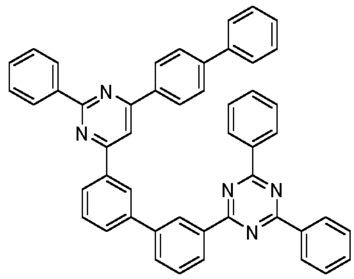

ET-4

**[Examples 111 to 114]**

The same procedure as in Examples 107 to 110 was performed except that Compounds A-2 synthesized in Synthesis Example 2 was used instead of Compound A-1 used as a light emission host material in the formation of an emissive layer, thereby fabricating a green organic EL element (see Table 4 below).

**[Examples 115 to 118]**

The same procedure as in Examples 107 to 110 was performed except that Compounds A-3 synthesized in Synthesis Example 3 was used instead of Compound A-1 used as a light emission host material in the formation of an emissive layer, thereby fabricating a green organic EL element (see Table 4 below).

**[Examples 119 to 122]**

The same procedure as in Examples 107 to 110 was performed except that Compounds A-4 synthesized in Synthesis Example 4 was used instead of Compound A-1 used as a light emission host material in the formation of an emissive layer, thereby fabricating a green organic EL element (see Table 4 below).

**[Comparative Examples 6 to 9] Fabrication of green organic EL elements**

The same procedure as in Example 107 was performed except that the single electro transfer material ET having one EWG was used as a material for an electron transport layer, instead of ET-1, and Compounds A-1 to A-4 were used as light emission host materials, thereby fabricating green organic EL elements.

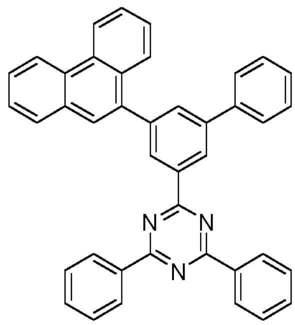

ET

[Evaluation Example 4]

The organic electroluminescent elements fabricated in Examples 107 to 122 and Comparative Examples 6 to 9 were measured for a driving voltage, current efficiency, light emission wavelength, and lifespan (T97) at a current density of 10 mA/cm$^4$, and the results are shown in Table 4 below.

TABLE 4

| Sample | Host | Electron transport layer | Driving voltage | EL peak | Current efficiency | Lifespan |
|---|---|---|---|---|---|---|
| | | | (V) | (nm) | (cd/A) | (hr, $T_{97}$) |
| Example 107 | A-1 | ET-1 | 4.81 | 516 | 61.2 | 320 |
| Example 108 | A-1 | ET-2 | 4.87 | 515 | 63.2 | 331 |
| Example 109 | A-1 | ET-3 | 4.56 | 516 | 62.3 | 324 |
| Example 110 | A-1 | ET-4 | 4.84 | 518 | 64.1 | 315 |
| Example 111 | A-2 | ET-1 | 4.45 | 518 | 64.3 | 342 |
| Example 112 | A-2 | ET-2 | 4.54 | 518 | 62.3 | 321 |
| Example 113 | A-2 | ET-3 | 4.65 | 517 | 65.4 | 333 |
| Example 114 | A-2 | ET-4 | 4.55 | 515 | 64.2 | 342 |
| Example 115 | A-3 | ET-1 | 4.67 | 518 | 66.1 | 351 |
| Example 116 | A-3 | ET-2 | 4.54 | 518 | 64.3 | 321 |
| Example 117 | A-3 | ET-3 | 4.56 | 517 | 65.1 | 334 |
| Example 118 | A-3 | ET-4 | 4.45 | 515 | 61.4 | 331 |
| Example 119 | A-4 | ET-1 | 4.56 | 518 | 62.4 | 341 |
| Example 120 | A-4 | ET-2 | 4.58 | 518 | 66.2 | 332 |
| Example 121 | A-4 | ET-3 | 4.89 | 517 | 63.1 | 322 |
| Example 122 | A-4 | ET-4 | 4.56 | 515 | 66.5 | 329 |
| Comparative Example 6 | A-1 | ET | 5.42 | 516 | 52.3 | 300 |
| Comparative Example 7 | A-2 | ET | 5.32 | 515 | 50.9 | 310 |
| Comparative Example 8 | A-3 | ET | 5.31 | 516 | 51.8 | 304 |
| Comparative Example 9 | A-4 | ET | 5.40 | 518 | 53.2 | 301 |

As shown in Table 4, the green organic EL elements of Examples 107 to 122 using materials for an emissive layer, containing deuterated Compounds A-1 to A-4, together with a dual EWG-type materials for an electron transport layer showed significant improvements in driving voltage, current efficiency, and lifespan characteristics of an element compared with Comparative Examples 6 to 9 using the deuterated compounds and a conventional material for a single

EWG-type electron transport layer. It can be seen from the results that a remarkable synergy effect in view of element performance characteristics can be achieved due to a combination of excellent hole stability resulting from the deuterated compounds of the present invention used in the emissive layers and high electron transporting ability resulting from the dual EWG-type materials for the electron transport layers. Therefore, the present invention enables the optimization of the performance of an organic electroluminescent element through the improvement in hole stability of an emissive layer (EML) and the maximization of electron transporting ability of an electron transport layer (ETL), by the use in combination of an emissive layer containing a compound of Chemical Formula 1 and a dual EWG-type electron transport layer containing at least two EWGs.

## Claims

**1.** A compound represented by Chemical Formula 1 below:

[Chemical Formula 1]

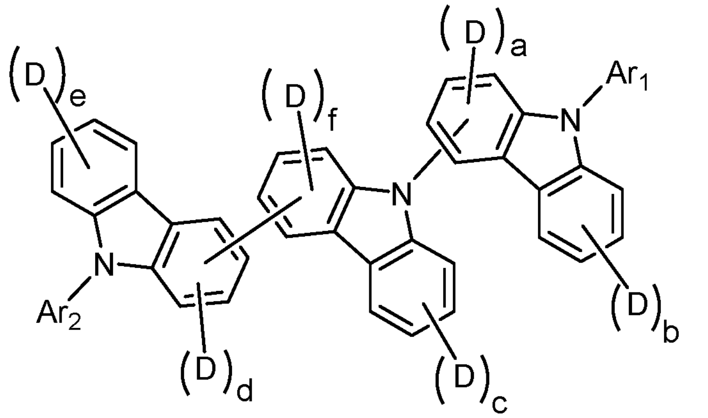

wherein Chemical Formula 1,

$Ar_1$ and $Ar_2$ are the same or different from each other and are each independently selected from the group consisting of a $C_1$ to $C_{40}$ alkyl group, a $C_2$ to $C_{40}$ alkenyl group, a $C_2$ to $C_{40}$ alkynyl group, a $C_6$ to $C_{40}$ aryl group, a heteroaryl group having 5 to 40 nuclear atoms, a $C_6$ to $C_{40}$ aryloxy group, a $C_1$ to $C_{40}$ alkyloxy group, a $C_6$ to $C_{40}$ arylamine group, a $C_3$ to $C_{40}$ cycloalkyl group, a heterocycloalkyl group having 3 to 40 nuclear atoms, a $C_1$ to $C_{40}$ alkylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylsilyl group;

a, d, and f are each independently 1 to 3;

b, c, and e are each independently 1 to 4, provided that $a + b + c + d + e + f \geq 15$;

hydrogen of a benzene ring within a carbazole unsubstituted with deuterium (D) may be substituted with a substituent selected from the group consisting of a $C_1$ to $C_{40}$ alkyl group, a $C_6$ to $C_{40}$ aryl group, and a heteroaryl group having 5 to 40 nuclear atoms; and

the alkyl group, alkenyl group, alkynyl group, aryl group, heteroaryl group, aryloxy group, alkyloxy group, arylamine group, cycloalkyl group, heterocycloalkyl group, alkylsilyl group, alkylboron group, arylboron group, arylphosphine group, arylphosphine oxide group and arylsilyl group of $Ar_1$ to $Ar_2$ are each independently substituted or unsubstituted with at least one substituent selected from the group consisting of deuterium (D), halogen, a cyano group, a nitro group, a $C_1$ to $C_{40}$ alkyl group, a $C_2$ to $C_{40}$ alkenyl group, a $C_2$ to $C_{40}$ alkynyl group, a $C_3$ to $C_{40}$ cycloalkyl group, a heterocycloalkyl group having 3 to 40 nuclear atoms, a $C_6$ to $C_{40}$ aryl group, a heteroaryl group having 5 to 40 nuclear atoms, a $C_1$ to $C_{40}$ alkyloxy group, a $C_6$ to $C_{60}$ aryloxy group, a $C_1$ to $C_{40}$ alkylsilyl group, a $C_6$ to $C_{40}$ arylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylamine group, and when substituents are plural in number, the substituents may be the same or different from each other.

**2.** The compound of claim 1, wherein the compound represented by Chemical Formula 1 contains 21 or more deuteriums (D).

**3.** The compound of claim 1, $Ar_1$ and $Ar_2$ are the same or different from each other and are each independently selected

from the group consisting of a $C_6$ to $C_{40}$ aryl group and a heteroaryl group having 5 to 40 nuclear atoms; and the aryl group and heteroaryl group of $Ar_1$ to $Ar_2$ are each independently substituted or unsubstituted with at least one substituent selected from the group consisting of deuterium (D), halogen, a cyano group, a $C_6$ to $C_{40}$ aryl group, and a heteroaryl group having 5 to 40 nuclear atoms.

**4.** The compound of claim 1, wherein $Ar_1$ and $Ar_2$ are each independently selected from the group consisting of substituents S1 to S9 below:

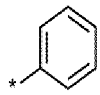
S1

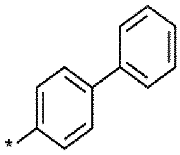
S2

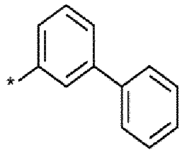
S3

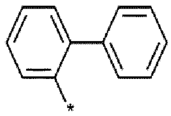
S4

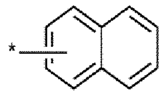
S5

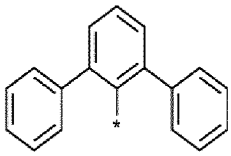
S6

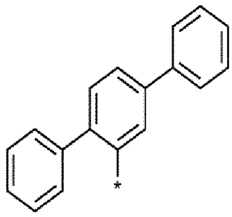
S7

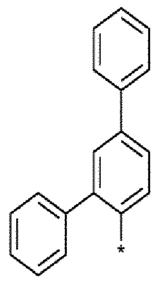
S8

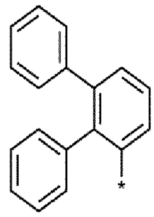
S9

wherein the formulas,
the mark * is a site linked to Chemical Formula 1.

**5.** The compound of claim 1, wherein the compound represented by Chemical Formula 1 is a compound represented by any one of Chemical Formulas 2 to Chemical Formula 17 below:

[Chemical Formula 2]

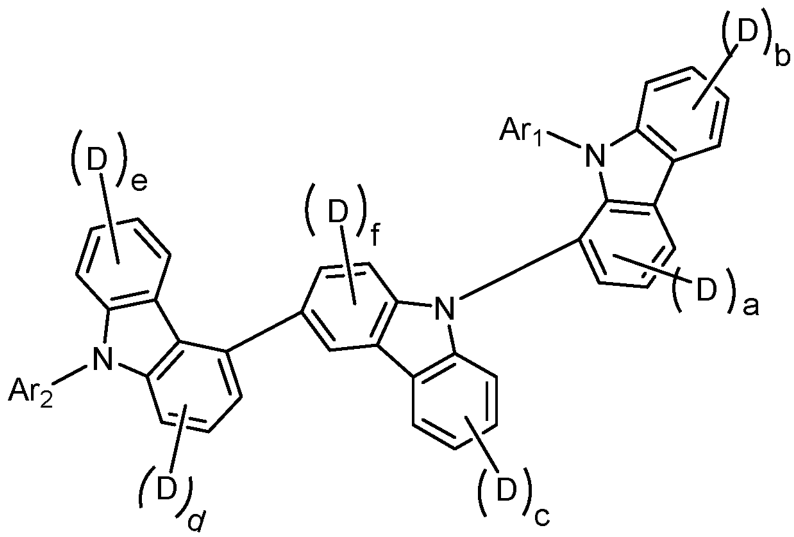

[Chemical Formula 3]

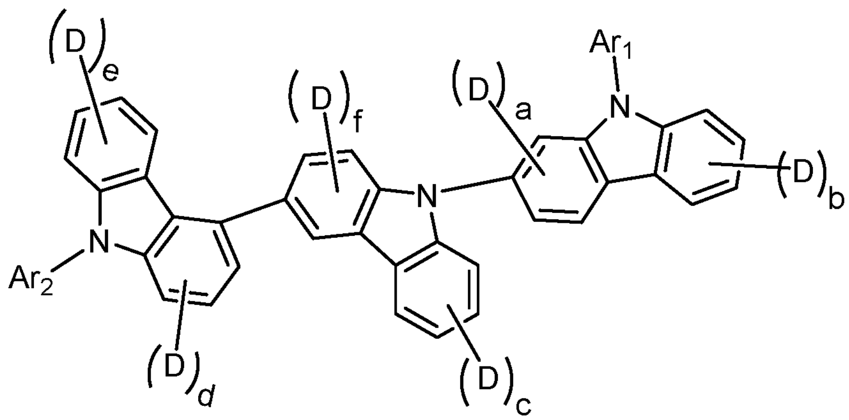

[Chemical Formula 4]

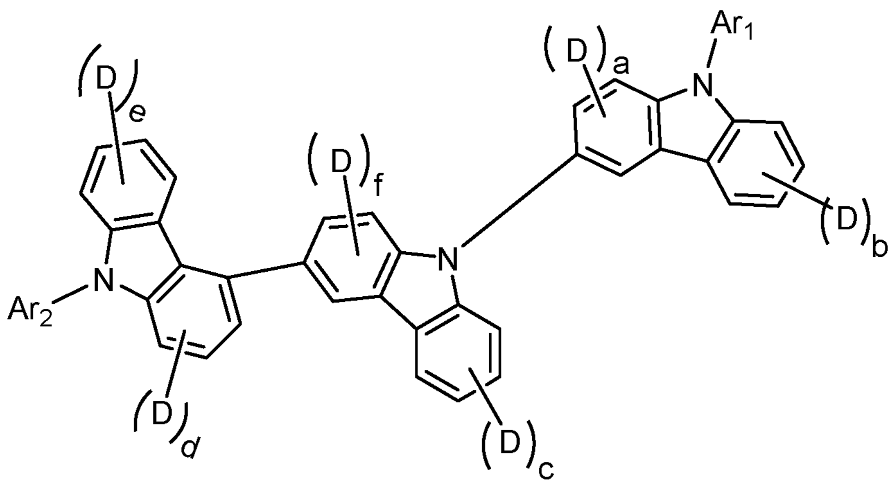

[Chemical Formula 5]

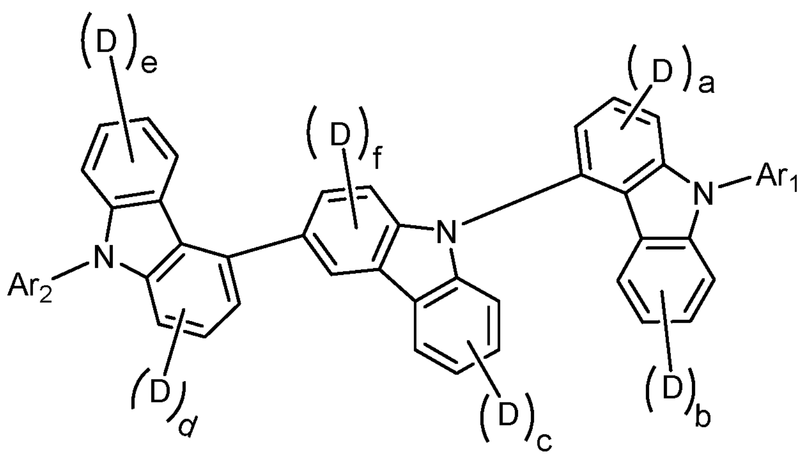

[Chemical Formula 6]

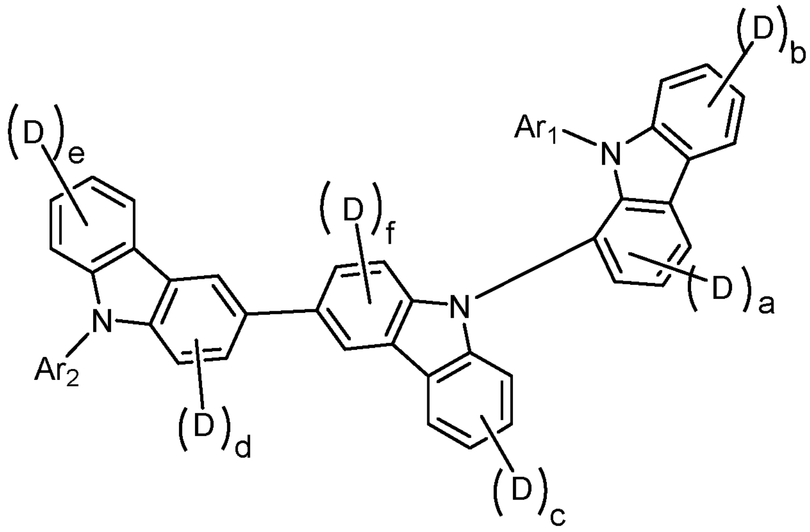

[Chemical Formula 7]

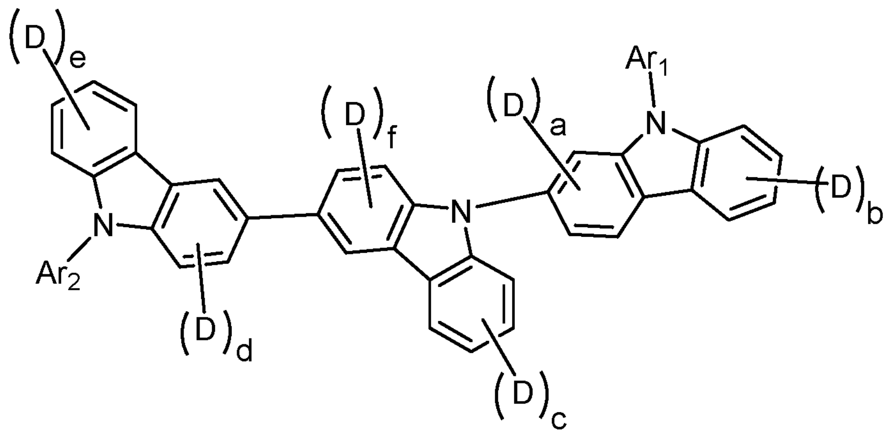

[Chemical Formula 8]

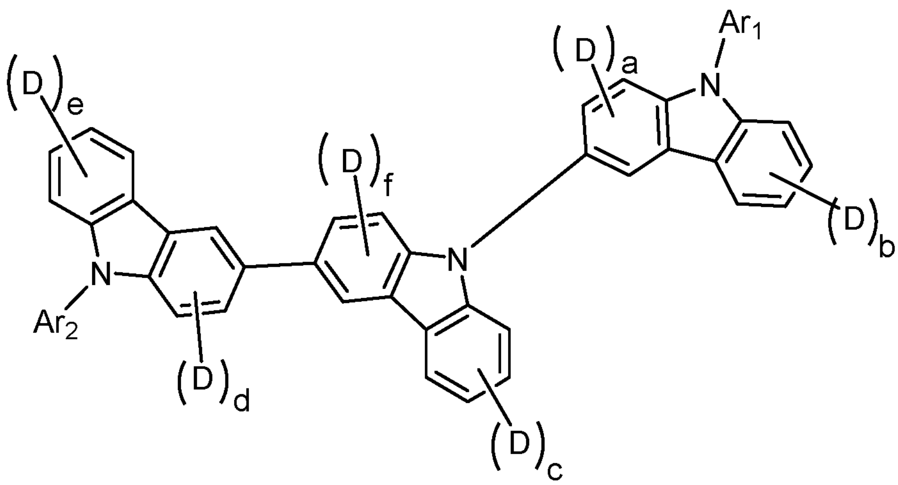

[Chemical Formula 9]

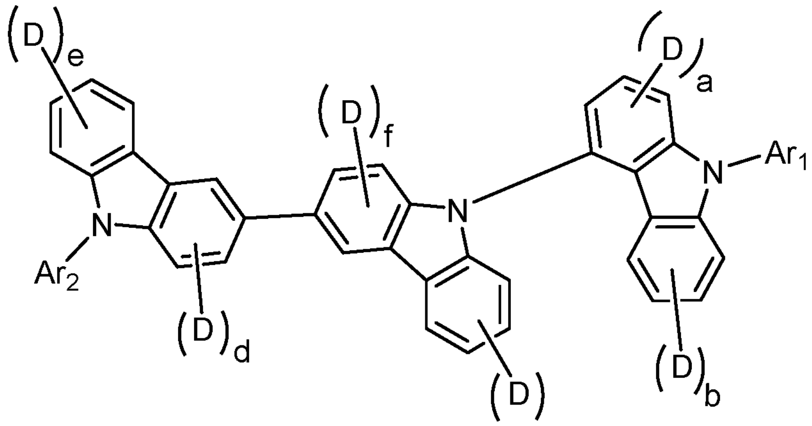

[Chemical Formula 10]

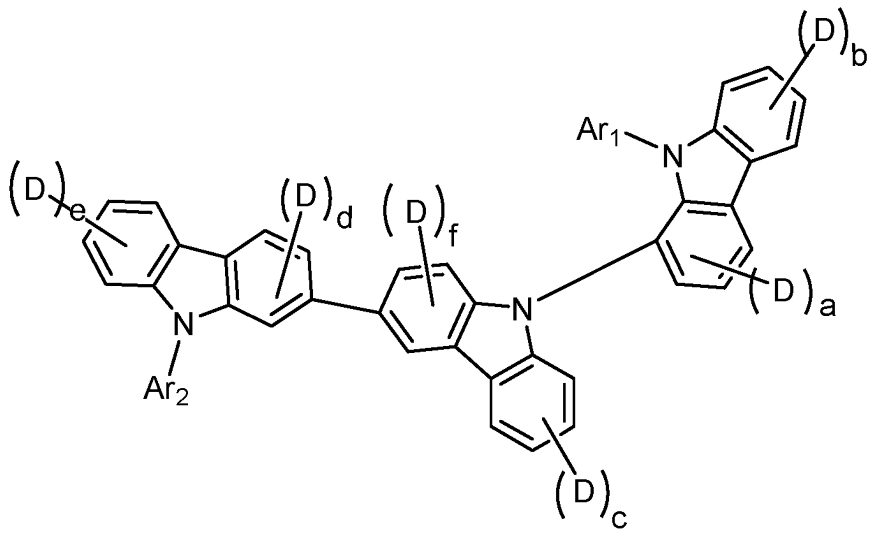

[Chemical Formula 11]

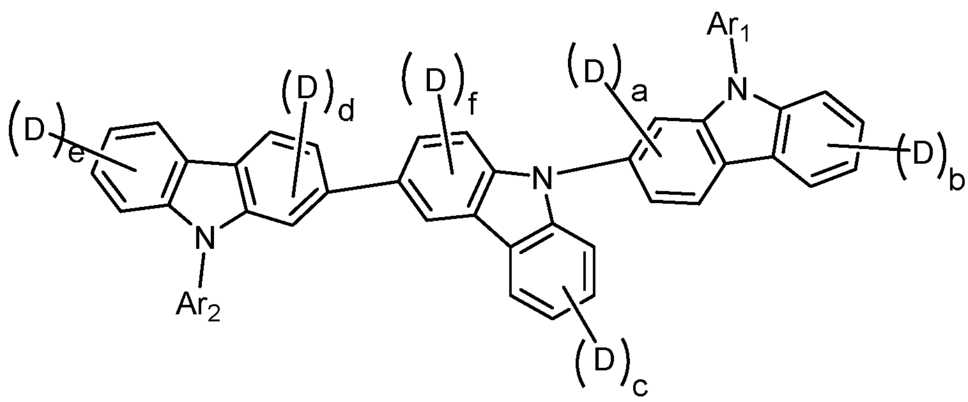

[Chemical Formula 12]

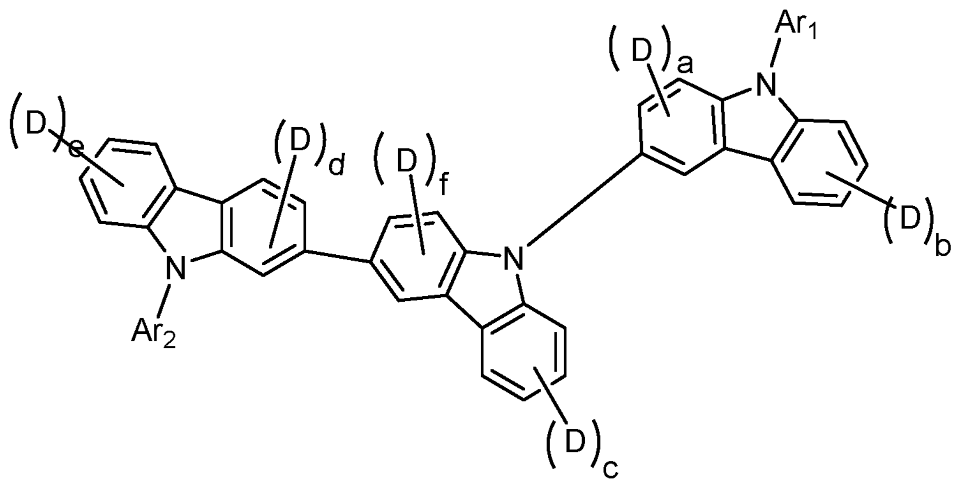

[Chemical Formula 13]

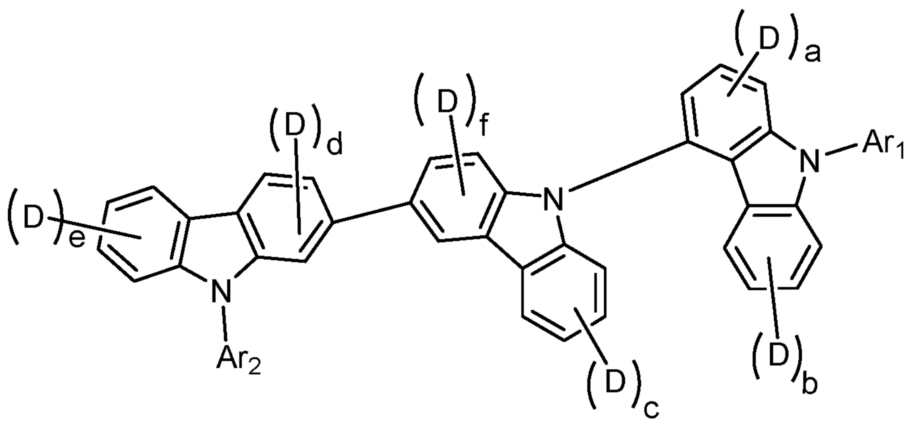

[Chemical Formula 14]

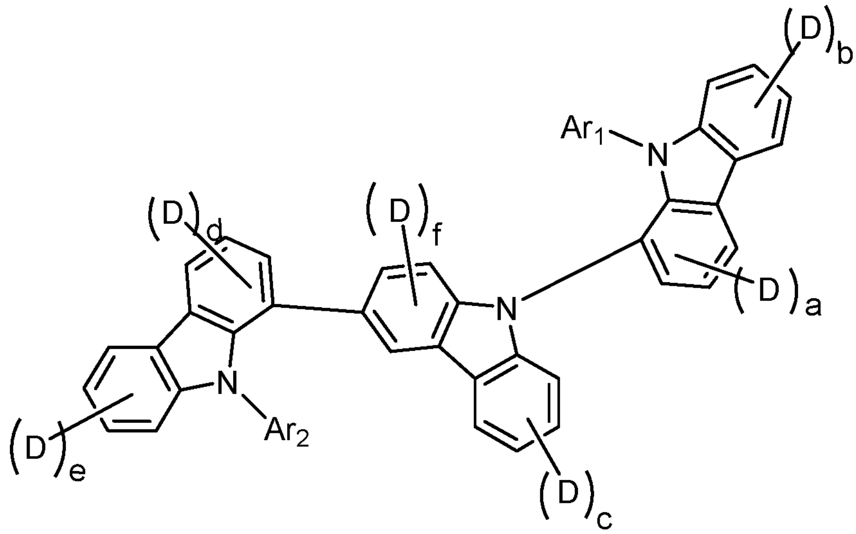

[Chemical Formula 15]

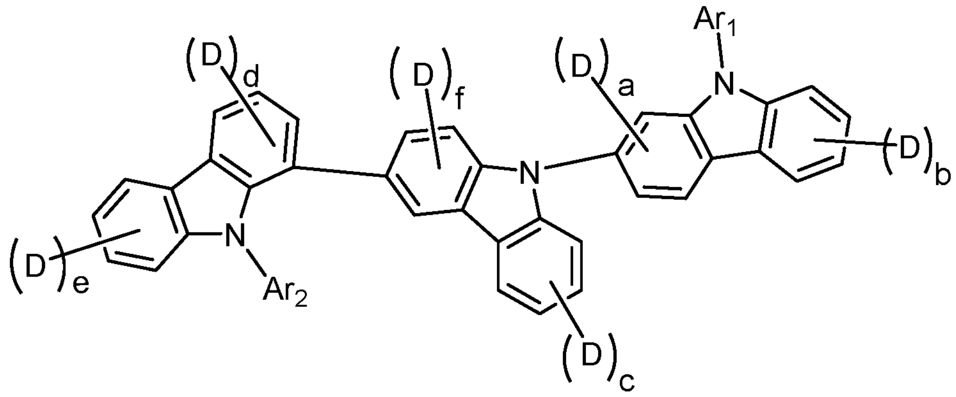

[Chemical Formula 16]

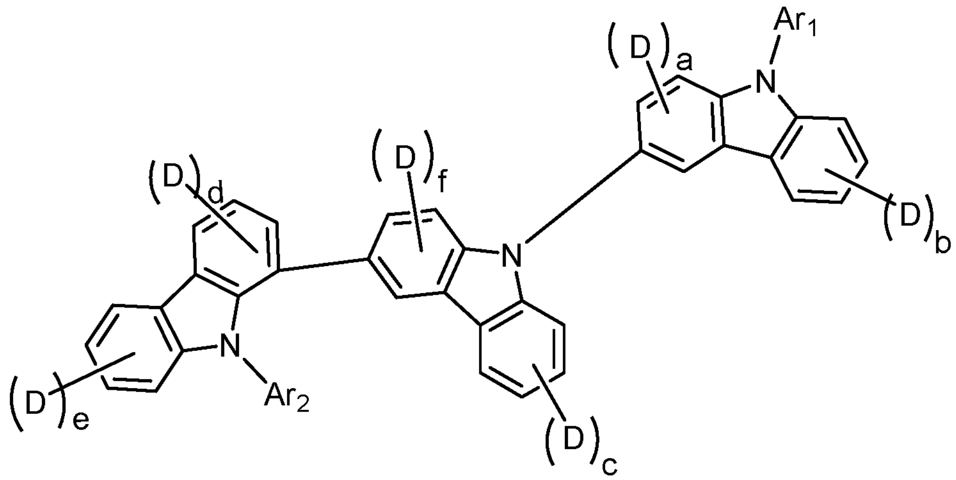

[Chemical Formula 17]

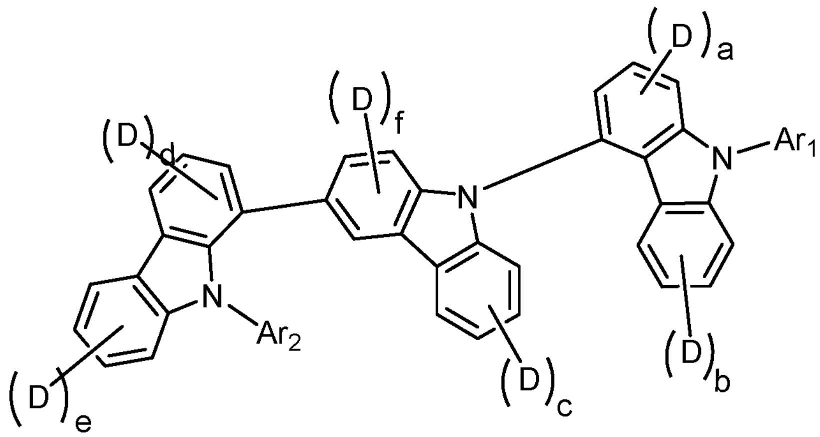

wherein the formulas,
$Ar_1$, $Ar_2$, a, b, c, d, e, and f are each as defined in claim 1.

**6.** The compound of claim 1, wherein the compound represented by Chemical Formula 1 is a host material for an emissive layer.

7. The compound of claim 1, wherein the compound represented by Chemical Formula 1 is selected from the group consisting of Compounds A-1 to U-1 below:

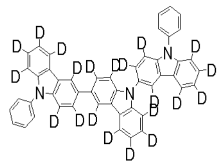
A-1

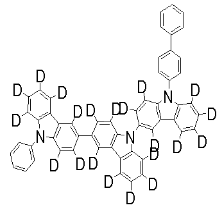
A-2

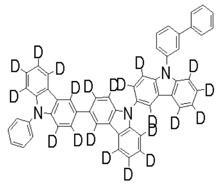
A-3

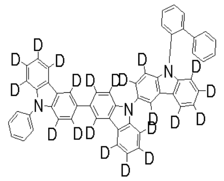
A-4

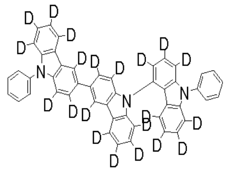
A-5

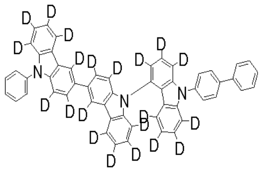
A-6

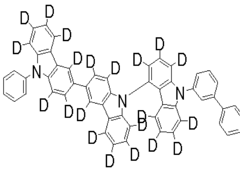
A-7

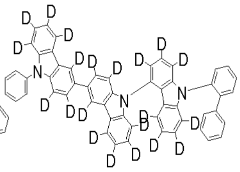
A-8

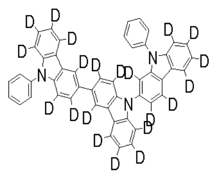
A-9

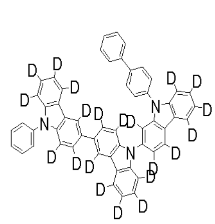
A-10

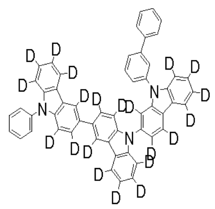
A-11

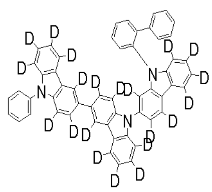
A-12

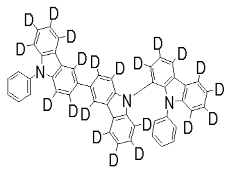
A-13

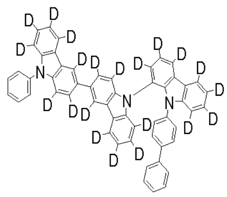
A-14

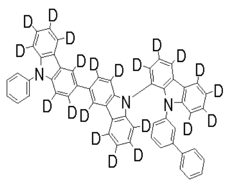
A-15

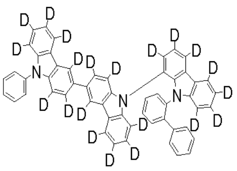
A-16

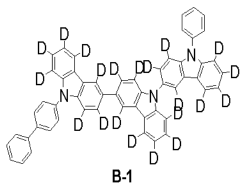
B-1

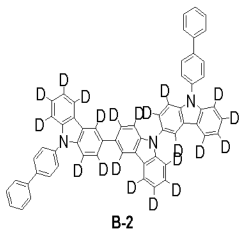
B-2

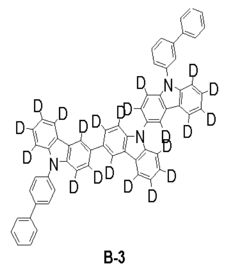
B-3

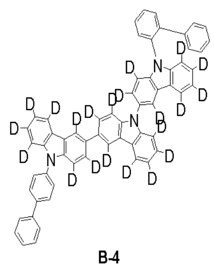
B-4

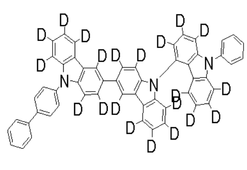
B-5

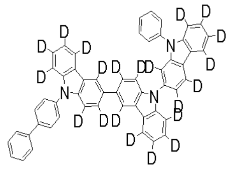
B-6

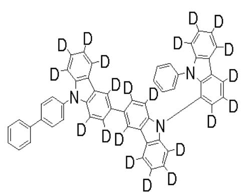
B-7

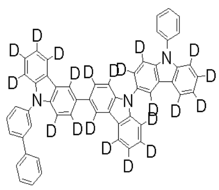
C-1

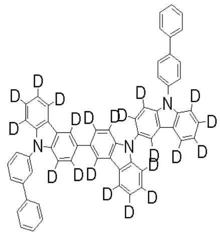
C-2

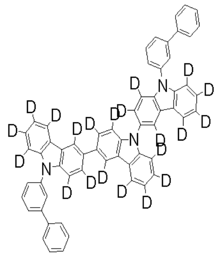
C-3

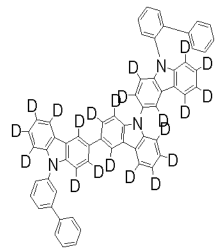
C-4

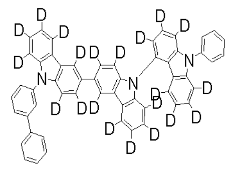
C-5

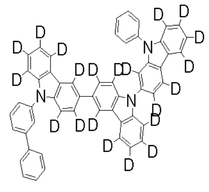
C-6

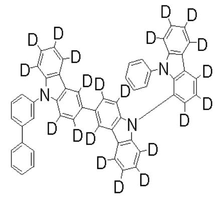
C-7

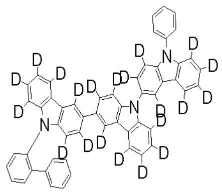
D-1

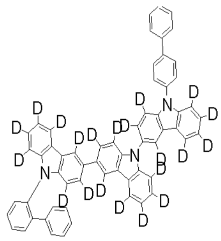
D-2

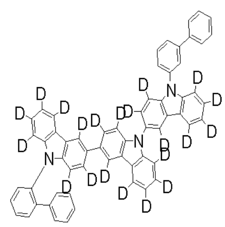
D-3

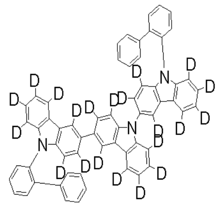
D-4

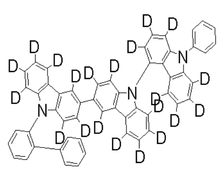
D-5

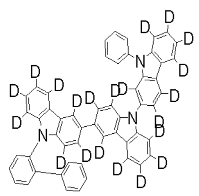
D-6

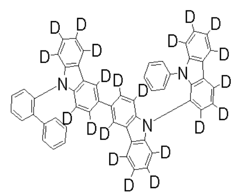
D-7

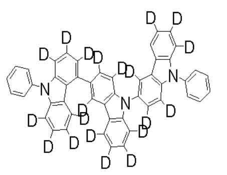
E-1

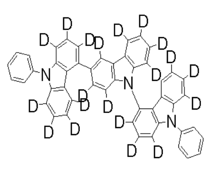
E-2

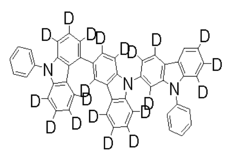
E-3

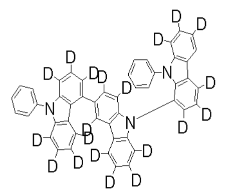
E-4

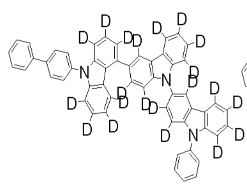
F-1

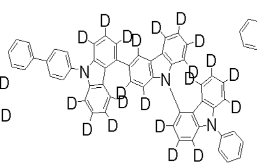
F-2

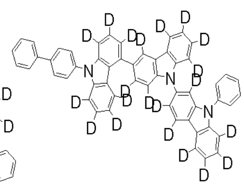
F-3

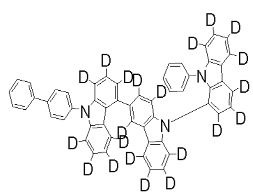
F-4

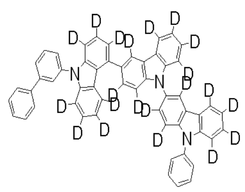
G-1

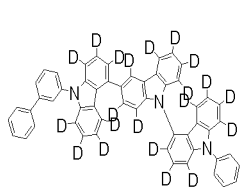
G-2

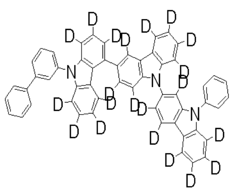
G-3

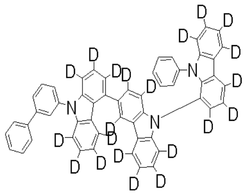
G-4

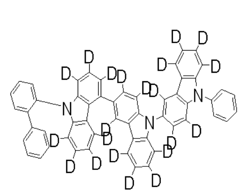
H-1

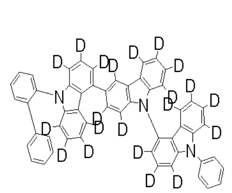
H-2

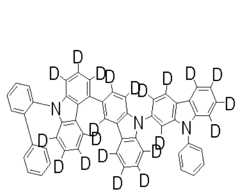
H-3

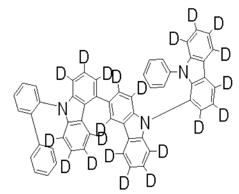
H-4

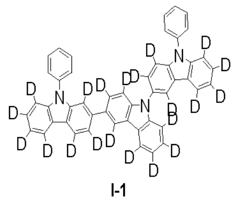
I-1

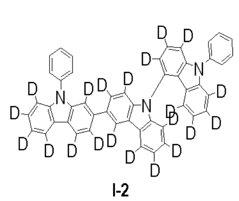
I-2

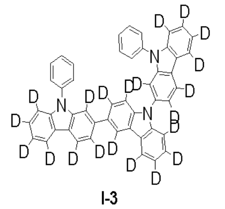
I-3

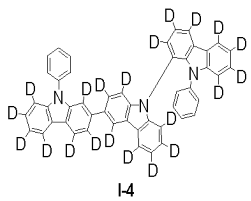
I-4

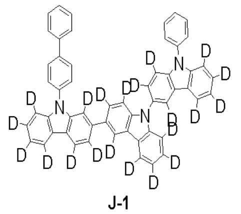
J-1

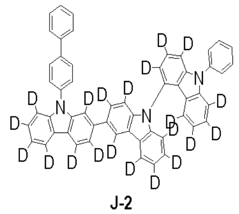
J-2

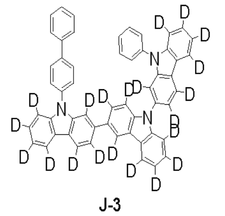
J-3

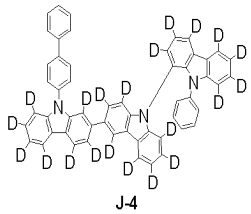
J-4

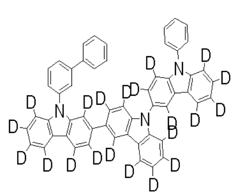
K-1

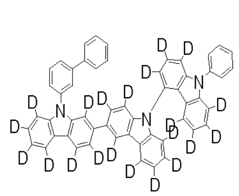
K-2

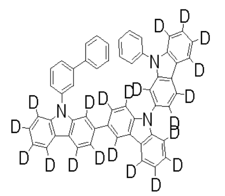
K-3

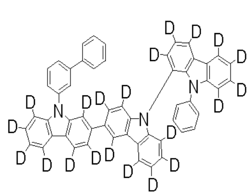
K-4

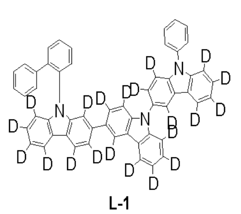
L-1

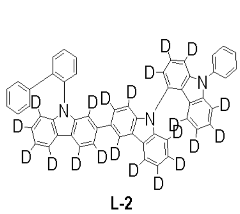
L-2

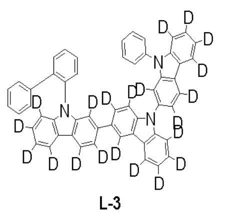
L-3

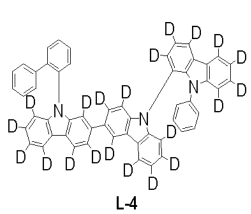
L-4

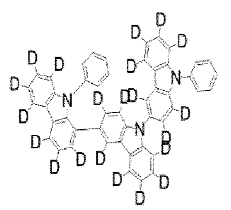
M-1

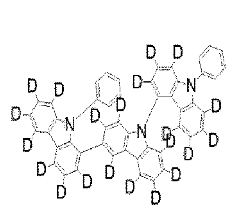
M-2

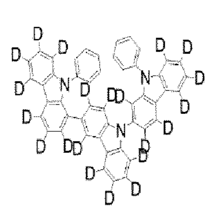
M-3

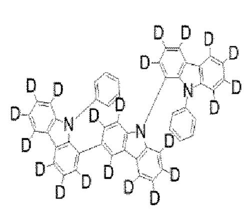
M-4

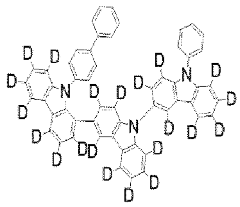

N-1

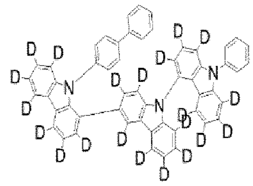

N-2

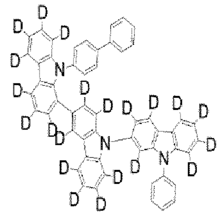

N-3

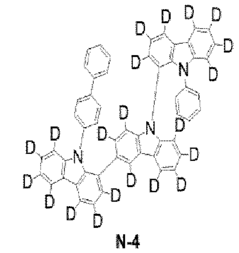

N-4

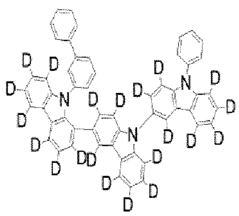

O-1

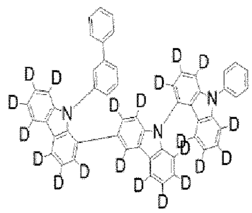

O-2

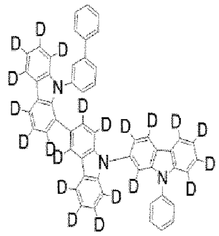

O-3

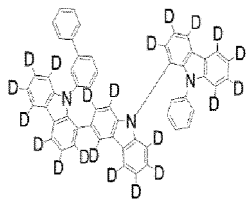

O-4

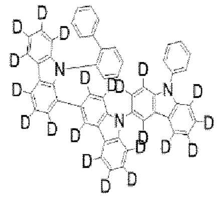

P-1

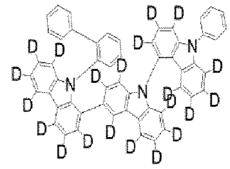

P-2

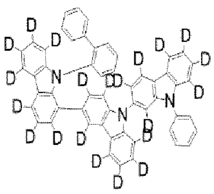

P-3

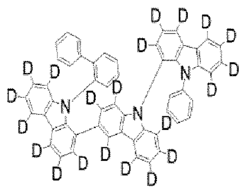

P-4

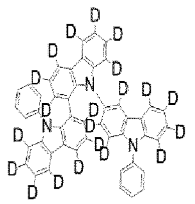

Q-1

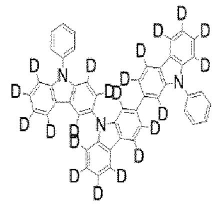

R-1

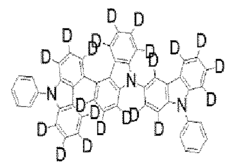

S-1

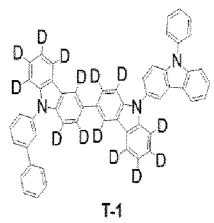

T-1

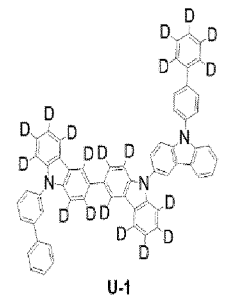

U-1

**8.** An organic electroluminescent element, comprising:

an anode; a cathode; and one or more organic layers interposed between the anode and the cathode, wherein at least one of the one or more organic layers contains the compound of any one of claims 1 to 7.

**9.** The organic electroluminescent element of claim 8, wherein the one or more organic layers include at least one selected from the group consisting of an emissive layer, an auxiliary emissive layer, a hole injection layer, a hole transport layer, an electron injection layer, an electron transport layer, and an auxiliary electron transport layer, and the emissive layer contains the compound of Chemical Formula 1 as a host.

**10.** The organic electroluminescent element of claim 9, wherein the emissive layer further contains at least one of a P-type

host material, an N-type host material, a fluorescent emissive dopant, and a phosphorescent emissive dopant.

**11.** The organic electroluminescent element of claim 9, wherein the electron transport layer contains a compound represented by Chemical Formula 18 below:

[Chemical Formula 18]

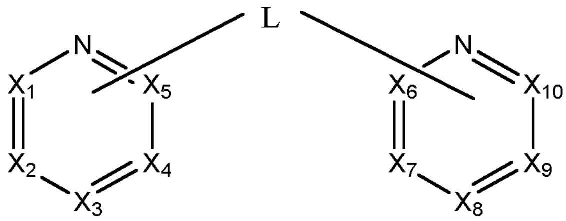

wherein Chemical Formula 18,

L is selected from the group consisting of a $C_6$ to $C_{40}$ arylene group and a substituted or unsubstituted heteroarylene group having 5 to 40 nuclear atoms;

$X_1$ to $X_{10}$ are each independently N or $C(R_1)$;

wherein when $C(R_1)$ is plural in number, the plurality of $R_1$ are the same or different from each other and are each independently selected from the group consisting of a $C_1$ to $C_{40}$ alkyl group, a $C_2$ to $C_{40}$ alkenyl group, a $C_2$ to $C_{40}$ alkynyl group, a $C_6$ to $C_{40}$ aryl group, a heteroaryl group having 5 to 40 nuclear atoms, a $C_6$ to $C_{40}$ aryloxy group, a $C_1$ to $C_{40}$ alkyloxy group, a $C_6$ to $C_{40}$ arylamine group, a $C_3$ to $C_{40}$ cycloalkyl group, a heterocycloalkyl group having 3 to 40 nuclear atoms, a $C_1$ to $C_{40}$ alkylsilyl group, a $C_1$ to $C_{40}$ alkylboron group, a $C_6$ to $C_{40}$ arylboron group, a $C_6$ to $C_{40}$ arylphosphine group, a $C_6$ to $C_{40}$ arylphosphine oxide group, and a $C_6$ to $C_{40}$ arylsilyl group; and

the arylene group and heteroarylene group of L, and the alkyl group, alkenyl group, alkynyl group, aryl group, heteroaryl group, aryloxy group, alkyloxy group, arylamine group, cycloalkyl group, heterocycloalkyl group, alkylsilyl group, alkylboron group, arylboron group, arylphosphine group, arylphosphine oxide group, and arylsilyl group of $R_1$ may each be independently substituted with at least one substituent selected from the group consisting of deuterium (D), halogen, a cyano group, a nitro group, a $C_1$ to $C_{40}$ alkyl group, a $C_2$ to $C_{40}$ alkenyl group, a $C_2$ to $C_{40}$ alkynyl group, a $C_3$ to $C_{40}$ cycloalkyl group, a heterocycloalkyl group having 3 to 40 nuclear atoms, a $C_6$ to $C_{60}$ aryl group, a heteroaryl group having 5 to 60 nuclear atoms, a $C_1$ to $C_{40}$ alkyloxy group, a $C_6$ to $C_{60}$ aryloxy group, a $C_1$ to $C_{40}$ alkylsilyl group, a $C_6$ to $C_{60}$ arylsilyl group, a $C_1$ to $C_{40}$ alkylboron group, a $C_6$ to $C_{60}$ arylboron group, a $C_6$ to $C_{60}$ arylphosphine group, a $C_6$ to $C_{60}$ arylphosphine oxide group, and a $C_6$ to $C_{60}$ arylamine group, and when substituents are plural in number, the substituents may be the same or different from each other.

**12.** The organic electroluminescent element of claim 11, wherein the $X_1$ to $X_5$ containing ring and the $X_6$ to $X_{10}$ containing ring are the same or different from each other and are each independently selected from the group consisting of substituents A-1 to A-5 below:

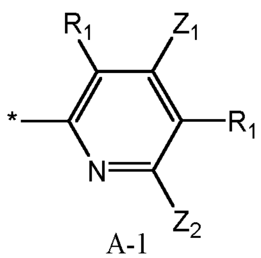

A-1

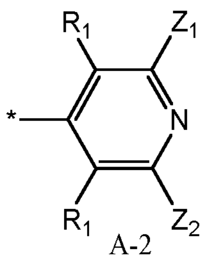

A-2

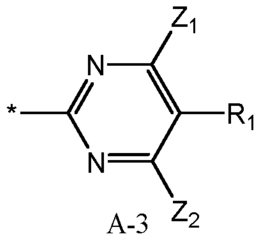 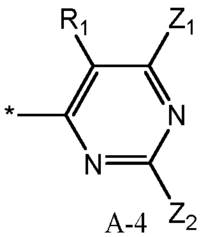 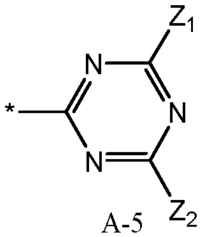

A-3 A-4 A-5

wherein the formulas,

$Z_1$ and $Z_2$ are the same or different from each other and are each independently selected from the group consisting of a $C_6$ to $C_{40}$ aryl group and a heteroaryl group having 5 to 40 nuclear atoms;

each $R_1$ is independently selected from the group consisting of hydrogen, a $C_1$ to $C_{40}$ alkyl group, a $C_6$ to $C_{40}$ aryl group, and a heteroaryl group having 5 to 40 nuclear atoms; and

the aryl group and heteroaryl group of $Z_1$ to $Z_2$, the alkyl group, aryl group, and heteroaryl group of $R_1$ may each be independently substituted with at least one substituent selected from the group consisting of deuterium (D), halogen, a cyano group, a $C_6$ to $C_{40}$ aryl group, and a heteroaryl group having 5 to 40 nuclear atoms, and when the substituents are plural in number, the substituents may be the same or different from each other.

**13.** The organic electroluminescent element of claim 12, $Z_1$ and $Z_2$ are each independently selected from the structural formulas below:

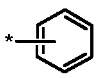 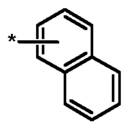 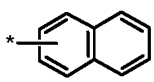 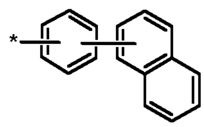

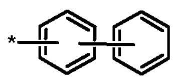 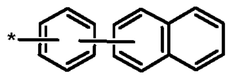 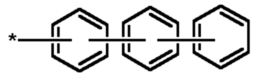

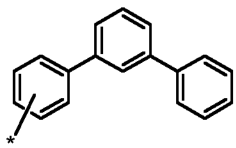 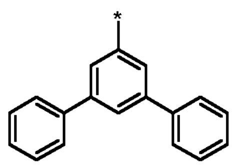 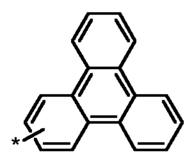

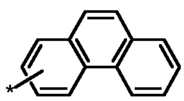 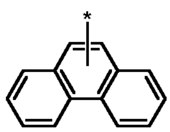 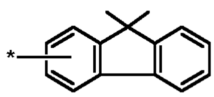

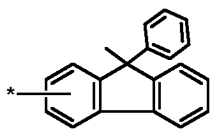 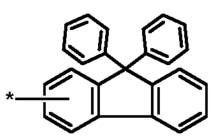 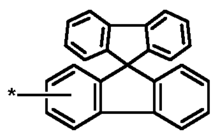

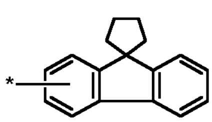 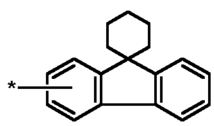 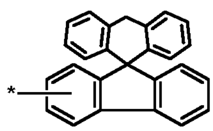

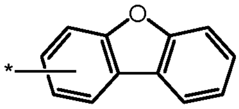 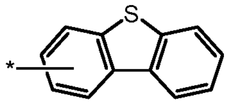 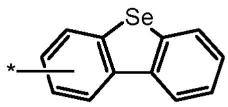

wherein the formulas,
the mark * is a site linked to Chemical Formula 18.

**14.** The organic electroluminescent element of claim 11, wherein L is a linker selected from the structural formulas below:

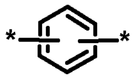 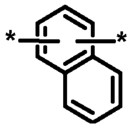 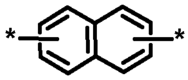 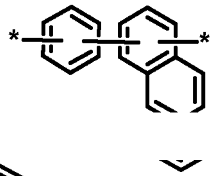

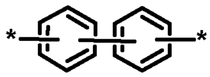 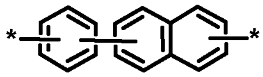

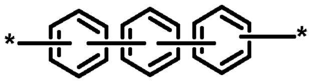 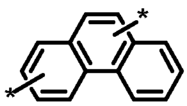 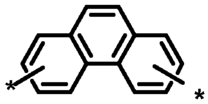

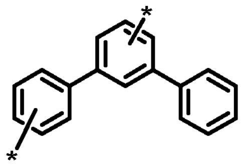 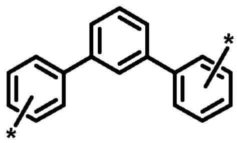 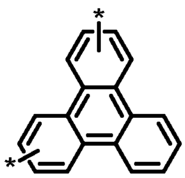

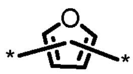 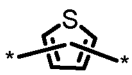 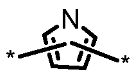 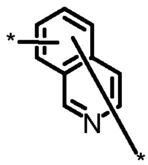 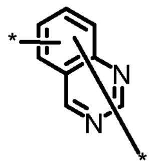

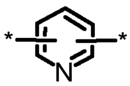 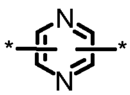 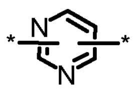 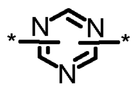

wherein the formulas,
the mark * is a site linked to Chemical Formula 18.

## INTERNATIONAL SEARCH REPORT

International application No.
**PCT/KR2023/006129**

**A. CLASSIFICATION OF SUBJECT MATTER**

**C07D 403/14**(2006.01)i; **H10K 85/60**(2023.01)i; **H10K 50/11**(2023.01)i; **C09K 11/06**(2006.01)i

According to International Patent Classification (IPC) or to both national classification and IPC

**B. FIELDS SEARCHED**

Minimum documentation searched (classification system followed by classification symbols)

C07D 403/14(2006.01); C07D 209/86(2006.01); C07D 401/14(2006.01); C07D 405/14(2006.01); H01L 51/00(2006.01); H01L 51/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: 유기발광소자(OLED, Organic Light Emitting Diode), 수명(lifetime), 구동 전압(driving voltage), 카바졸(carbazole), 중수소(deuterium)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2283849 B1 (ROHM AND HAAS ELECTRONIC MATERIALS KOREA LTD.) 02 August 2021 (2021-08-02)<br>See claim 11; and paragraphs [0046], [0196]-[0233] and [0245]-[0252]. | 1-10<br>11-14 |
| Y | KR 10-2020-0067058 A (DOOSAN SOLUS CO., LTD.) 11 June 2020 (2020-06-11)<br>See claims 1, 6 and 7. | 11-14 |
| Y | KR 10-2021-0032184 A (SOLUS ADVANCED MATERIALS CO., LTD.) 24 March 2021 (2021-03-24)<br>See claims 1, 7 and 11; and the examples, compound ET-4. | 11-14 |
| A | CN 114195700 A (SHAANXI LIGHTE OPTOELECTRONICS MATERIAL CO., LTD.) 18 March 2022 (2022-03-18)<br>See abstract; and claims 1, 13 and 14. | 11-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 August 2023** | **09 August 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/006129**

**C. DOCUMENTS CONSIDERED TO BE RELEVANT**

| Category* | Citation of document, with indication, where appropriate, of the relevant passages | Relevant to claim No. |
|---|---|---|
| PX | KR 10-2022-0122557 A (UNIVERSAL DISPLAY CORPORATION) 02 September 2022 (2022-09-02) See claim 1; and paragraphs [0230], [0240] and [0534]-[0535]. | 1-3,5,6,8-14 |

Form PCT/ISA/210 (second sheet) (July 2022)

**INTERNATIONAL SEARCH REPORT**
**Information on patent family members**

International application No.
**PCT/KR2023/006129**

| Patent document cited in search report | Publication date (day/month/year) | Patent family member(s) | Publication date (day/month/year) |
|---|---|---|---|
| KR 10-2283849 B1 | 02 August 2021 | CN 114075132 A | 22 February 2022 |
| | | CN 115548226 A | 30 December 2022 |
| | | JP 2022-036073 A | 04 March 2022 |
| | | KR 10-2022-0023700 A | 02 March 2022 |
| | | KR 10-2023-0002039 A | 05 January 2023 |
| | | US 2022-0109109 A1 | 07 April 2022 |
| KR 10-2020-0067058 A | 11 June 2020 | CN 113166107 A | 23 July 2021 |
| | | JP 2022-511481 A | 31 January 2022 |
| | | JP 7221389 B2 | 13 February 2023 |
| | | US 2022-0077400 A1 | 10 March 2022 |
| | | WO 2020-116881 A1 | 11 June 2020 |
| KR 10-2021-0032184 A | 24 March 2021 | CN 114424358 A | 29 April 2022 |
| | | EP 4033556 A1 | 27 July 2022 |
| | | JP 2022-548898 A | 22 November 2022 |
| | | US 2022-0344597 A1 | 27 October 2022 |
| | | WO 2021-054640 A1 | 25 March 2021 |
| CN 114195700 A | 18 March 2022 | CN 114195700 B | 13 September 2022 |
| | | EP 4198020 A1 | 21 June 2023 |
| | | KR 10-2023-0066006 A | 12 May 2023 |
| | | WO 2023-070987 A1 | 04 May 2023 |
| KR 10-2022-0122557 A | 02 September 2022 | CN 114957298 A | 30 August 2022 |
| | | CN 114975810 A | 30 August 2022 |
| | | CN 115707267 A | 17 February 2023 |
| | | EP 4059915 A2 | 21 September 2022 |
| | | EP 4059915 A3 | 28 December 2022 |
| | | EP 4060758 A2 | 21 September 2022 |
| | | EP 4060758 A3 | 29 March 2023 |
| | | JP 2022-132157 A | 07 September 2022 |
| | | JP 2022-132158 A | 07 September 2022 |
| | | JP 2023-024320 A | 16 February 2023 |
| | | KR 10-2022-0122932 A | 05 September 2022 |
| | | KR 10-2023-0022391 A | 15 February 2023 |
| | | US 2022-0177492 A1 | 09 June 2022 |
| | | US 2022-0181561 A1 | 09 June 2022 |
| | | US 2022-0399517 A1 | 15 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TONG et al.** *J. Phys. Chem. C*, 2007, vol. 111, 3490-4
- *Molecules*, 2014, 19
- *Chem. Commun.*, 2014, vol. 50, 14870-14872
- *J. Org. Chem.*, 2004, vol. 69, 7212-7219